# EUROPEAN PATENT SPECIFICATION

(11) **EP 2 199 405 B1**
(45) Date of publication and mention of the grant of the patent: **07.03.2012**
(21) Application number: 09252042.8
(22) Date of filing: 24.08.2009
(51) Int. Cl.: C12Q 1/48, C07K 4/00, C12N 15/00, G01N 33/68

(54) **Polypeptides substrates of LRRK2 and uses thereof**
LRRK2 Polypeptidesubstrate und ihre Verwendungen
Substrats polypeptidiques de LRRK2 et leurs utilisations

(30) Priority: 18.09.2008 GB 0817106; 02.10.2008 US 244715
(43) Date of publication of application: 23.06.2010
(73) Proprietor: Medical Research Council, Swindon Wiltshire SN2 1FL (GB)
(72) Inventor: Alessi, Dario, Dundee DD1 5EH (GB); Nichols, R. Jeremy, Dundee DD1 5EH (GB)
(74) Representative: Pilkington, Stephanie Joan

(56) References cited:
- ALEXA TELGMANN-RAUBER ET AL: "Genetic and physical maps around the sex-determining M-locus of the dioecious plant asparagus" MOLECULAR GENETICS AND GENOMICS, SPRINGER, BERLIN, DE LNKD- DOI:10.1007/S00438-007-0235-Z, vol. 278, no. 3, 3 July 2007 (2007-07-03), pages 221-234, XP019542785 ISSN: 1617-4623
- JERNEJ JAKSE ET AL: "Comparative sequence and genetic analyses of asparagus BACs reveal no microsynteny with onion or rice" THEORETICAL AND APPLIED GENETICS ; INTERNATIONAL JOURNAL OF PLANT BREEDING RESEARCH, SPRINGER, BERLIN, DE LNKD- DOI:10.1007/S00122-006-0407-Y, vol. 114, no. 1, 22 September 2006 (2006-09-22), pages 31-39, XP019457316 ISSN: 1432-2242
- JALEEL MAHABOOBI ET AL: "LRRK2 phosphorylates moesin at threonine-558: characterization of how Parkinson's disease mutants affect kinase activity" BIOCHEMICAL JOURNAL, THE BIOCHEMICAL SOCIETY, LONDON, GB, vol. 405, no. Part 2, 1 July 2007 (2007-07-01), pages 307-317, XP002494862 ISSN: 0264-6021
- MATSUI T ET AL: "RHO-KINASE PHOSPHORYLATES COOH-TERMINAL THREONINES OF EZRIN/RADIXIN/MOESIN (ERM) PROTEINS AND REGULATES THEIR HEAD-TO-TAIL ASSOCIATION" THE JOURNAL OF CELL BIOLOGY, ROCKEFELLER UNIVERSITY PRESS, US LNKD- DOI:10.1083/JCB.140.3.647, vol. 140, no. 3, 9 February 1998 (1998-02-09), pages 647-657, XP001083757 ISSN: 0021-9525
- GIASSON BENOIT I ET AL: "Mutations in LRRK2 as a cause of Parkinson's disease." NEURO-SIGNALS 2008 LNKD- PUBMED:18097165, vol. 16, no. 1, 5 December 2007 (2007-12-05), pages 99-105, XP002577164 published online ISSN: 1424-862X

## Description

There has been much interest raised by the recent discovery that different autosomal dominant point mutations within the gene encoding for the Leucine Rich Repeat protein Kinase-2 (LRRK2), predispose humans to develop late-onset Parkinson's disease (PD, OMIM accession number 609007), with a clinical appearance indistinguishable from idiopathic PD [1-4]. The genetic analysis undertaken to date indicates that mutations in LRRK2 are relatively frequent, not only accounting for 5-10% of familial PD, but are also found in a significant proportion of sporadic PD cases [5, 6]. Little is known about how LRRK2 is regulated in cells, what are its physiological substrates and how mutations in LRRK2 cause or increase risk of PD. In mammals there are two isoforms of the LRRK protein kinase, LRRK1 (2038 residues) and LRRK2 (2527 residues). They belong to a protein family that has also been termed Roco [7]. Thus far mutations in LRRK2, but not LRRK1 have been linked to PD.

The LRRK/Roco class of protein kinases was initially characterised in the slime mould Dictyostelium discoideum, as a protein termed GbpC (cGMP binding protein C), that comprised an unusual member of the Ras/GTPase superfamily, distinct from other small GTPase domains as it possesses other domains including a protein kinase [7, 8]. Subsequent studies suggested that GbpC regulates chemotaxis and cell polarity in Dictyostelium [9], but the physiological substrates for this enzyme have not been elucidated. The defining feature of the LRRK/Roco-proteins is that they possess Leucine Rich Repeat (LRR) motif, a Ras-like small GTPase, a region of high amino acid conservation that has been termed the C-terminal Of Ras of complex (COR) domain, and a protein kinase catalytic domain [7, 10]. The protein kinase domain of LRRK2 belongs to the tyrosine-like serine/threonine protein kinases and is most similar to the Rho-Interacting Protein kinases (RIPK), that play key roles in innate immunity signalling pathways [11]. Other domains are also found on specific members of the LRRK kinases. For example, the GbpC possesses an additional DEP, cyclicGMP-binding and Ras-GEF domains that are not found in mammalian LRRK1 and LRRK2. Human LRRK1 possesses 3 ankyrin repeats at its N-terminus, whereas LRRK2 lacks these domains, but possesses a WD40 repeat located towards its C-terminus not found in LRRK1 [7].

Human LRRK2 consists of leucine rich repeats (residues 1010-1287), a small GTPase domain (residues 1335-1504), a COR domain (residues 1517-1843), a serine/threonine protein kinase domain (residues 1875-2132) and a motif that has low resemblance to a WD40 repeat (2231-2276). To date -20 single amino acid substitution mutations have been linked to autosomal-dominant PD, and these have been found within or in close proximity to conserved residues of the small GTPase, COR, protein kinase and WD40 domains [3, 4].

The most prevalent mutant form of LRRK2 accounting for ∼6% of familial PD and 3% of sporadic PD cases in Europe, comprises an amino acid substitution of Gly2019 located within the conserved DYG-Mg2+-binding motif, in subdomain-VII of the kinase domain, to a Ser residue [3]. Recent reports suggest that this mutation moderately enhances, -2-3-fold, the autophosphorylation of LRRK2, as well as its ability to phosphorylate myelin basic protein (12, 13). These findings suggest that over-activation of LRRK2 predisposes humans to develop PD, implying that drugs which inhibited LRRK2, could be utilised to delay the onset or even treat some forms of PD. The study of LRRK2 has been hampered by the difficulty in expressing active recombinant enzyme and by the lack of a robust quantitative assay.

Jaleel et al (2007) Biochem J 405(2), 307-317 describes substrates for LRRK2, including ERM family polypeptides moesin, ezrin, radixin and merlin, and characterises how Parkinson's disease mutants affect kinase activity.

The listing or discussion of a prior-published document in this specification should not necessarily be taken as an acknowledgement that the document is part of the state of the art or is common general knowledge.

We have developed a further substrate for LRRK2, which we have utilised in developing a robust and quantitative assay for LRRK2, useful for, for example, assessing the effect of test compounds on LRRK2 activity.

A first aspect of the invention provides a method for identifying a compound expected to be useful in modulating, for example inhibiting, LRRK2 protein kinase activity, the method comprising the steps of (1) determining whether a test compound modulates, for example inhibits, the protein kinase activity of a LRRK2 polypeptide on a substrate polypeptide and (2) selecting a compound which modulates, for example inhibits, the said LRRK2 polypeptide protein kinase activity, wherein the substrate polypeptide comprises the sequence (W/F/R/K)(W/F/R/K)(R/K)(F/W/H/R)(Y/W/R)(S/T)(L/V/I)(R/K)(R/K)(A/Y) or (W/R)(X)(X)(F/Y/H/T)(Y/W/R)(T)(X)(R/T)(R)(X), where X represents any amino add.

The underlined residue is the residue that is considered to be phosphorylated by LRRK2. It is preferred that this residue is a threonine residue. Preferences for the other residues (numbered relative to the phosphorylated TIS residue) are as follows:
- 5: W or R, -4: W, F, R, K or I; -3: R, K or W -2: F, Y, H or T; -1: Y, R, W; +1: L, V, K or I; +2: R, K or T; +3: R. It is preferred that none of positions -5, -4, -3, -2, -1, 0, +1, +2 , +3 or +4 are D or E. It is preferred that position -2 is not R. It is preferred that position -5 is not A.

The protein kinase activity of the LRRK2 polypeptide that is modulated/assessed in the screening method is phosphorylation of a substrate polypeptide as defined above. Phosphorylation of the substrate polypeptide may be assessed by techniques including those discussed further below and in the Examples. For example, antibodies specific for a phosphorylated (or unphosphorylated) phosphorylation site of the substrate polypeptide may be used in assessing phosphorylation of that phosphorylation site, as well known to those skilled in the art. Further methods will be apparent to the skilled person on the basis of this teaching and the many known methods of assessing protein phosphorylation.

Substrate polypeptide phosphorylation may be assessed in vitro or in a cell, for example by assessing phosphorylation of the substrate polypeptide following immunoprecipitation of the substrate polypeptide from the cellular material, for example following incubation of the cell with ³²P- or ³³P-γ-labelled ATP.

A further aspect of the invention provides a method for identifying a compound expected to be useful in modulating, for example inhibiting, the phosphorylation of an ERM family polypeptide in a cell, the method comprising the steps of (1) determining whether a test compound modulates, for example inhibits, the protein kinase activity of a LRRK2 polypeptide on a substrate polypeptide and (2) selecting a compound which modulates, for example inhibits, the said LRRK2 polypeptide protein kinase activity, wherein the substrate polypeptide comprises the sequence (W/F/R/K)(W/F/R/K)(R/K)(F/W/H/R)(Y/W/R)(S/T)(L/V/I)(R/K)(R/K)(A/Y) (or (W/R)(X)(X)(F/Y/H/T)(Y/W/R)(T)(X)(R/T)(R)(X)) (or, as explained above, is otherwise as defined in relation to the first aspect of the invention).

In an embodiment of the first aspect, the method may be for identifying a compound expected to be useful in treating or preventing Parkinson's Disease (PD) or Parkinsonism, or other neurodegenerative condition. Thus, the method may comprise the steps of (1) determining whether a test compound modulates, for example inhibits, the phosphorylation of a substrate polypeptide, and (2) selecting a compound which modulates, for example inhibits, the phosphorylation of the substrate polypeptide, wherein the substrate polypeptide comprises the sequence (W/F/R/K)(W/F/R/K)(R/K)(F/W/H/R) (Y/W/R)(S/T)(L/V/I)(R/K)(R/K)(A/Y) (or (W/R)(X)(X)(F/Y/H/T)(Y/W/R)(T)(X)(R/T)(R)(X)). The method may comprise the steps of (1) determining whether a test compound modulates, for example inhibits, the phosphorylation of the said substrate polypeptide by an LRRK2 polypeptide, and (2) selecting a compound which modulates, for example inhibits, the phosphorylation of the substrate polypeptide by the LRRK2 polypeptide. Examples of methods for assessing the phosphorylation of the substrate polypeptide are discussed above and in the Examples and include methods making use of phosphorylation-specific antibodies, as discussed above.

The activity of the LRRK2 polypeptide may be measured by measuring the phosphorylation by the LRRK2 polypeptide, in the presence of a suitable phosphate donor, of a substrate polypeptide, as discussed above. Examples of methods of assessing the phosphorylation of the substrate polypeptide are indicated above.

The protein kinase activity may be increased or reduced by an alteration in the Vₘₐₓ or the Kₘ (or both) of the LRRK2 polypeptide for a particular substrate. For example, activity may be increased by an increased Vₘₐₓ or decreased Kₘ. It will be appreciated that it may not be necessary to determine the value of either Vₘₐₓ or Kₘ in order to determine whether the LRRK2 polypeptide has been activated or deactivated.

Activity may be measured as the amount of a substrate phosphorylated in a given time; a change of activity may therefore be detected as a change in the amount of substrate (for example, at a single concentration) that is phosphorylated in a given time. It is preferred that the activity is increased or decreased, as appropriate, by at least 2, preferably 5, 10, 15, 20, 25, 30 or 50-fold.

It will be appreciated that it may be necessary to determine the effect of the compound on the properties of the substrate, for example by measuring the properties of the substrate when exposed to the compound (1) after exposure of the substrate to the LRRK2 polypeptide, (2) before exposure of the substrate to the LRRK2 polypeptide and/or (3) without exposure to the LRRK2 polypeptide.

By modulation of the protein kinase activity is included inhibition or an increase in the protein kinase activity.

It will be appreciated that in the methods of the invention wherein phosphorylation of a polypeptide may occur that the presence of a suitable phosphate donor may be required, as described for the above aspect of the invention. Suitable phosphate donors will be known to those skilled in the art and include ATP, for example as the magnesium salt (MgATP), as described in the Examples.

It may be useful to assess the effect of the test compound on the binding of the LRRK2 polypeptide and the substrate polypeptide. Methods of assessing polypeptide:polypeptide interactions will be well known to those skilled in the art.

The LRRK2 polypeptide may, for example, be purified from cells in which the LRRK2 polypeptide is expressed naturally, but it may be more convenient for the LRRK2 polypeptide to be recombinant. As described further below and in the Examples, an LRRK2 fragment has been identified that is useful in raising or selecting an antibody that is useful in, for example, preparing LRRK2 that retains protein kinase activity from cells in which the LRRk2 polypeptide is expressed naturally. Thus, the LRRK2 polypeptide may be an LRRK2 polypeptide prepared using such an antibody.

The term ERM family polypeptide will be well known to those skilled in the art. ERM family members include moesin, ezrin and radixin. Merlin is another ERM family member. ERM family members are considered to be substrates of LRRK2, as discussed in Jaleel et al (2007) *supra* and in PCT/GB2008/001211, filed on 7 April 2008.

Examples of Accession numbers for ERM family polypeptides in the NCBI database include:
AAB02864, M86450 (pig moesin)
AAA39728, M86390.1, NP_034963, NM 010833.2(house mouse moesin)
NP_002435, NM 002444.2 (human moesin)
NP_110490, NM 030863.1 (Norway rat moesin)
NP_001039942, NM 001046477.1 (bovine moesin)
NP_062230, NM 019357.1 (Norway rat ezrin)
CAA43086, X60671.1 (house mouse ezrin)
P15311 (human ezrin)

NP_002897, NM 002906.3 (human radixin)
NP_033067, NM_009041 (house mouse radixin)
NP_001005889, NM 001005889.2 (Norway rat radixin)
NP_001009576, NM 001009576.1 (pig radixin)

P35240 (human merlin)
P46662 (house mouse merlin)
Q63648 (Norway rat merlin)

Numerous further examples of mammalian and non-mammalian ERM family polypeptide sequences can be accessed in the sequence databases accessible from the NCBI Medline™ service, as will be well known to the person skilled in the art.

The substrate polypeptide used in the assay may typically be recombinant or chemically synthesised. The substrate polypeptide may be, for example, a baderially-expressed or mammalian cell-expressed substrate polypeptide (for example as described in the Examples). The substrate polypeptide may be or comprise a fusion polypeptide (for example as described in Example 1) that retains the ability to be phosphorylated by a LRRK2 polypeptide, for example by LRRK2[1326-2527] or LRRK2[1326-2527, G2019S], for example as described in the Examples. The fusion may typically be with a tag, for example a GST tag, as will be well known to those skilled in the art. Alternatively or in addition, it may be with all or part of an ERM polypeptide. In an example the wild-type phosphorylation site of the ERM polypeptide may be replaced by the substrate polypeptide sequence of the invention.

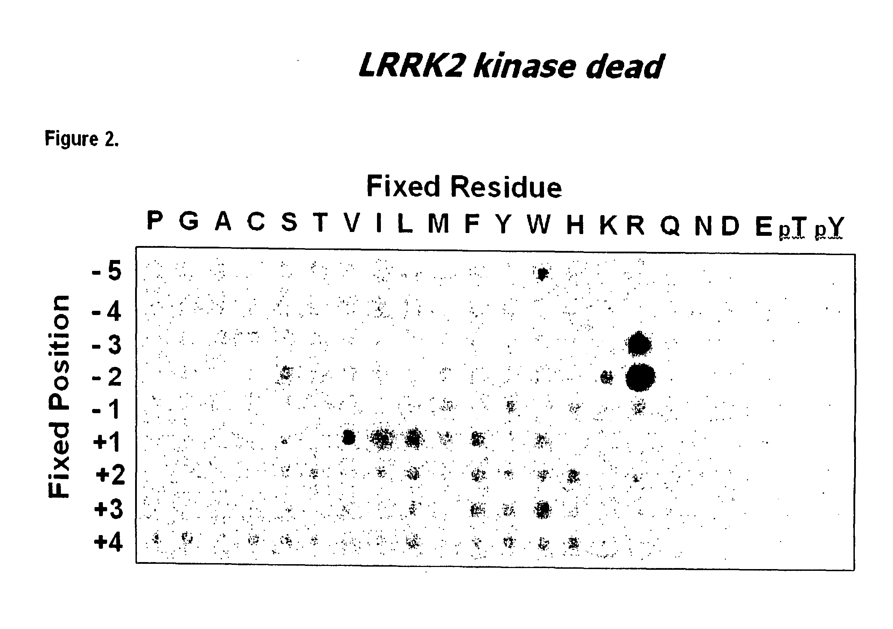

As indicated above, the substrate polypeptide comprises the sequence (W/F/R/K)(W/F/R/K)(R/K)(F/W/H/R)(Y/W/R)(S/T)(L/V/I)(R/K)(R/K)(A/Y) or (W/R)(X)(X)(F/Y/H/T)(Y/W/R)(T)(X)(R/T)(R)(X). For example, the substrate polypeptide may comprise the sequence WWRFYTLRRA. Alternatively, the substrate polypeptide may comprise the sequence WWKFYTLRRA or WWRFYTLRKA. An example of a suitable substrate polypeptide is RLGWWRFYTLRRARQGNTKQR. The residue phosphorylated by LRRK2 is underlined. This substrate polypeptide is termed "Nictide" in the Examples and Figures. The following are further examples of suitable substrate peptides: RLGWWKFYTLRRARQGNTKQR and RLGWWRFYTLRKARQGNTKQR. Figures 2, 3, 12, 13 and 14 also indicate peptide sequences that are considered to be compatible with the ability of the peptide to act as a substrate for LRRK2, reflected in the consensus sequence.

As noted above, the substrate polypeptide may comprise a tag sequence, as will be well known to those skilled in the art, for example a Glutathione S-Transferase (GST) or a Myc tag. Thus, a further example of a suitable substrate polypeptide is a fusion of RLGWWRFYTLRRARQGNTKQR and a tag sequence, for example a GST tag, for example as described in the Examples.

The substrate polypeptide can be a polypeptide of less than 100, 80, 60, 50, 40, 30, 25, 20, 19, 18, 17 or 16 amino acids, comprising the amino acid sequence (W/F/R/K)(W/F/R/K)(R/K)(F/W/H/R)(Y/W/R)(S/T)(L/V/I)(R/K)(R/K)(A/Y) or (W/R)(X)(X)(F/Y/H/T)(Y/W/R)(T)(X)(R/T)(R)(X). The substrate polypeptide sequence may typically comprise one or more amino acid sequences of at least five amino acids in length derived from the sequence of a naturally occurring ERM family polypeptide, for example moesin, radixin or ezrin, for example human moesin, radixin or ezrin, optionally with conservative or non-conservative substitutions of residues (for example of up to 10, 20, 30, 40, 50 or 60% of the residues). The amino acid sequence derived from the sequence of a naturally occurring ERM family polypeptide typically does not include the residue(s) of the ERM family polypeptide that are phosphorylated by LRRK2, for example the residue corresponding to Thr558 (or Thr 526) of moesin.

The substrate polypeptides shown above include a sequence (GNTKQR) that is present in the previously identified substrate sequence RLGRDKYK(T/S)LRQIRQGNTKQR (termed Long-LRRKtide) but not in the previously identified shorter sequence RLGRDKYK(T/S)LRQIRQ (short LRRKtide). Long LRRKtide is considered to allow the use 5-10-fold lower amounts of peptide relative to short LRRKtide. The sequence GNTKQR is a sequence found in moesin, ezrin, or radixin.

It may be necessary to denature the substrate polypeptide (for example if it comprises both a FERM domain (for example residues 1 to 298 of human moesin) and the C-terminal tail region (C-ERMAD domain, for example residues 489 to 575 of human moesin)) in order for it to be phosphorylated *in vitro* by an LRRK2 polypeptide, as discussed in Jaleel et al (2007) *supra* and in PCT/GB2008/001211, filed on 7 April 2008 (published as WO 2008/122789). Accordingly, it may be desirable for the substrate polypeptide not to comprise a functional FERM domain.

The term LRRK2 will be well known to those skilled in the art, as indicated above. The LRRK2 polypeptide used in the assay may be recombinant or non-recombinant. The LRRK2 polypeptide may, for example, be a recombinant or non-recombinant polypeptide prepared using an antibody to LRRK2 that allows the LRRK2 to retain protein kinase activity, for example an antibody raised or selected using a fragment of LRRK2 as discussed further below. The LRRK2 polypeptide may be, for example, a bacterially-expressed or mammalian cell-expressed LRRK2 polypeptide (for example as described in the Examples, in Jaleel et al (2007) *supra* or in PCT/GB2008/001211, *supra).* It may be appropriate to express the LRRK2 polypeptide alongside the substrate polypeptide. It may be useful for the substrate polypeptide to comprise a further portion that is considered to bind or co-localise with LRRK2, for example all or part of an ERM family polypeptide (as discussed above) or all or part of LRP130. The LRRK2 polypeptide may have the amino acid sequence of a naturally occurring LRRK2, or may be or comprise a fusion polypeptide (for example as described in the Examples or in Jaleel et al (2007) *supra* or in PCT/GB2008/001211, *supra*), or may be a fragment or variant of a naturally occurring LRRK2 that retains the ability to phosphorylate the substrate polypeptide as defined herein, for example the substrate polypeptide termed Nictide, described above, or an ERM family polypeptide or myelin basic protein, for example as described in in Jaleel et al (2007) *supra* or in PCT/GB2008/001211, *supra,* for example that retains the ability to phosphorylate denatured moesin or a fragment thereof on the residue corresponding to Thr558 (or Thr 526) of full length human moesin. Thus, the LRRK2 polypeptide is an LRRK2 polypeptide that retains an active kinase domain. It is also considered that in order to be catalytically active, the LRRK2 polypeptide retains regions corresponding to the GTPase domain, COR domain, WD40-like motif and C-terminal tail, as discussed in Jaleel et al (2007) *supra* or in PCT/GB2008/001211, *supra.* The LRRK2 polypeptide may not comprise the Leucine Rich Repeat (LRR) motif present in full length LRRK2. The LRRK2 polypeptide may comprise or consist of residues 1326-2527 of wild-type human LRRK2, or a GST fusion of such a fragment, as described in the Examples and in Jaleel et al (2007) *supra* or in PCT/GB2008/001211, *supra.* A fragment of a LRRK2 which contains the intact kinase domain and other domains indicated above but not other regions of LRRK2 (for example the Leucine Rich Repeat (LRR) motif) may be useful; this region of LRRK2 is sufficient to retain protein kinase activity but is shorter than full length LRRK2 and easier to express in an active form. The LRRK2 polypeptide used in the assay is not a kinase-dead mutant such as is described in Jaleel et al (2007) *supra* or in PCT/GB2008/001211, *supra* (for example LRRK2 in which the residue equivalent to residue D2017 of full length human LRRK2 is mutated, for example to Alanine).

Thus, the LRRK2 polypeptide can be wild type human LRRK2, a fragment thereof, or a fusion of either the wild type human LRRK2 or the fragment, wherein the fragment comprises at least residues 1326-2527 of wild type human LRRK2. It is considered that truncation at the C-terminus may adversely affect the protein kinase activity of the truncated LRRK2 polypeptide, whilst truncation at the N-terminus of the fragment may be better tolerated. Thus, the N-terminus of the truncated LRRK2 polypeptide may alternatively lie after residue 1326, for example between residue 1326 and about residue 1336.

The LRRK2 polypeptide can be human LRRK2 having a naturally occurring mutation of wild type human LRRK2; a fragment thereof; or a fusion of either the human LRRK2 having a naturally occurring mutation of wild type human LRRK2 or the fragment; wherein the fragment comprises at least residues 1326-2527 of human LRRK2 having a naturally occurring mutation.

The naturally occurring mutation of human LRRK2 may be a mutation associated with Parkinson's Disease (PD). The mutation, using the numbering of wild type human LRRK2, may be G2019S. This mutation is considered to enhance the protein kinase activity of LRRK2, as discussed further in Jaleel et al (2007) *supra* or in PCT/GB2008/001211, *supra.*

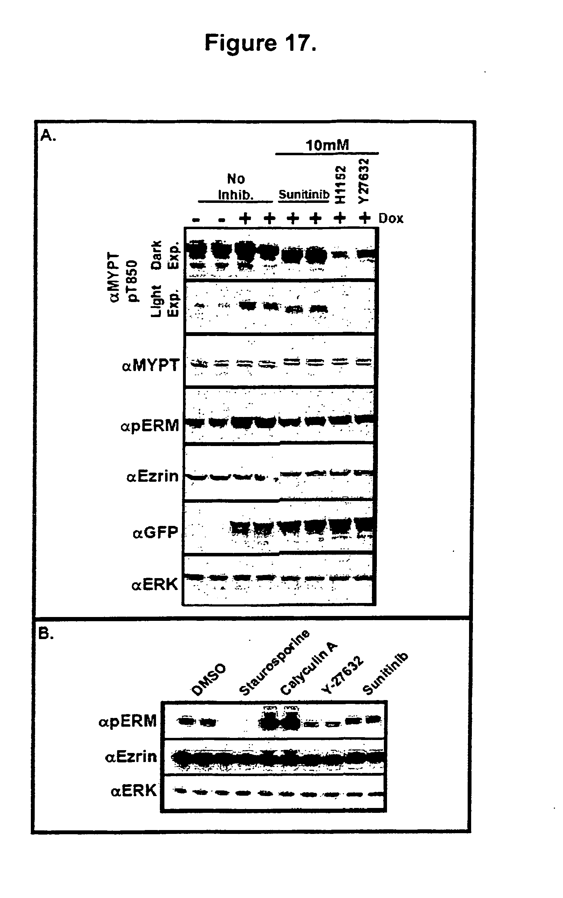

The mutation, using the numbering of wild type human LRRK2, may alternatively be R1441C, R1441G, Y1699C, R1914H, I2012T, I2020T, or G2385R. LRRK2 with mutations R1441C, R1441G, Y1699C or T2356I is considered to have similar protein kinase activity to wild-type LRRK2. LRRK2 with mutation R1914H or I2012T is considered to be nearly inactive. LRRK2 with mutation 12020T is considered to have activity intermediate between wild-type LRRK2 and LRRK2 with mutation R1914H or I2012T. LRRK2 with mutation G2385R is also considered to be nearly inactive. The activities of further mutants are shown in Figure 17 of PCT/GB2008/001211, *supra.*

The LRRK2 polypeptide can be human LRRK2 having the mutation A2016T; or a fragment thereof; or a fusion either thereof, wherein the fragment comprises at least residues 1326-2527 of human LRRK2 having the A2016T mutation. As discussed in Example 4, this mutant is considered to be resistant to inhibition by compounds such as H-1152, Y-27632 and suntinib.

It may be helpful to test compounds against more than one LRRK2 polypeptide; for example against more than one mutant LRRK2 polypeptide. This may assist in deciding on further compounds to design and test.

The LRRK2 polypeptide may be a GST fusion polypeptide, as discussed in Example 1 and in Jaleel et al (2007) *supra* or in PCT/GB2008/001211, *supra.* For example, the LRRK2 polypeptide may be GST-LRRK2[1326-2527, G2019S]. Alternative fusion moieties may also be used, as will be well known to those skilled in the art.

It is particularly preferred, although not essential, that the LRRK2 polypeptide has at least 30% of the enzyme activity of full-length human LRRK2 with respect to the phosphorylation of full-length human moesin on residue Thr558 or Thr526; or the phosphorylation of a peptide substrate encompassing such a residue (for example as discussed above; for example RLGRDKYKTLRQIRQ or RLGRDKYKTLRQIRQGNTKQR); or of the substrate polypeptide of the invention, as defined above, for example RLGWWRFYTLRRARQGNTKQR. It is more preferred if the LRRK2 polypeptide has at least 50%, preferably at least 70% and more preferably at least 90% of the enzyme activity of full-length human LRRK2 with respect to the phosphorylation of full-length human moesin on residue Thr558 or Thr526; or the phosphorylation of a peptide substrate encompassing such a residue, as discussed above; or of the substrate polypeptide of the invention as defined above, for example RLGWWRFYTLRRARQGNTKQR.

Accession numbers for mammalian LRRK2 sequences in the NCBI database include:
AAV63975.1 human
XP_001168494.1 Pan troglodytes, (chimpanzee)
XP_615760.3 Bos Taurus (domestic cow)
XP_543734.2 Canis familiaris (dog)
NP_080006.2 Mus musculus (mouse)
XP_235581.4 Rattus norvegicus (rat)

Numerous further examples of mammalian and non-mammalian LRRK2 polypeptide sequences can be accessed in the sequence databases accessible from the NCBI Medline™ service, as will be well known to the person skilled in the art.

By "variants" of a polypeptide we include insertions, deletions and substitutions, either conservative or non-conservative. In particular we include variants of the polypeptide where such changes do not substantially alter the protein kinase activity or ability to be phosphorylated, as appropriate. The skilled person will readily be able to design and test appropriate variants, based on, for example, comparison of sequences of examples of each polypeptide, for example from different species. The skilled person will readily be able to determine where insertions or deletions can be made; or which residues can appropriately be left unchanged; replaced by a conservative substitution; or replaced by a non-conservative substitution. The variant polypeptides can readily be tested, for example as described in the Examples.

By "conservative substitutions" is intended combinations such as Gly, Ala; Val, Ile, Leu; Asp, Glu; Asn, Gln; Ser, Thr; Lys, Arg; and Phe, Tyr.

The three-letter or one letter amino acid code of the IUPAC-IUB Biochemical Nomenclature Commission is used herein, with the exception of the symbol Zaa, defined above. In particular, Xaa represents any amino acid. It is preferred that at least the amino acids corresponding to the consensus sequences defined herein are L-amino acids.

It is particularly preferred if the polypeptide variant has an amino acid sequence which has at least 65% identity with the amino acid sequence of the relevant human polypeptide, more preferably at least 70%, 71%, 72%, 73% or 74%, still more preferably at least 75%, yet still more preferably at least 80%, in further preference at least 85%, in still further preference at least 90% and most preferably at least 95% or 97% identity with the amino acid sequence of the relevant human polypeptide.

It is still further preferred if a protein kinase variant has an amino acid sequence which has at least 65% identity with the amino acid sequence of the catalytic domain of the human polypeptide, more preferably at least 70%, 71%, 72%, 73% or 74%, still more preferably at least 75%, yet still more preferably at least 80%, in further preference at least 83 or 85%, in still further preference at least 90% and most preferably at least 95% or 97% identity with the relevant human amino acid sequence.

It will be appreciated that the catalytic domain of a protein kinase-related polypeptide may be readily identified by a person skilled in the art, for example using sequence comparisons as described below. Protein kinases show a conserved catalytic core, as reviewed in Johnson et al (1996) Cell, 85, 149-158 and Taylor & Radzio-Andzelm (1994) Structure 2, 345-355. This core folds into a small N-terminal lobe largely comprising antiparallel β-sheet, and a large C-terminal lobe which is mostly α-helical.

The percent sequence identity between two polypeptides may be determined using suitable computer programs, for example the GAP program of the University of Wisconsin Genetic Computing Group and it will be appreciated that percent identity is calculated in relation to polypeptides whose sequence has been aligned optimally.

The alignment may alternatively be carried out using the Clustal W program (Thompson *et al.,* 1994). The parameters used may be as follows:
Fast pairwise alignment parameters: K-tuple(word) size; 1, window size; 5, gap penalty; 3, number of top diagonals; 5. Scoring method: x percent.
Multiple alignment parameters: gap open penalty; 10, gap extension penalty; 0.05. Scoring matrix: BLOSUM.

The alignment may alternatively be carried out using the program T-Coffee [19], or EMBOSS [20], as discussed in Example 1.

The residue corresponding (equivalent) to, for example, Thr 558 of full-length human moesin may be identified by alignment of the sequence of the polypeptide with that of full-length human moesin in such a way as to maximise the match between the sequences. The alignment may be carried out by visual inspection and/or by the use of suitable computer programs, for example the GAP program of the University of Wisconsin Genetic Computing Group, which will also allow the percent identity of the polypeptides to be calculated. The Align program (Pearson (1994) in: Methods in Molecular Biology, Computer Analysis of Sequence Data, Part II (Griffin, AM and Griffin, HG eds) pp 365-389, Humana Press, Clifton). Thus, residues identified in this manner are also "corresponding residues".

It will be appreciated that in the case of truncated forms of (for example) moesin or in forms where simple replacements of amino acids have occurred it is facile to identify the "corresponding residue".

It is preferred that the polypeptides used in the screen are mammalian, preferably human (or a species useful in agriculture or as a domesticated or companion animal, for example dog, cat, horse, cow), including naturally occurring allelic variants (including splice variants). The polypeptides used in the screen may comprise a GST portion or may be biotinylated or otherwise tagged, for example with a 6His, HA, myc or other epitope tag, as known to those skilled in the art, or as described in the Examples. This may be useful in purifying and/or detecting the polypeptide(s).

The effect of the compound may be determined by comparing the rate or degree of phosphorylation of the substrate polypeptide by the LRRK2 polypeptide in the presence of different concentrations of the compound, for example in the absence and in the presence of the compound, for example at a concentration of about 100µM, 30µM, 10µM, 3µM, 1µM, 0.1µM. 0.01 µM and/or 0.001 µM.

It is considered that a compound identified by a method of the invention modulates the ability of the LRRK2 polypeptide to phosphorylate different substrates, for example moesin, radixin or ezrin or the peptide substrate RLGRDKYKTLRQIRQ or RLGRDKYKTLRQIRQGNTKQR. The extent of modulation may be different for different substrates. Thus, it may be desirable, but not essential, to test the effect of a compound identified by a method of the invention on the ability of the LRRK2 polypeptide to phosphorylate a polypeptide of interest, for example an endogenous polypeptide, for example moesin, radixin or ezrin.

The method is useful in identifying compounds that, for example, modulate, for example inhibit, the protein kinase activity of LRRK2 or the phosphorylation of an ERM family polypeptide by LRRK2. A compound that modulates, for example inhibits, the protein kinase activity of LRRK2 or the phosporylation of an ERM family polypeptide by LRRK2 may be useful in the treatment of Parkinson's Disease (for example idiopathic Parkinson's Disease or late-onset Parkinson's Disease) or Parkinsonism.

A compound that modulates, for example inhibits, the protein kinase activity of LRRK2 or the phosphorylation of an ERM family polypeptide, for example moesin, may also be useful in other neurodegenerative conditions.

The compound may be one which binds to or near a region of contact between a LRRK2 polypeptide and a substrate polypeptide, or may be one which binds to another region and, for example, induces a conformational or allosteric change which stabilises (or destabilises) the complex; or promotes (or inhibits) its formation. The compound may bind to the LRRK2 polypeptide or to the substrate polypeptide so as to increase the LRRK2 polypeptide protein kinase activity by an allosteric effect. This allosteric effect may be an allosteric effect that is involved in the natural regulation of the LRRK2 polypeptide's activity.

The compounds identified in the methods may themselves be useful as a drug or they may represent lead compounds for the design and synthesis of more efficacious compounds.

The compound may be a drug-like compound or lead compound for the development of a drug-like compound for each of the above methods of identifying a compound. It will be appreciated that the said methods may be useful as screening assays in the development of pharmaceutical compounds or drugs, as well known to those skilled in the art.

The term "drug-like compound" is well known to those skilled in the art, and may include the meaning of a compound that has characteristics that may make it suitable for use in medicine, for example as the active ingredient in a medicament. Thus, for example, a drug-like compound may be a molecule that may be synthesised by the techniques of organic chemistry, less preferably by techniques of molecular biology or biochemistry, and is preferably a small molecule, which may be of less than 5000 daltons. A drug-like compound may additionally exhibit features of selective interaction with a particular protein or proteins and be bioavailable and/or able to penetrate cellular membranes, but it will be appreciated that these features are not essential.

The term "lead compound" is similarly well known to those skilled in the art, and may include the meaning that the compound, whilst not itself suitable for use as a drug (for example because it is only weakly potent against its intended target, non-selective in its action, unstable, difficult to synthesise or has poor bioavailability) may provide a starting-point for the design of other compounds that may have more desirable characteristics.

It will be understood that it will be desirable to identify compounds that may modulate the activity of the protein kinase *in vivo.* Thus it will be understood that reagents and conditions used in the method may be chosen such that the interactions between, for example, the LRRK2 polypeptide and the substrate polypeptide, are substantially the same as between the human LRRK2 and an endogenous human substrate polypeptide, for example human ERM family polypeptide, for example moesin, radixin or ezrin polypeptide. It will be appreciated that the compound may bind to the LRRK2 polypeptide, or may bind to the substrate polypeptide.

The compounds that are tested in the screening methods of the assay or in other assays in which the ability of a compound to modulate the protein kinase activity of a protein kinase, for example an LRRK2 polypeptide, may be measured, may be (but do not have to be) compounds that have been selected and/or designed (including modified) using molecular modelling techniques, for example using computer techniques. The selected or designed compound may be synthesised (if not already synthesised) and tested for its effect on the LRRK2 polypeptide, for example its effect on the protein kinase activity. The compound may be tested in a screening method of the invention.

The compounds that are tested may be compounds that are already considered likely to be able to modulate the activity of a protein kinase; or may be compounds that have not been selected on the basis of being likely to modulate the activity of a protein kinase. Thus, the compounds tested may be compounds forming at least part of a general, unselected compound bank; or may alternatively be compounds forming at least part of a pre-selected compound bank, for example a bank of compounds pre-selected on the basis of being considered likely to modulate the activity of a protein kinase.

It will be appreciated that screening assays which are capable of high throughput operation will be particularly preferred. For example, assays using an antibody binding to the phosphorylated form of the substrate polypeptide but not the unphosphorylated form (or vice versa) may be suitable. Examples may include cell based assays and protein-protein binding assays. A further example is an SPA-based (Scintillation Proximity Assay; Amersham International) system as well known to those skilled in the art. For example, beads comprising scintillant and a substrate polypeptide, for example RLGWWRFYTLRRARQGNTKQR as discussed above may be prepared. The beads may be mixed with a sample comprising ³²P- or ³³P-γ-labelled ATP, a LRRK2 polypeptide and with the test compound. Conveniently this is done in a 96-well format. The plate is then counted using a suitable scintillation counter, using known parameters for ³²P or ³³P SPA assays. Only ³²P or ³³P that is in proximity to the scintillant, i.e. only that bound to the substrate that is bound to the beads, is detected. Variants of such an assay, for example in which the substrate polypeptide is immobilised on the scintillant beads via binding to an antibody or antibody fragment, may also be used. High throughput protein kinase activity assays are well known to those skilled in the art and can be readily adapted in view of the information provided herein on the phosphorylation of the substrate polypeptide of the invention by LRRK2 polypeptides.

The screening method may further comprise the step of assessing whether the compound modulates ERM family polypeptide, for example moesin, phosphorylation (or other parameter, for example actin binding or membrane component binding or cell characteristics, as discussed in Jaleel et al (2007) *supra* or in PCT/GB2008/001211, *supra*) in a whole cell, tissue or organism; and selecting a compound that modulates the phosphorylation (or other parameter). The compounds may be tested in whole cells, tissue or organisms that have an LRRK2 mutation linked to Parkinson's Disease, as discussed above; or that otherwise over-express LRRK2. The compounds may be tested, for example, in a neuronal cell line. Thus, the effect of the compound on phosphorylation of an ERM family polypeptide, for example moesin, may be assessed in a neuronal cell line.

As will be apparent to those skilled in the art, it may be desirable to assess what effect the compound has on other protein kinases. For example, it may be desirable to assess the effect of the compound on phosphorylation of substrates of other protein kinases, for example substrates of RockII, in order to distinguish between LRRK2 and ROCK inhibitors. For example, as shown in, for example, Figures 20 and 22 of PCT/GB2008/001211, *supra* or discussed in the legends thereto, the substrate preferences of LRRK2 and Rock-II are different. As an example, LRRK2 does not phosphorylate MYPT, while RockII does phosphorylate MYPT.

The screening method may comprise the step of comparing the effect of the test compound with the effect of a comparator compound. For example, as discussed in the Examples, sunitinib is considered to be a compound that inhibits LRRK2 but not ROCK; and Y-27632 and H-1152 are considered to be dual ROCK and LRRK2 inhibitors. A compound that inhibits LRRK2 but not ROCK may show similar effects to sunitinib.

The screening method may comprise the step of assessing whether the compound modulates the activity of LRRK2, in the whole cell, tissue or organism, and selecting a compound that modulates the activity selected. The method may further comprise the step of comparing the effect of the test compound with a comparator compound in the whole cell, tissue or organism. A compound that inhibits LRRK2 but not ROCK may show similar effects to sunitinib and may show different effects to Y-27632 and H-1152.

Information on PD models, biomarkers and assessment techniques, in/against which it may be appropriate further to test compounds identified using the screening methods described herein, can be found at, for example, the following links, which are representative of information available to those skilled in the art.
http://www.ninds.nih.gov/about ninds/plans/nihparkinsons agenda.htm#Models
http://www.sciencedaily.com/releases/2006/07/060729134653.htm (mouse model with mitochondrial disturbance)
http://www.sciencedaily.com/releases/2004/10/041005074846.htm (embryonic stem cell model)
http://en.wikipedia.org/wiki/Parkinson's disease

PD animal models include the 6-hydroxydopamine treated rodent and the MPTP treated primate. Both are based on toxic destruction of dopaminergic brain cells (and some other types), and usually employ young, otherwise healthy animals. Because these models reproduce some key features of Parkinson's disease, they are considered useful to test emerging new therapies.

Compound may also be subjected to other tests, for example toxicology or metabolism tests, as is well known to those skilled in the art.

The screening method of the invention may comprise the step of synthesising, purifying and/or formulating the selected compound.

A further aspect of the invention provides a purified preparation comprising an LRRK2 polypeptide (for example as discussed above) or polynucleotide (ie a polynucleotide encoding an LRRK2 polypeptide) or antibody capable of binding to LRRK2 obtained or obtainable by a method comprising the step of raising the antibody to, or selecting the antibody on the basis of binding to, a polypeptide consisting of residues 100 to 498, or residues 100 to 500, of LRRK2, a fragment thereof, or a fusion of either the polypeptide or the fragment, other than with an LRRK2-derived sequence; and a substrate polypeptide of the invention as defined above (or a polynucleotide encoding a substrate polypeptide of the invention); and optionally a comparator compound such as sunitinib; Y-27632 or H-1152. The preparation or kit may, for example, comprise a recombinant LRRK2 polynucleotide or polypeptide and a recombinant or chemically synthesised substrate polypeptide. The kit may further comprise an ERM family polypeptide or a fragment derivable from an ERM family polypeptide, for example moesin, radixin or ezrin, which encompasses the residue corresponding to Thr558 residue of moesin and at least part of the surrounding sequence which includes this residue, for example at least the 2, 3, 4, 5, 6 or 7 residues C-terminal and N-terminal of this residue; for example the polypeptide RLGRDKYKTLRQIRQ or RLGRDKYKTLRQIRQGNTKQR; or a polypeptide of less than 100. 80, 60, 50, 40, 30, 25, 20, 19, 18, 17 or 16 amino acids, comprising the amino acid sequence RLGRDKYK(T/S)LRQIRQ or RLGRDKYK(T/S)LRQIRQGNTKQR, each with no or up to one, two, three, four, five, six, seven, weight, nine or ten conservative or non-conservative substitutions of residues other than the T/S residue, as discussed above.

The preparation may be useful in an assay of the first, second or third aspect of the invention.

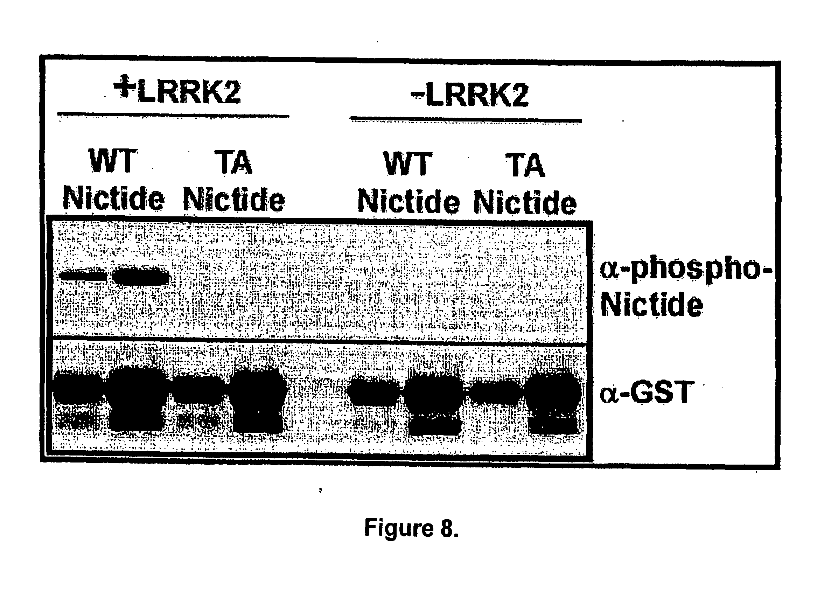

The kit may further comprise a specific binding partner, typically an antibody, that binds in a phosphorylation state-sensitive manner to an epitope encompassing the phosphorylatable residue of the substrate polypeptide of the invention. By "binding in a phosphorylation state-sensitive manner" is included the meaning that the specific binding partner is capable of binding to the epitope (or substrate polypeptide comprising the epitope) when phosphorylated on the phosphorylatable portion, but is not capable of binding to the epitope (or substrate polypeptide comprising the epitope) when it is not phosphorylated on the phosphorylatable portion of that epitope. Thus, it is preferred that the specific binding partner has at least a 5-fold, preferably 10, 20, 50, 100, 200, 500,1000, 2000 or 5000-fold difference in affinity for the phosphorylated and nonphosphorylated substrate polypeptide. In practice, a specific binding partner prepared and purified/selected using methods known in the art (see, for example, WO 03/087400; for example affinity purified using a phosphorylated peptide affinity column and a nonphosphorylated peptide affinity column) is expected to have the required affinity and specificity of binding. An example of such an antibody prepared using these techniques is described/used in Figure 8 and the legend thereto.

By the term "antibody" is included synthetic antibodies and fragments and variants (for example as discussed above) of whole antibodies which retain the antigen binding site. The antibody may be a monoclonal antibody, but may also be a polyclonal antibody preparation, a part or parts thereof (for example an F_{ab} fragment or F(ab')₂) or a synthetic antibody or part thereof. Fab, Fv, ScFv and dAb antibody fragments can all be expressed in and secreted from *E. coli*, thus allowing the facile production of large amounts of the said fragments. By "ScFv molecules" is meant molecules wherein the V_{H} and V_{L} partner domains are linked via a flexible oligopeptide. IgG class antibodies are preferred.

Suitable monoclonal antibodies to selected antigens may be prepared by known techniques, for example those disclosed in "Monoclonal Antibodies: A manual of techniques". H. Zola (CRC Press, 1988) and in "Monoclonal Hybridoma Antibodies: techniques and Applications", JGR Hurrell (CRC Press, 1982), modified as indicated above. Bispecific antibodies may be prepared by cell fusion, by reassociation of monovalent fragments or by chemical cross-linking of whole antibodies. Methods for preparing bispecific antibodies are disclosed in Corvalen et al, (1987) Cancer Immunol. Immunother. 24, 127-132 and 133-137 and 138-143.

A general review of the techniques involved in the synthesis of antibody fragments which retain their specific binding sites is to be found in Winter & Milstein (1991) Nature 349, 293-299.

By "purifed" is meant that the preparation has been at least partially separated from other components in the presence of which it has been formed, for example other components of a recombinant cell. Examples of methods of purification that may be used are described in the Examples or in Jaleel et al (2007) *supra* or in PCT/GB2008/001211, *supra.*

The preparation may be substantially pure. By "substantially pure" we mean that the said polypeptide(s) are substantially free of other proteins. Thus, we include any composition that includes at least 2, 3, 4, 5, 10, 15, 20 or 30% of the protein content by weight as the said polypeptides, preferably at least 50%, more preferably at least 70%, still more preferably at least 90% and most preferably at least 95% of the protein content is the said polypeptides.

Thus, the invention also includes compositions comprising the said polypeptides and a contaminant wherein the contaminant comprises less than 96, 95, 94, 90, 85, 80 or 70% of the composition by weight, preferably less than 50% of the composition, more preferably less than 30% of the composition, still more preferably less than 10% of the composition and most preferably less than 5% of the composition by weight.

The invention also includes the substantially pure said polypeptides when combined with other components *ex vivo,* said other components not being all of the components found in the cell in which said polypeptides are found.

A further aspect of the invention provides a recombinant cell capable of expressing a LRRK2 polypeptide and a substrate polypeptide according to the invention, comprising a recombinant LRRK2 polynucleotide and a recombinant substrate polypeptide polynucleotide. The substrate polypeptide may comprise a tag or a further portion considered to bind to or co-localise with LRRK2, for example an ERM family polypeptide or fragment, as discussed above. The cell may be capable of overexpressing the LRRK2 polypeptide from the endogenous sequence encoding the said polypeptide, for example using techniques of sequence-specific targeting of transcription activators. Thus the cell may be modified in a way intended to lead to increased expression of the LRRK2 polypeptide relative to a cell which has not been so modified. The cell may be a prokaryotic or eukaryotic cell. For example it may be a eukaryotic cell, for example an insect, yeast or mammalian cell, for example a human cell. Examples of suitable cells are described, for example, in the Examples or in Jaleel et al (2007) *supra* or in PCT/GB2008/001211, *supra.*

The recombinant nucleic acid is preferably suitable for expressing the encoded polypeptide. The recombinant nuclei acid may be in the form of an expression vector. Recombinant polynucleotides suitable for expressing a given polypeptide are well known to those skilled in the art, and examples are described in the Examples and in Jaleel et al (2007) *supra* or in PCT/GB2008/001211, *supra.*

The cell may comprise at least 1.1, 1.2, 1.5, 2, 3, 5, 10 or 20-fold more LRRK2 polypeptide than an equivalent cell which has not been modified in order to overexpress the LRRK2 polypeptide or to express the recombinant LRRK2 polypeptide.

By "suitable for expressing" is mean that the polynucleotide is a polynucleotide that may be translated to form the polypeptide, for example RNA, or that the polynucleotide (which is preferably DNA) encoding the polypeptide of the invention is inserted into an expression vector, such as a plasmid, in proper orientation and correct reading frame for expression. The polynucleotide may be linked to the appropriate transcriptional and translational regulatory control nucleotide sequences recognised by any desired host; such controls may be incorporated in the expression vector.

Characteristics of vectors suitable for replication in mammalian/eukaryotic cells are well known to those skilled in the art, and examples are given below. It will be appreciated that a vector may be suitable for replication in both prokaryotic and eukaryotic cells.

A variety of methods have been developed to operably link polynucleotides, especially DNA, to vectors for example via complementary cohesive termini. Suitable methods are described in Sambrook et al (1989) Molecular Cloning, A Laboratory Manual, Cold Spring Harbor Laboratory, Cold Spring Harbor, NY.

A desirable way to modify the DNA encoding a polypeptide of the invention is to use the polymerase chain reaction as disclosed by Saiki et al (1988) Science 239, 487-491. This method may be used for introducing the DNA into a suitable vector, for example by engineering in suitable restriction sites, or it may be used to modify the DNA in other useful ways as is known in the art.

In this method the DNA to be enzymatically amplified is flanked by two specific primers which themselves become incorporated into the amplified DNA. The said specific primers may contain restriction endonuclease recognition sites which can be used for cloning into expression vectors using methods known in the art.

The DNA (or in the case of retroviral vectors, RNA) is then expressed in a suitable host to produce a polypeptide comprising the compound of the invention. Thus, the DNA encoding the polypeptide constituting the compound of the invention may be used in accordance with known techniques, appropriately modified in view of the teachings contained herein, to construct an expression vector, which is then used to transform an appropriate host cell for the expression and production of the polypeptide of the invention. Such techniques include those disclosed in US Patent Nos. 4,440,859 issued 3 April 1984 to Rutter et al*,* 4,530,901 issued 23 July 1985 to Weissman, 4,582,800 issued 15 April 1986 to Crowl, 4,677,063 issued 30 June 1987 to Mark et al*,* 4,678,751 issued 7 July 1987 to Goeddel, 4,704,362 issued 3 November 1987 to Itakura et al, 4,710,463 issued 1 December 1987 to Murray, 4,757,006 issued 12 July 1988 to Toole, Jr. et al*,* 4,766,075 issued 23 August 1988 to Goeddel et al and 4,810,648 issued 7 March 1989 to Stalker.

The DNA (or in the case of retroviral vectors, RNA) encoding the polypeptide may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognised by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance. Alternatively, the gene for such selectable trait can be on another vector, which is used to co-transform the desired host cell.

Host cells that have been transformed by the recombinant DNA of the invention are then cultured for a sufficient time and under appropriate conditions known to those skilled in the art in view of the teachings disclosed herein to permit the expression of the polypeptide, which can then be recovered.

Many expression systems are known, including bacteria (for example *E. coli* and *Bacillus subtilis),* yeasts (for example *Saccharomyces cerevisiae*), filamentous fungi (for example *Aspergillus),* plant cells, animal cells and insect cells.

The vectors include a prokaryotic replicon, such as the CoiE1 *ori,* for propagation in a prokaryote, even if the vector is to be used for expression in other, non-prokaryotic, cell types. The vectors can also include an appropriate promoter such as a prokaryotic promoter capable of directing the expression (transcription and translation) of the genes in a bacterial host cell, such as *E. coli,* transformed therewith.

A promoter is an expression control element formed by a DNA sequence that permits binding of RNA polymerase and transcription to occur. Promoter sequences compatible with exemplary bacterial hosts are typically provided in plasmid vectors containing convenient restriction sites for insertion of a DNA segment of the present invention.

Typical prokaryotic vector plasmids are pUC18, pUC19, pBR322 and pBR329 available from Biorad Laboratories, (Richmond, CA, USA) and pTrc99A and pKK223-3 available from Pharmacia, Piscataway, NJ, USA.

A typical mammalian cell vector plasmid is pSVL available from Pharmacia, Piscataway, NJ, USA. This vector uses the SV40 late promoter to drive expression of cloned genes, the highest level of expression being found in T antigen-producing cells, such as COS-1 cells.

An example of an inducible mammalian expression vector is pMSG, also available from Pharmacia. This vector uses the glucocorticoid-inducible promoter of the mouse mammary tumour virus long terminal repeat to drive expression of the cloned gene.

Useful yeast plasmid vectors are pRS403-406 and pRS413-416 and are generally available from Stratagene Cloning Systems, La Jolla, CA 92037, USA. Plasmids pRS403, pRS404, pRS405 and pRS406 are Yeast Integrating plasmids (YIps) and incorporate the yeast selectable markers *HIS3, TRP1, LEU2* and *URA3*. Plasmids pRS413-416 are Yeast Centromere plasmids (YCps).

The host cell can be either prokaryotic or eukaryotic. Bacterial cells are preferred prokaryotic host cells and typically are a strain of *E. coli* such as, for example, the *E*. *coli* strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include yeast, insect and mammalian cells, preferably vertebrate cells such as those from a mouse, rat, monkey or human fibroblastic cell line. Yeast host cells include YPH499, YPH500 and YPH501 which are generally available from Stratagene Cloning Systems, La Jolla, CA 92037, USA. Preferred mammalian host cells include human embryonic kidney 293 cells (see Example 1), Chinese hamster ovary (CHO) cells available from the ATCC as CCL61, NIH Swiss mouse embryo cells NIH/3T3 available from the ATCC as CRL 1658, and monkey kidney-derived COS-1 cells available from the ATCC as CRL 1650. Preferred insect cells are Sf9 cells which can be transfected with baculovirus expression vectors.

Transformation of appropriate cell hosts with a DNA construct is accomplished by well known methods that typically depend on the type of vector used. With regard to transformation of prokaryotic host cells, see, for example, Cohen et al (1972) Proc. Natl. Acad. Sci. USA 69, 2110 and Sambrook et al (1989) Molecular Cloning, A Laboratory Manual, Cold Spring Harbor Laboratory, Cold Spring Harbor, NY. Transformation of yeast cells is described in Sherman et al (1986) Methods In Yeast Genetics, A Laboratory Manual, Cold Spring Harbor, NY. The method of Beggs (1978) Nature 275, 104-109 is also useful. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA.

Electroporation is also useful for transforming and/or transfecting cells and is well known in the art for transforming yeast cell, bacterial cells, insect cells and vertebrate cells.

For example, many bacterial species may be transformed by the methods described in Luchansky et al (1988) Mol. Microbiol 2, 637-646. The greatest number of transformants is consistently recovered following electroporation of the DNA-cell mixture suspended in 2.5X PEB using 6250V per cm at 25:FD.

Methods for transformation of yeast by electroporation are disclosed in Becker & Guarente (1990) Methods Enzymol. 194, 182.

Successfully transformed cells, ie cells that contain a DNA construct of the present invention, can be identified by well known techniques. For example, cells resulting from the introduction of an expression construct of the present invention can be grown to produce the polypeptide of the invention. Cells can be harvested and lysed and their DNA content examined for the presence of the DNA using a method such as that described by Southern (1975) J. Mol. Biol. 98, 503 or Berent et al (1985) Biotech. 3, 208.

In addition to directly assaying for the presence of recombinant DNA, successful transformation can be confirmed by well known immunological methods when the recombinant DNA is capable of directing the expression of the protein. For example, cells successfully transformed with an expression vector produce proteins displaying appropriate antigenicity. Samples of cells suspected of being transformed are harvested and assayed for the protein using suitable antibodies.

Thus, in addition to the transformed host cells themselves, the present invention also contemplates a culture of those cells, preferably a monoclonal (clonally homogeneous) culture, or a culture derived from a monoclonal culture, in a nutrient medium.

An appropriate method for making the preparation of the invention, may comprise the step of purifying the preparation from a cell according to the invention. Methods of cultivating host cells and isolating recombinant proteins are well known in the art. Examples of suitable purification techniques are described in the Examples or in Jaleel et al (2007) *supra* or in PCT/GB2008/001211, *supra.* For example, one or more component of the preparation may be tagged so as to aid purification using affinity reagents, as will be well known to those skilled in the art and as described in the Examples or in Jaleel et al (2007) *supra* or in PCT/GB2008/001211, *supra.* Chromatographic techniques may also be used, for example as described in the Examples or in Jaleel et al (2007) *supra* or in PCT/GB2008/001211, *supra.*

The method of the first, second or third aspect of the invention may be performed with the LRRK2 polypeptide and substrate polypeptide in the form of a preparation of the invention; or in a cell of the invention.

The above polypeptides may be made by methods well known in the art and as described below and in the Examples, for example using molecular biology methods or automated chemical peptide synthesis methods.

It will be appreciated that peptidomimetic compounds may also be useful. Thus, by 'polypeptide" or "peptide" we include not only molecules in which amino acid residues are joined by peptide (-CO-NH-) linkages but also molecules in which the peptide bond is reversed. Such retro-inverso peptidomimetics may be made using methods known in the art, for example such as those described in MϑziΠre et al (1997) J. Immunol. 159, 3230-3237. This approach involves making pseudopeptides containing changes involving the backbone, and not the orientation of side chains. Meziere et al (1997) show that, at least for MHC class II and T helper cell responses, these pseudopeptides are useful. Retro-inverse peptides, which contain NH-CO bonds instead of CO-NH peptide bonds, are much more resistant to proteolysis.

Similarly, the peptide bond may be dispensed with altogether provided that an appropriate linker moiety which retains the spacing between the CI atoms of the amino acid residues is used; it is particularly preferred if the linker moiety has substantially the same charge distribution and substantially the same planarity as a peptide bond.

It will be appreciated that the peptide may conveniently be blocked at its N- or C-terminus so as to help reduce susceptibility to exoproteolytic digestion.

Thus, it will be appreciated that the LRRK2 or, more preferably, the substrate polypeptide may be a peptidomimetic compound.

A further aspect of the invention provides a polypeptide comprising the sequence (W/F/R/K)(W/F/R/K)(R/K)(F/W/H/R)(Y/W/R)(/T)(L/V/I)(R/K)(R/K)(A/Y) or (W/R)(X)(X)(F/Y/H)(Y/W/R)(T)(X)(R/T)(R)(X), where X represents any amino acid. Such a polypeptide is considered to be a substrate for LRRK2, as discussed above. Preferences for the substrate polypeptide are as indicated above.

A further aspect of the invention provides a polynucleotide encoding the polypeptide of the invention.

The polynucleotide may be a vector suitable for replication and/or expression of the polypeptide in a mammalian/eukaryotic cell. A still further aspect of the invention is a recombinant polynucleotide suitable for expressing a polypeptide of the invention. Typically the recombinant polynucleotide comprises a polynucleotide encoding the polypeptide of the invention.

The polynucleotide or recombinant polynucleotide may be DNA or RNA, preferably DNA. The polynucleotide may or may not contain introns in the coding sequence; preferably the polynucleotide is or comprises a cDNA.

The invention is now described in more detail by reference to the following, non-limiting, Figures and Examples.

### Figure Legends.

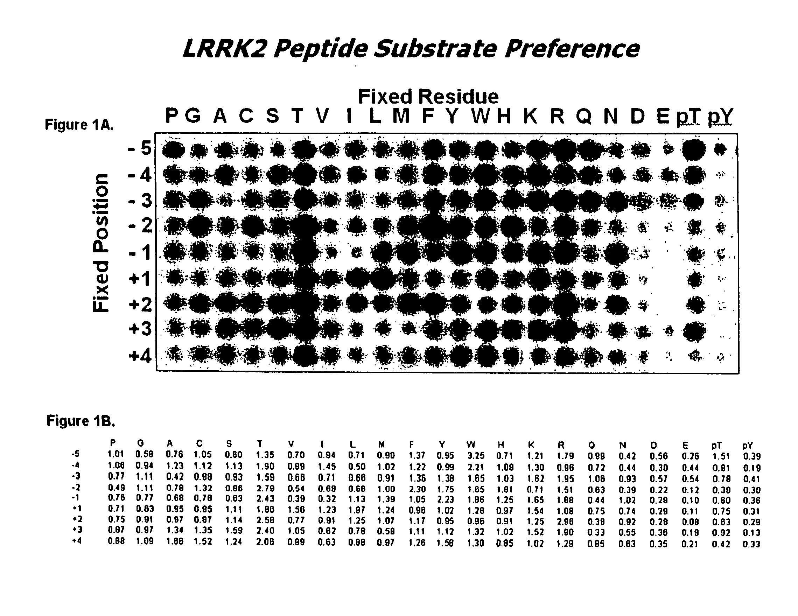

Figure 1. GST tagged, 1326-End recombinant LRRK2 G2019S was purified from transfected HEK293 cells as described in Jaleel et al., Biochemistry 2007. This enzyme was used to determine the peptide substrate preference as described in Hutti et al., Nat Methods. 2004 by the Laboratory of Lewis Cantley, Harvard Medical School. Residue preferences were derived from digital quantitation of reaction products and values are shown in Figure 1B. Control reactions were performed with a kinase dead preparation (Figure 2).

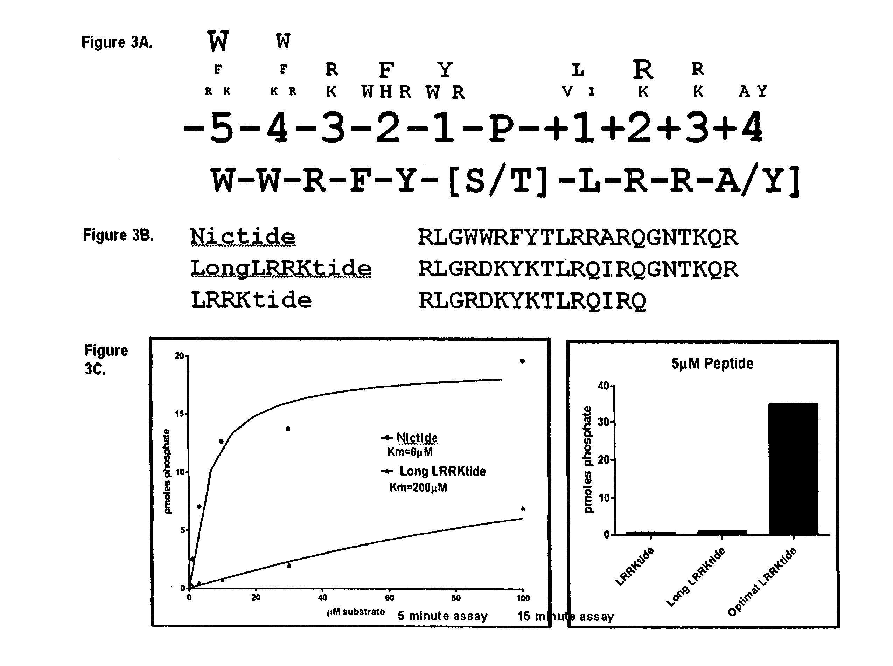

Figure 3. In Figure 3A, LRRK2 residue preferences are displayed semi-quantitatively by proportional representation of single letter amino acid symbols above the position relative to phosphorylation site. A derived consensus sequences is shown below the positions. Figure 3B is an alignment of the peptide substrate sequences previously used for LRRK2, LRRKtide and LongLRRKtide, along with a new peptide in which the preferred LRRK2 residues are substituted, henceforth referred to as Nictide. The left panel of Figure 3C shows concentration dependent phosphorylation of Nictide versus LongLRRKtide at 5 minute reaction times. Calculated Km values are 6micromolar for Nictide. The right panel of Figure 3C shows a comparison of LRRKtide, LongLRRKtide and Nictide at 15 minutes at 5 micromolar peptide concentration.

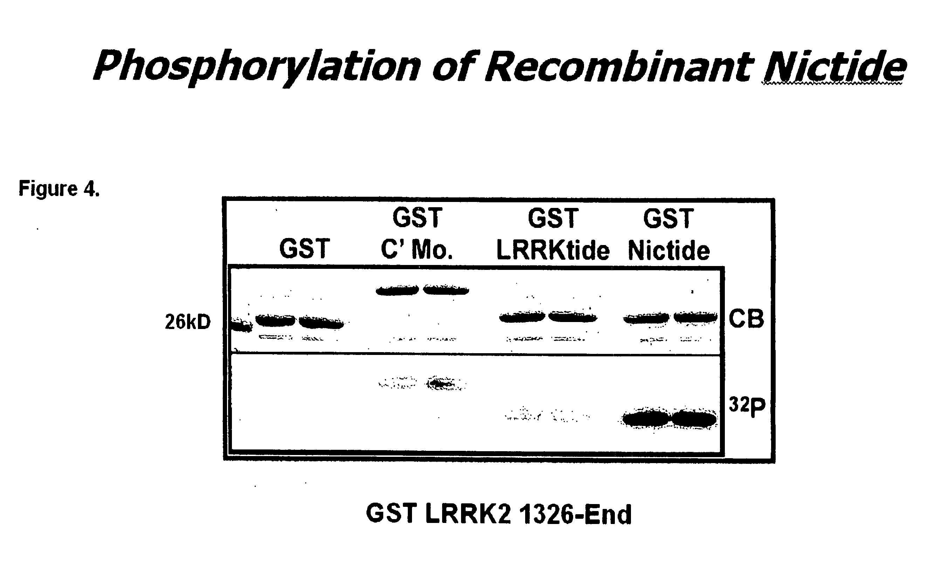

Figure 4. Modification of Nictide diplayed as a fusion to a recombinant protein. GST or fusion proteins of GST with the carboxy terminus of moesin (amino acids 500-End), the sequence of LRRKtide or Nictide were prepared from bacteria. These recombinant proteins were presented as substrates for GST tagged LRRK2 1326-End G2019S for 10 minutes in the presence of [γ-³²P]-ATP. Reaction products were resolved on 12% SDS-polyacrylamide gels and visualized by coomassie blue staining (CB) and autoradiography (³²P).

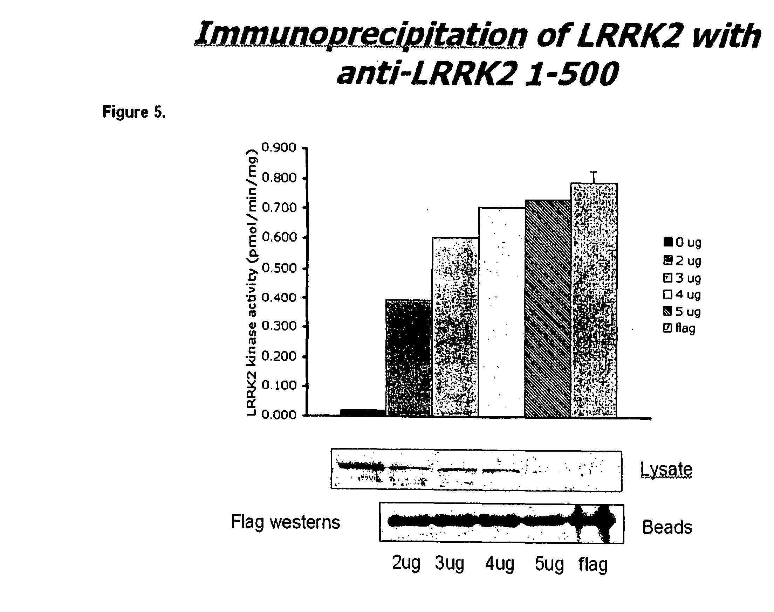

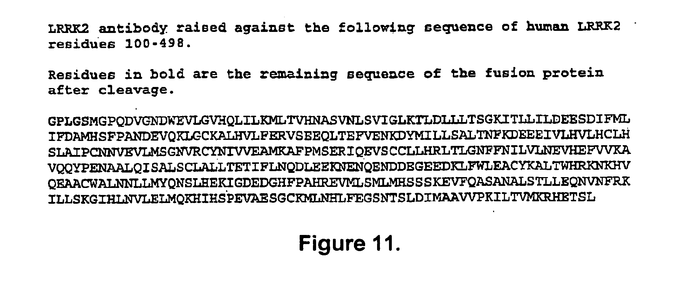

Figure 5. An antibody was raised in sheep against the amino terminal 500 amino acids of LRRK2 using recombinant protein as an immunogen (see Figure 11). Specific antibodies were isolated by positive selection with recombinant protein. 1mg of lysates of HEK293 cells stably expressing full length Flag tagged LRRK2 G2019S were subjected to immunoprecipition with increasing amounts of antibody conjugated to 10µl of protein G sepharose and anti-FLAG M2 agarose (Sigma) as control. Immunocomplexes were used in kinase assay with LongLRRKtide. Immunodepletion of LRRK2 from the lysates is shown as well as immunoblot analysis of the immunoprecipitates using anti-flag.

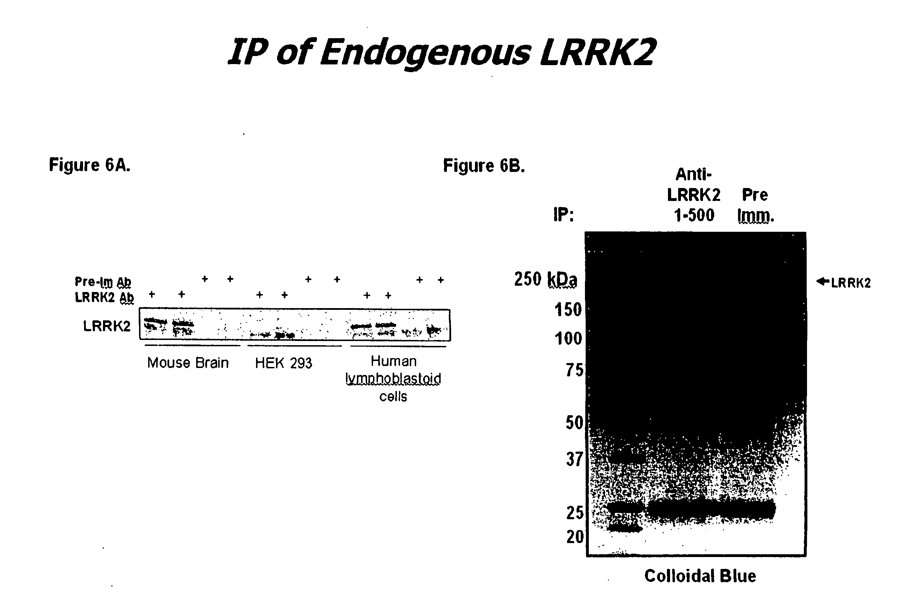

Figure 6. The anti-LRRK2 1-500 antibody immunoprecipitates endogenous protein from mouse brain lysate, and human lymphoblastoid lystates, Figure 6A. The LRRK2 antibody was used in an immunoprecipitation of 13mg of NIH3T3 lysate. The immunoprecipitates were eluted with sample buffer and resolved on a 4-12% Novex gel and stained with colloidal blue, Figure 6B. Bands 1 and 2 were excised and subjected to analysis by mass spectrometry. Band contained 16 peptide matches for LRRK2, while band 2 contained no peptides matching LRRK2.

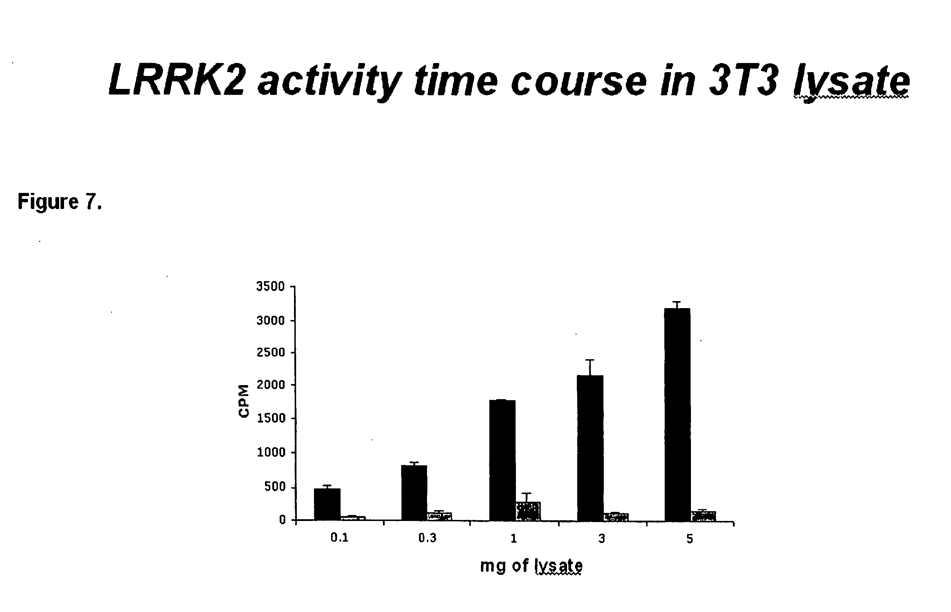

Figure 7. Increasing amounts of NIH3T3 lysate were used to immunoprecipitate LRRK2 and were assayed for activity against Nictide. Anti-LRRK2 reactions are represented in black bars and pre-immune reactions are in grey bars.
Figure 8. Phosphorylation of Nictide assessed using an antibody raised against a phospho-Nictide antigen. GST fusion proteins with either the wild-type Nictide sequence or the phosphorylation site mutated to alanine were produced in bacteria (GST-RLGWWRFYTLRRARQGNTKQR or GST-RLGWWRFYALRRARQGNTKQR). These proteins were subjected to kinase reactions containing LRRK2 (293 cell expressed GST tagged 1326-END G2019S) or buffer. A titration of these reactions containing 66 or 330 ng of the GST fusion was probed with an antibody raised against a phospho-Nictide antigen. Immunoblots were performed in the presence of 10ug/ml dephospho Nictide.

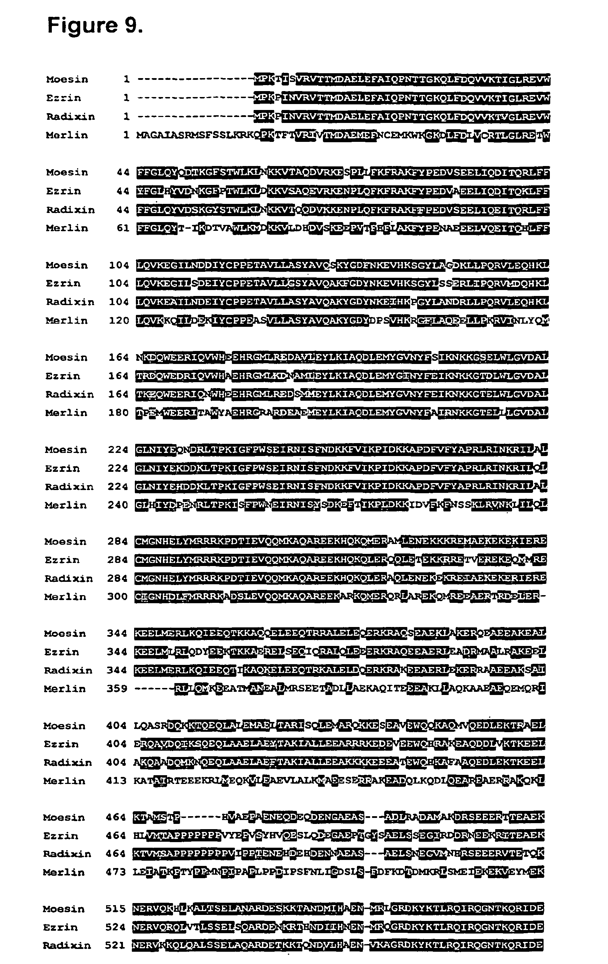

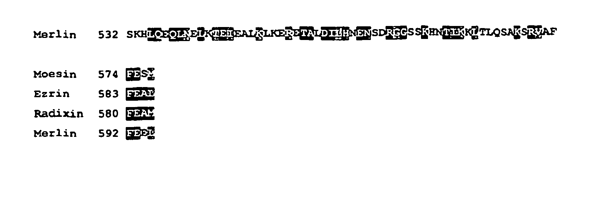

Figure 9: human ERM family polypeptide sequence alignment.

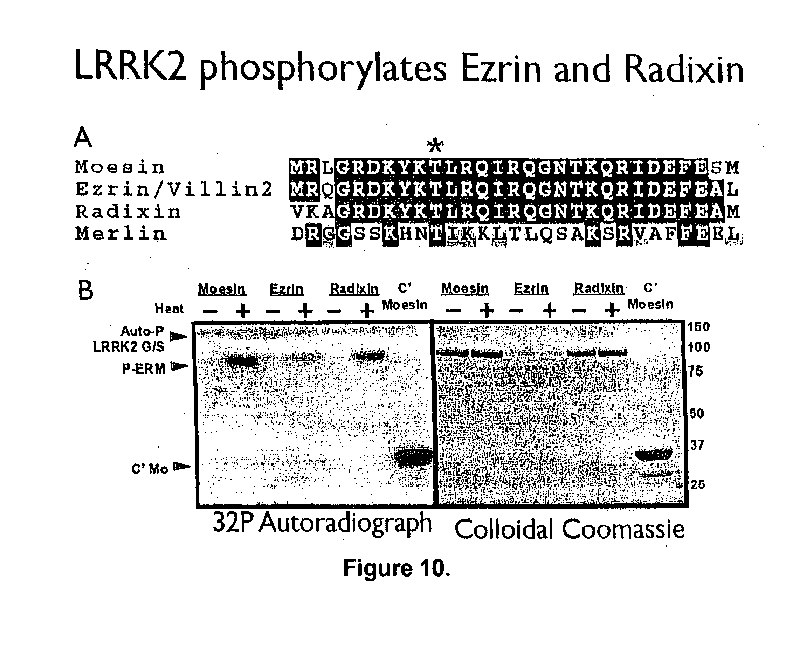

Figure 10: Analysis of phosphorylation of moesin by LRRK2 (A) Sequence alignment of the C-terminal regions of Ezrin, Radixin and Merlin. The asterisks indicate the Thr residue equivalent to Thr555 on moesin. Black and grey shaded residues represent identical and homologous residues, respectively.
Figure 11: Fusion of human LRRK2 residues 100-498 used in raising an antibody useful in binding to and in immunoprecipitating LRRK2.

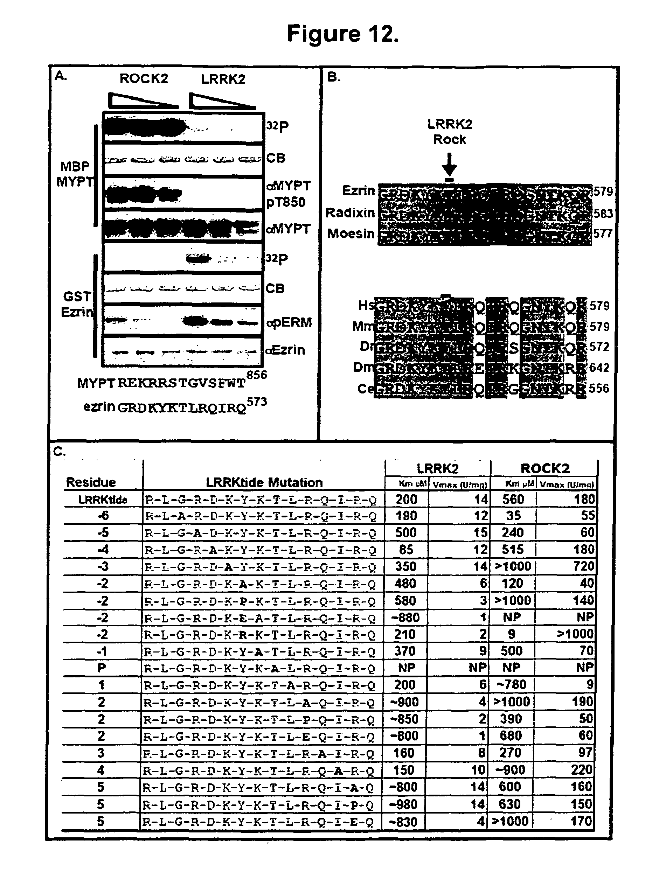

Figure 12 Comparison of ROCK substrates as substrates for LRRK2 (A-upper panel) ROCK2 [2-543] and LRRK2 [1326-2527] were diluted to a concentration where they possessed identical activity towards LRRKtide and then incubated with 2 µM MBP-MYPT [714-1004] or heat treated GST-Ezrin [full-length] [9] in the presence of Mg[γ-³²P]-ATP. Enzyme was decreased successively two fold, represented by decreasing slope in triangle. Reactions were terminated after 15 min by addition of sample buffer and products were subjected to SDS-PAGE. Gels were analysed by staining with colloidal blue (CB) and phosphorylation was monitored by autoradiography. Immunoblotting analysis was also undertaken with the indicated antibodies. (**A**-lower panel) Comparison of the amino acid sequences surrounding Thr850-MYPT and Thr567-ezrin showing little similarity between peptides. **(B)** Sequence alignments of the indicated species of ERM proteins surrounding the ROCK/LRRK2 phosphorylation site. Identical residues are shaded. The accession sequences used were: human moesin [accession no. NP_002435] human ezrin [accession no. NP_03370] human radixin [accession no. NP_002897]. Mus musculus [accession no. NP_034963], Danio rerio [accession no. NP_001004296], Drosophila melanogaster (Dm) [accession no. NP_727290], Caenorhabditis elegans (Ce) [accession no. NP491550]. **(C)** Analysis of substrate recognition determinants in LRRKtide. Residues from -6 to -5 of the LRRKtide substrate (RLGRDKYKTLRQIRQ) were mutated to the residue indicated in bold lettering. These peptides were analyzed for their ability to be phosphorylated by GSTLRRK2 1326-2527 [G2019S] purified from HEK293 cells or ROCK2 (amino acids 2-543) purified from baculovirus. NP denotes that the peptide was phosphorylated so poorly that kinetic analysis was not feasible. For Kₘ values above 500 µM an "∼" sign is added to stress that these values were inferred from kinetic analysis undertaken at peptide concentrations of lower than 1 mM. Similar results were obtained in at least two experiments.

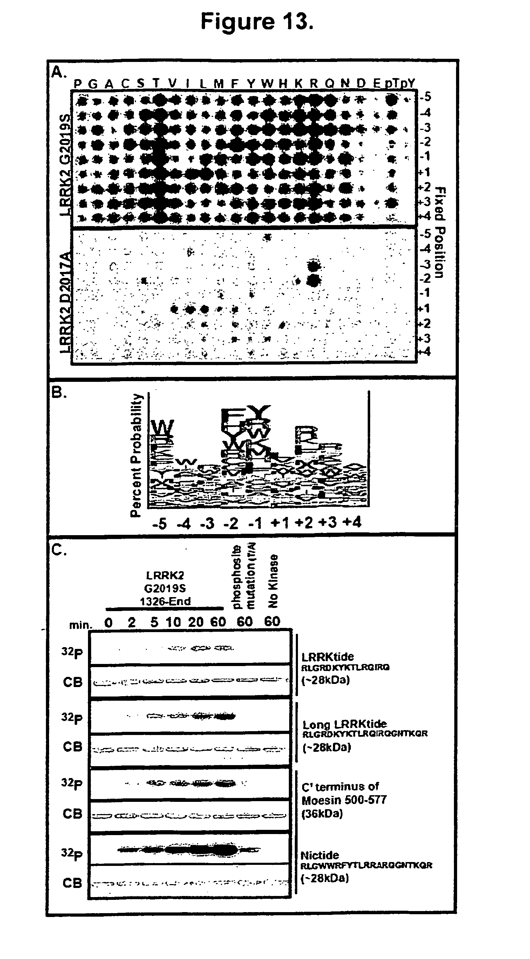

Figure 13. Determination of the preferred substrate phosphorylation sequence for LRRK2. **(A)** Recombinant HEK293 purified LRRK2 (1326-2527) [G2019S] enzyme and LRRK2 (1326-2527) [D2017A] was used to screen a positional scanning peptide library consisting of 189 biotinylated-peptide libraries in individual kinase assays. Reaction products were bound to streptavidin coated membrane and after washing, phosphorylation was visualised by phosphorimaging. **(B)** Logo of LRRK2 phosphorylation site was derived from empirical data from **(A)** input into enologos ®. The height of the stack of single amino acid letters indicates the entropy of the site and the size of each letter indicates its preference at the position relative to the phosphorylation site between -5 and +4. The largest letters at each position in the logo were chosen to substitute for residues in a longer version of the LRRKtide substrate peptide to derive Nictide, shown below the logo. **(C)** GST fusion proteins with the indicated peptide sequences of LRRKtide, the longer LRRKtide, the carboxy terminus of moesin (500-577) and the Nictide substrates were subjected to phosphorylation by HEK293 purified LRRK2 (1326-2527) [G2019S] the indicated times. Reactions were stopped by the addition of sample buffer and products were subjected to SDS-PAGE. Gels were analysed by staining with colloidal blue (CB) and phosphorylation was monitored by autoradiography (³²P). Similar results were obtained in replicate experiments.

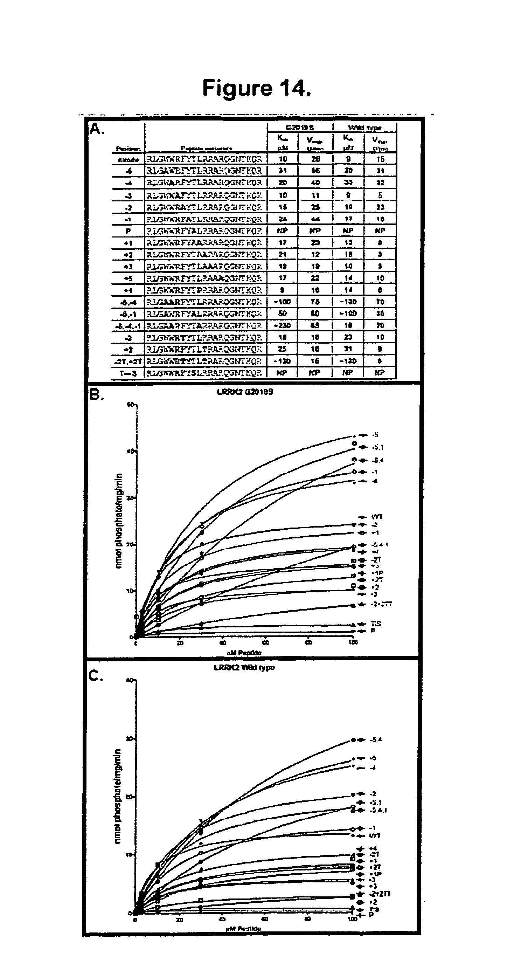

Figure 14. Kinetic analysis of the Nictide Substrate. **(A)**. Residues from -5 to +5 of the Nictide substrate (RLGWWRFYTLRRARQGNTKQR) were mutated to the residue indicated in bold lettering. These peptides were analyzed for their ability to be phosphorylated by GST-LRRK2 1326-2527 [G2019S] or GST-LRRK2 1326-2527 [wild type] purified from HEK293 cells and *K*m and *V*max values were derived by nonlinear regression analysis as described in Materials and Methods. Similar results were obtained in at least three experiments. **(B).** Average values from a representative experiment from which data in A were derived for GST-LRRK2 1326-2527 [G2019S] **(C)**. As in **B.** except with or GST-LRRK2 1326-2527 [wild type].

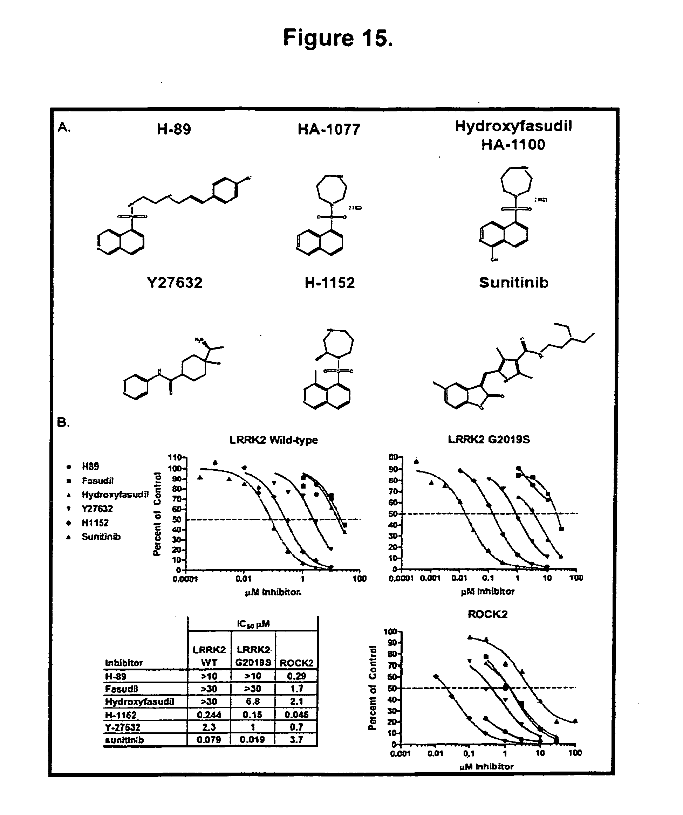

Figure 15. Characterization of ROCK inhibitors as LRRK2 inhibitors. **(A).** Panels 1-8 show the chemical structures of the inhibitors utilized in this study. **(B)** LRRK2 1326-2527 [wild type] and [G2019S] and ROCK2 [2-543] were assayed in the presence or absence of the indicated concentration of the indicated inhibitor, in the presence of 100 µM ATP. The results are presented as percentage of kinase activity relative to the control measured in the presence of vehicle control. Results are the average of at least duplicate reactions where similar results were observed in at least one other experiment. The inhibitors are represented as follows: H89 by black line and circles; fasudil by grey line squares; hydroxyfasudil by grey line and triangles; Y-27632 by grey line and inverted triangles; H-1152 by grey diamonds; and sunitinib by grey triangles. **(C)** The IC50 values derived from the graphs in **(B)** are shown in µM and displayed in tabular format.

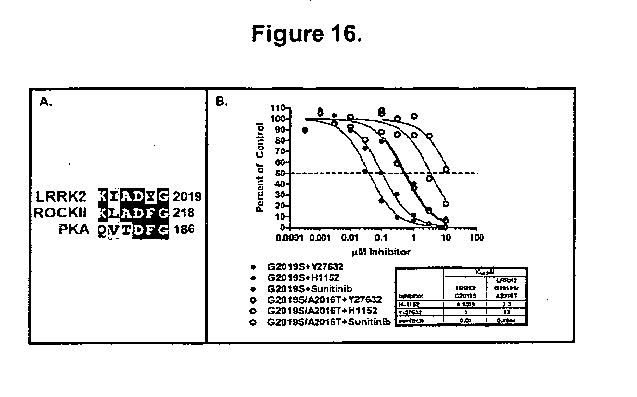

Figure 16. Design of an LRRK2 inhibitor desensitized mutant. **(A)** Sequence alignment of the amino acids surrounding the Asp of subdomain VII for LRRK2, Protein Kinase A (PKA) and ROCK2. Identical amino acids are shaded in black and similar amino acids are shaded in grey. Terminal amino acid residues are numbered. **(C)** LRRK2 (1326-2527) [G2019S] and LRRK2 (1326-2527) [G2019S/A2016T] were assayed in the presence or absence of the indicated concentration of the inhibitor, in the presence of 100 µM ATP and expressed as a percent of control reactions performed in the presence of vehicle alone. LRRK2 (1326-2527) [G2019S] reactions are represented by filled circles and LRRK2 (1326-2527) [G2019S/A2016T] reactions are represented by open circles. Colour scheme is the same as in Figure 15B.
Figure 17. Testing the efficacy of LRRK2 and ROCK inhibitors in vivo. **(A)** Flp-in T-REx cells that harbor GFP tagged constitutively active G14V-Rho were either left uninduced or induced by the inclusion of 1 □g/ml doxycycline in the culture medium. At 7 hours post induction, Cells were treated with 10uM H-1152 and Y-27632 for 1 h. Cells were lysed in direct SDS-Lysis Buffer and resolved on 4-12% Novex gels and subjected to immunoblot analysis with the indicated antibodies. **(B)** As in **(A)** except that HEK 293 cells were used..

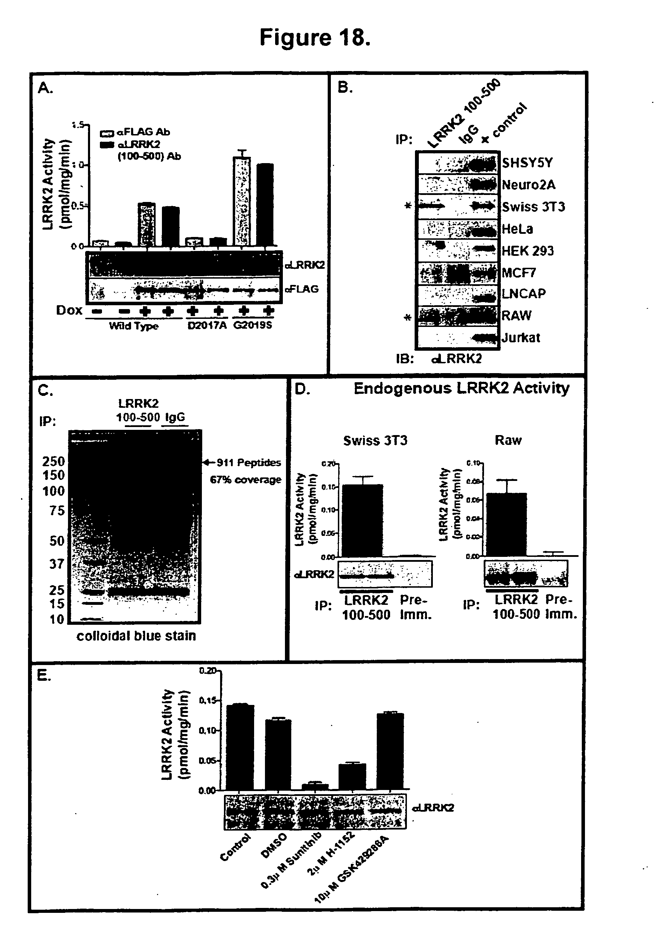

Figure 18. Analysis of Endogenous LRRK2 **(A)** Doxycycline inducible HEK 293 cells overexpressing full length flag-tagged human LRRK2 and LRRK2 containing D2017A and G2019S mutations were generated as described in material and methods. Anti-LRRK2 [100-500] S348C antibody was used to retrieve recombinant protein. Precipitates were assayed for kinase activity using LRRKtide and were immunoblotted with anti-FLAG and S374C anti-LRRK2 **(B)** Extracts of the indicated cell lines were screened for the presence of LRRK2 protein following immunoprecipitation with the indicated antibodies and immunoblotting with the LRRK2 [2498-2514] antibody. **(C)** anti-LRRK2 [100-500] and control IgG were used in immunoprecipitations of 60 mg Swiss 3T3 lysate. Specific bands corresponding to the predicted molecular weight of LRRK2 were excised and tryptic peptides were identified by mass spectrometry. **(D)** Endogenous LRRK2 kinase activity from S348C anti-LRRK2 [100-500] immunoprecipitates was measured against the Nictide substrate in Swiss 3T3 and RAW cells following immunoprecipitation. **(E)** Immunoprecipitates from Swiss-3T3 cell lines were assayed against Nictide substrate in the presence of the indicated concentrations of the indicated inhibitors. Kinase assays of immunecomplexes were carried out in triplicate and are representative of at least two seperate experiments.

**Table 1. Kinase profiling of LRRK2 and ROCK inhibitors.** Results are presented as percentage of kinase activity compared to control incubations in which inhibitor was omitted. Protein kinases were assayed as described at the material and methods section. The results are an average of a triplicate determination ± standard deviation. Abbreviations not defined in main text: AMPK, AMP-activated protein kinase BRSK, brain-specific kinase; BTK, Bruton's tyrosine kinase; CaMK1, calmodulin-dependent kinase; CaMKK, CaMK kinase; CDK, cyclin-dependent kinase; CHK, checkpoint kinase; CK1, casein kinase 1; CSK, Cterminal Src kinase; DYRK, dual-specificity tyrosine-phosphorylated and regulated kinase; ERK, mitogen activated protein kinase; EF2K, elongation-factor-2 kinase; EPH, ephrin; FGF-R, fibroblast growth factor receptor; GCK, germinal center kinase; GSK3, glycogen synthase kinase 3; HIPK, homeodomain-interacting protein kinase; HER4, V-erb a erythroblastic leukemia viral oncogene homolog 1; IRAK, Interleukin-1 Receptor-Associated Kinase 4; IGF1R, IGF1 receptor, IKK, inhibitory □B kinase; IR, insulin receptor; IRR, insulin-related receptor; JNK, c-Jun N-terminal kinase; Lck, lymphocyte cell-specific protein tyrosine kinase; MAPKAP-K, MAPK-activated protein kinase; MARK, microtubule-affinityregulating kinase; MELK, maternal embryonic leucine-zipper kinase; MKK1, MAPK kinase-1; MLCK, smooth muscle myosin light-chain kinase; MNK, MAPK-integrating protein kinase; MLK, mixed lineage kinase; MINK, Misshapen-like Kinase; MSK, mitogen- and stress-activated protein kinase; MST, mammalian homologue Ste20-like kinase; NEK, NIMA (never in mitosis in Aspergillus nidulans)-related kinase; NUAK1, SNF1 like kinase1; PAK, p21-activated protein kinase; PHK, phosphorylase kinase; PIM, provirus integration site for Moloney murine leukaemia virus; PKA, cAMP-dependent protein kinase; PDK1, 3-phosphoinositide-dependent protein kinase-1; PKB, protein kinase B; PKC, protein kinase C; PKD, protein kinase D; PLK, polo-like kinase; PRAK, p38-regulated activated kinase; PRK, protein kinase C-related kinase; RSK, ribosomal S6 kinase; S6K, p70 ribosomal S6 kinase; SGK1, serum and glucocorticoid kinase 1; SRPK, serine-arginine protein kinase; SYK, spleen tyrosine kinase; TBK1, TANK-binding kinase 1; TTK, tau-tubulin kinase; VEGFR, vascular endothelial growth factor receptor; YES1, Yamaguchi sarcoma viral oncogene homologue 1. n.d., not determined.

**Table I**

| | HA1100 10µM | HA-1077 10µM | H-1162 1µM | Y27632 10µM | Sunitinib 0.1µM | 1µM |
|---|---|---|---|---|---|---|
| LRRK2 wild type | 72 = 11 | 77 ± 13 | 20 ± 3 | 22 ± 2 | 42 ± 2 | 9 ± 0 |
| LRRK2 G2019S | 28 ± 1 | 67 ± 3 | 13 ± 1 | 11 ± 1 | 14 ± 1 | 3 ± 0 |
| ROCK 2 | 7 ± 2 | 3 ± 1 | 3 ± 0 | 4 ± 1 | 82 ± 2 | 67 ± 6 |
| MKK1 | 83 ± 3 | 41 ± 2 | 69 ± 15 | 71 ± 17 | 37 ± 2 | 16 ± 1 |
| ERK1 | 109 ± 6 | 104 ± 2 | 105 ± 8 | 82 ± 1 | 108 ± 2 | 104 ± 7 |
| ERK2 | 102 ± 8 | 88 ± 2 | 94 ± 4 | 84 ± 2 | 101 ± 1 | 103 ± 10 |
| JNK1 | 105 ± 8 | 115 ± 8 | 96 ± 5 | 108 ± 6 | 123 ± 16 | 104 ± 5 |
| JNK2 | 90 ± 5 | 94 ± 4 | 91 ± 6 | 96 ± 9 | 101 ± 8 | 88 ± 1 |
| p38a MAPK | 98 ± 1 | 108 ± 13 | 109 ± 11 | 103 ± 4 | 111 ± 2 | 109 ± 0 |
| p38bMAPK | 90 ± 6 | 111 ± 2 | 107 ± 2 | 101 ± 10 | 116 ± 0 | 113 ± 4 |
| p38g MAPK | 97 ± 9 | 99 ± 5 | 101 ± 18 | 96 ± 13 | 109 ± 21 | 86 ± 18 |
| p38d MAPK | 100 ± 4 | 77 ± 1 | 93 ± 4 | 86 ± 1 | 110 ± 2 | 107 ± 1 |
| ERK8 | 76 ± 1 | 88 ± 44 | 81 ± 11 | 83 ± 6 | 85 ± 1 | 59 ± 4 |
| RSK1 | 11 ± 15 | 14 ± 5 | 57 ± 1 | 26 ± 3 | 38 ± 53 | 76 ± 9 |
| RSK2 | 41 ± 6 | 31 ± 1 | 76 ± 1 | 42 ± 5 | 80 ± 23 | 41 ± 5 |
| PDK1 | 112 ± 4 | 97 ± 1 | 74 ± 5 | 120 ± 9 | 124 ± 2 | 95 ± 1 |
| PKBa | 27 ± 7 | 27 ± 1 | 64 ± 8 | 56 ± 14 | 87 ± 10 | 73 ± 6 |
| PKBb | 100 ± 3 | 77 ± 11 | 112 ± 8 | 105 ± 1 | 101 ± 4 | 88 ± 2 |
| SGKJ | 55 ± 13 | 89 ± 21 | 82 ± 8 | 50 ± 8 | 79 ± 26 | 26 ± 5 |
| S6K1 | 11 ± 1 | 13 ± 0 | 72 ± 2 | 70 ± 11 | 82 ± 4 | 41 ± 3 |
| PKA | 89 ± 10 | 21 ± 0 | 76 ± 5 | 106 ± 16 | 104 ± 3 | 87 ± 3 |
| PRK2 | 12 ± 0 | 6 ± 0 | 70 ± 68 | 1 ± 3 | 88 ± 3 | 81 ± 17 |
| PKCa | 64 ± 5 | 39 ± 6 | 70 ± 11 | 52 ± 1 | 83 ± 13 | 77 ± 9 |
| PKCz | 84 ± 8 | 66 ± 2 | 88 ± 6 | 68 ± 2 | 110 ± 1 | 94 ± 6 |
| PKD1 | 60 ± 3 | 25 ± 1 | 79 ± 18 | 86 ± 0 | 79 ± 7 | 34 ± 5 |
| MSK1 | 17 ± 0 | 14 ± 1 | 39 ± 4 | 41 ± 8 | 94 ± 12 | 60 ± 8 |
| MNK1 | 76 ± 8 | 20 ± 3 | 50 ± 6 | 20 ± 10 | 99 ± 10 | 83 ± 6 |
| MNK2 | 77 ± 4 | 27 ± 6 | 49 ± 8 | 44 ± 2 | 83 ± 17 | 80 ± 7 |
| MAPKAP-K2 | 86 ± 9 | 91 ± 9 | 93 ± 0 | 85 ± 4 | 91 ± 17 | 85 ± 5 |
| PRAK | 88 ± 16 | 83 ± 12 | 87 ± 17 | 86 ± 14 | 102 ± 1 | 75 ± 15 |
| CAMKKb | 93 ± 3 | 95 ± 5 | 81 ± 6 | 102 ± 8 | 87 ± 12 | 39 ± 6 |
| CAMK1 | 108 ± 14 | 109 ± 4 | 105 ± 2 | 85 ± 22 | 113 ± 19 | 66 ± 2 |
| SmMLCK | 86 ± 3 | 66 ± 6 | 96 ± 5 | 70 ± 1 | 49 ± 2 | 26 ± 3 |
| PHK | 89 ± 4 | 46 ± 6 | 26 ± 2 | 79 ± 1 | 11 ± 1 | 2 ± 0 |
| CHK1 | 87 ± 1 | 103 ± 6 | 89 ± 14 | 85 ± 9 | 76 ± 0 | 33 ± 3 |
| CHK2 | 89 ± 13 | 34 ± 2 | 44 ± 0 | 93 ± 7 | 23 ± 1 | 5 ± 1 |
| GSK3b | 91 ± 11 | 91 ± 5 | 72 ± 28 | 92 ± 3 | 108 ± 3 | 93 ± 11 |
| CDK2-Cyclin A | 99 ± 5 | 82 ± 6 | 74 ± 7 | 72 ± 4 | 98 ± 2 | 87 ± 5 |
| PLKJ | 101 ± 8 | 111 ± 14 | 105 ± 1 | 108 ± 16 | 103 ± 9 | 108 ± 10 |
| Aurora B | 59 ± 1 | 51 ± 1 | 11 ± 2 | 79 ± 1 | 72 ± 5 | 29 ± 4 |
| AMPK | 72 ± 2 | 60 ± 1 | 45 ± 4 | 77 ± 4 | 51 ± 3 | 15 ± 4 |
| MARK3 | 84 ± 2 | 79 ± 2 | 56 ± 3 | 105 ± 5 | 77 ± 4 | 36 ± 4 |
| BRSK2 | 65 ± 3 | 38 ± 2 | 17 ± 4 | 43 ± 4 | 94 ± 0 | 40 ± 6 |
| MELK | 76 ± 2 | 19 ± 1 | 70 ± 4 | 79 ± 2 | 55 ± 6 | 17 ± 1 |
| CK1 | 100 ± 0 | 77 ± 1 | 112 ± 9 | 107 ± 12 | 50 ± 2 | 10 ± 0 |
| CK2 | 84 ± 4 | 81 ± 9 | 84 ± 17 | 85 ± 2 | 91 ± 2 | 72 ± 1 |
| DYRK1A | 86 ± 16 | 102 ± 3 | 96 ± 6 | 86 ± 3 | 103 ± 6 | 83 ± 8 |
| DYRK2 | 89 ± 1 | 80 ± 2 | 84 ± 2 | 82 ± 5 | 92 ± 8 | 76 ± 9 |
| DYRK3 | 99 ± 2 | 39 ± 6 | 94 ± 0 | 100 ± 6 | 104 ± 5 | 84 ± 3 |
| NEK2a | 103 ± 2 | 98 ± 9 | 99 ± 3 | 104 ± 0 | 103 ± 12 | 87 ± 6 |
| NEK6 | 95 ± 8 | 107 ± 28 | 99 ± 18 | 80 ± 18 | 95 ± 26 | 83 ± 15 |
| IKKb | 86 ± 6 | 79 ± 4 | 100 ± 8 | 79 ± 4 | 80 ± 9 | 80 ± 4 |
| PIM1 | 90 ± 14 | 84 ±14 | 90 ± 6 | 94 ± 12 | 95 ± 5 | 86 ± 14 |
| PIM2 | 104 ± 1 | 111 ± 3 | 91 ± 7 | 102 ± 3 | 111 ± 7 | 104 ± 2 |
| PIM3 | 75 ± 1 | 81 ± 1 | 98 ± 1 | 87 ± 2 | 84 ± 1 | 50 ± 6 |
| SRPKI | 90 ± 12 | 82 ± 10 | 125 ± 1 | 65 ± 35 | 80 ± 0 | 86 ± 4 |
| MST2 | 44 ± 4 | 51 ± 2 | 41 ± 2 | 81 ± 8 | 59 ± 7 | 16 ± 2 |
| EF2K | 103 ± 14 | 120 ± 4 | 99 ± 24 | 81 ± 2 | 97 ± 15 | 106 ± 26 |
| HIPK2 | 109 ± 3 | 127 ± 6 | 100 ± 0 | 104 ± 1 | 79 ± 2 | 30 ± 1 |
| PAK4 | 104 ± 4 | 99 ± 17 | 89 ± 7 | 101 ± 23 | 99 ± 9 | 72 ± 5 |
| PAK5 | 113 ± 7 | 112 ± 16 | 100 ± 1 | 101 ± 11 | 121 ± 4 | 99 ± 3 |
| PAK6 | 103 ± 5 | 110 ± 8 | 103 ± 7 | 98 ± 7 | 115 ± 8 | 102 ± 10 |
| MST4 | 82 ± 2 | 79 ± 8 | 90 ± 7 | 87 ± 7 | 87 ± 5 | 80 ± 6 |
| TBK1 | 98 ± 5 | 110 ± 3 | 98 ± 7 | 69 ± 9 | 106 ± 9 | 54 ± 1 |
| IKKe | 89 ± 2 | 85 ± 8 | 80 ± 4 | 78 ± 8 | 101 ± 1 | 63 ± 3 |
| GCK | 73 ± 7 | 55 ± 4 | 56 ± 1 | 89 ± 4 | 45 ± 2 | 11 ± 1 |
| IRAK4 | 87 ± 4 | 89 ± 7 | 102 ± 7 | 101 ± 8 | 76 ± 19 | 23 ± 4 |
| NUAK1 | 36 ± 11 | 21 ± 2 | 51 ± 10 | 106 ± 1 | 30 ± 1 | 15 ± 3 |
| MLK1 | 75 ± 16 | 66 ± 5 | 82 ± 18 | 90 ± 19 | 74 ± 2 | 40 ± 0 |
| MINK1 | 68 ± 21 | 64 ± 6 | 104 ± 5 | 81 ± 0 | 46 ± 1 | 8 ± 0 |
| MLK3 | 45 ± 60 | 72 ± 8 | 85 ± 9 | 110 ± 3 | 72 ± 8 | 26 ± 1 |
| LKB1 | 50 ± 0 | 33 ± 0 | 50 ± 3 | 62 ± 12 | 59 ± 1 | 62 ± 2 |
| HER4 | 97 ± 5 | 128 ± 4 | 107 ± 2 | 69 ± 8 | 126 ± 14 | 103 ± 9 |
| TTX | 82 ± 2 | 73 ± 5 | 93 ± 9 | 73 ± 2 | 99 ± 5 | 72 ± 6 |
| Src | 111 ± 2 | 120 ± 8 | 81 ± 0 | 90 ± 4 | 98 ± 7 | 52 ± 1 |
| Lck | 79 ± 29 | 109 ± 7 | 74 ± 1 | 83 ± 32 | 51 ± 4 | 11 ± 2 |
| CSK | 95 ± 7 | 104 ± 12 | 101 ± 4 | 104 ± 14 | 98 ± 10 88 ± 2 | |
| FGF-R1 | 83 ± 2 | 65 ± 5 | 35 ± 3 | 95 ± 8 | 77 ± 2 | 29 ± 0 |
| IRR | 89 ± 1 | 86 ± 4 | 88 ± 2 | 91 ± 1 | 90 ± 1 | 77 ± 24 |
| EPE A2 | 95 ± 8 | 94 ± 6 | 49 ± 5 | 106 ± 4 | 104 ± 3 | 81 ± 9 |
| SYK | 112 ± 11 | 104 ± 1 | 104 ± 2 | 94 ± 8 | 107 ± 13 | 84 ±10 |
| YES1 | 11.1 ± 4 | 136 ± 8 | 76 ± 8 | 96 ± 5 | 30 ± 3 | 7 ± 1 |
| IGF-1R | 91 ± 8 | 92 ± 1 | 113 ± 2 | 88 ± 6 | 84 ± 1 | 33 ± 8 |
| VEG-FR | 115 ± 1 | 70 ± 4 | 82 ± 20 | 105 ± 4 | 33 ± 0 | 9 ± 0 |
| BTK | 108 ± 20 | 112 ± 21 | 82 ± 9 | 85 ± 17 | 80 ± 1 | 52 ± 12 |
| IR-HIS | 100 ± 4 | 105 ± 1 | 108 ± 8 | 113 ± 1 | 104 ± 7 | 65 ± 5 |
| EPH-B3 | 111 ± 2 | 65 ± 9 | 69 ± 11 | 83 ± 12 | 107 ± 8 | 103 ± 0 |

### Example 1

Methods are described in the Figure Legends.

### General immunoprecipitation and kinase assay protocol.

### 1) Harvest cells by scraping in lysis buffer (500µ1 for a 10cm dish)

Lysis buffer
50mM Tris pH 7.5
1 % Triton X-100
1 mM NaV
5mM sodium pyrophosphate
50 mM NaF
0.27 M Sucrose
1 mM EGTA
1 mM EDTA

Add reducing agent (0.1% β-mercaptoethanol) and inhibitors (1mM Benzamidine, 1mM PMSF) before use.
2) Centrifuge lysates 13000 rpm for 25 min at 4 °C and retain supernatant.
3) Perform protein assay.
4) IP from 1-2mg total protein lysate using 5µg LRRK2 100-500 antibody coupled to protein G sepharose (10ul of 50% slurry per sample).
5) Mix lysates and antibody bound beads for 2hr at 4 °C.
6) Spin down beads (13000 rpm 1 min) and remove lysate.
7) Resuspend beads in 500µl lysis buffer with the addition of 0.5M NaCl.
8) Spin down beads, remove buffer and repeat wash once more with lysis buffer plus 0.5M NaCl then twice more with lysis buffer with no NaCl then once more with 1X kinase assay buffer.
9) Spin down and remove all supernatant. For western blot add 20µl SDS PAGE sample buffer to beads, heat for 10 min at 70°C and run on gel.
10) For kinase assay resuspend beads in 50 µl kinase assay buffer and incubate for 20 min at 30°C.
11) Spot 40 µl of the kinase buffer onto 1.5cm square whatman p81 paper and place into 50 mM phosphoric acid to terminate the kinase reaction.
12) Wash p81 papers 3 x 15min in 50 mM phosphoric acid. Dry and count.

### Kinase Assay Buffer

50 mM Tris HCL pH 7.5
0.1 mM EGTA
10 mM MgCl
0.1 mM ³²P ATP (approx 300 cpm/pmol)
0.1% B-mercaptoethanol
20 uM Nictide substrate

Materials and Methods.

Materials. Protease-inhibitor cocktail tablets were obtained from Roche; P81 phosphocellulose paper was from Whatman; [γ32P]-ATP and all protein chromatography media were purchased from Amersham Biosciences. Myelin basic protein (MBP) was from Invitrogen, Precast SDS polyacrylamide Bis-Tris gels were from Invitrogen; tissue culture reagents were from Life Technologies; Millipore Immobilon-P was from Fisher Scientific. Active rat ROCKII [residues 2-543] was expressed in baculovirus by the Division of Signal Transduction Therapy Unit (University of Dundee). The LRRKtide peptide (RLGRDKYKTLRQIRQ) was synthesised by Dr Graham Bloomberg at the University of Bristol.

Antibodies. The anti-GST was raised in sheep against the glutathione S-transferase protein. The secondary antibodies coupled to horseradish peroxidase used for immunoblotting were obtained from Pierce.

General methods. Tissue culture, transfection, immunoblotting, restriction enzyme digests, DNA ligations, and other recombinant DNA procedures were performed using standard protocols. All mutagenesis was carried out using the Quick-Change site-directed mutagenesis method (Stratagene). DNA constructs used for transfection were purified from E.coli DH5α using Qiagen plasmid Mega or Maxi kit according to the manufacturer's protocol. All DNA constructs were verified by DNA sequencing, which was performed by The Sequencing Service, School of Life Sciences, University of Dundee, Scotland, UK, using DYEnamic ET terminator chemistry (Amersham Biosciences) on Applied Biosystems automated DNA sequencers.

Buffers. Lysis Buffer contained 50 mM Tris/HCl pH 7.5, 1 mM EGTA, 1 mM EDTA, 1 % (w/v) Triton-X100, 1 mM sodium orthovanadate, 10 mM sodium-β-glycerophosphate, 50 mM sodium fluoride, 5 mM sodium pyrophosphate, 0.27 M sucrose, 0.1 % (v/v) 2-mercaptoethanol and complete proteinase inhibitor cocktail (one tablet/50 ml, Boehringer). Buffer A contained 50 mM Tris/HCl pH 7.5, 0.1 mM EGTA and 0.1 % (v/v) 2-mercaptoethanol. Extraction Buffer contained 50 mM Tris/HCl pH 7.5, 5% (v/v) glycerol, 10 mM 2-mercaptoethanol, 1 mM EDTA, 1 mM EGTA, 0.03% (v/v) Brij-35, complete proteinase inhibitor cocktail (one tablet/50 ml). Sample Buffer was 1X NuPAGE® LDS sample buffer (Invitrogen) containing 1% (by vol) 2-mercaptoethanol.

Plasmids. A full-length cDNA clone encoding LRRK2 corresponding to NCBI Acc. AAV63975 was a generous gift from Dr Michel Goedert (LMB Cambridge). The full length and the fragments of LRRK2 gene that were utilized in this study were amplified from the LRRK2 cDNA fragment, according to standard PCR methods, using KOD polymerase (Novagen). The resulting PCR products were subcloned into mammalian pEBG2T and pCMV5 expression vectors as Bamh1-Not1 fragments. A cDNA encoding full-length as well as C-terminal fragments of human moesin (NCBI Acc. NP_002435) were amplified by PCR from an EST ordered from Geneservice (IMAGE clone 4908580). The PCR product was ligated into different expression vectors as Not1-Not1 fragments.

Expression and purification of GST-LRRK2. Typically 10 to100 ten cm diameter dishes of HEK 293 cells, were cultured and each dish transfected with 5 µg of the pEBG-2T construct encoding wild type or different mutant forms of LRRK2 using the polyethylenimine method [15]. The cells were cultured for a further 36 h and lysed in 0.5 ml of ice-cold lysis buffer, the lysates pooled and centrifuged at 4°C for 10 min at 26,000 x g. The GST-fusion proteins were purified by affinity chromatography on glutathione-Sepharose (10 µl per dish of 293 cells) and were eluted in Buffer A containing 20 mM glutathione and 0.27 M sucrose. The enzyme was snap frozen in small aliquots and stored at -80 °C.

Expression and purification of human moesin in E. coli. The pGEX expression constructs encoding wild type and mutant forms of human moesin were transformed into E.coli BL21 cells and 1-litre cultures were grown at 37°C in Luria Broth containing 100 µg/ml ampicillin until the absorbance at 600 nm was 0.8. Induction of protein expression was carried out by adding 100 µM isopropyl-β-D-galactoside and the cells were cultured for a further 16 hr at 26°C. Cells were isolated by centrifugation, resuspended in 15 ml of ice-cold Lysis Buffer and lysed in one round of freeze/thawing, followed by sonication to fragment DNA. The lysates were centrifuged at 4°C for 30 min at 26,000 x g, and the recombinant proteins were affinity purified on 0.2 ml of glutathione-Sepharose and were eluted in 0.4 ml of Buffer A containing 20 mM glutathione and 0.27 M sucrose.

Mapping the sites on Moesin phosphorylated by the G2019S LRRK2. Moesin (4 µg) was treated at 65 °C for 15 min and then incubated at 30 °C with 1.5 µg of GST-LRRK2[1326-2527, G2019S] in Buffer A containing 10 mM MgCl₂ and 100 µM [γ³²P]-ATP (10000 cpm/pmol) in a total reaction volume of 50 µl. The reaction was terminated after 40 min by adding Sample Buffer to a final concentration of 1% (w/v) LDS-10 mM dithiothreitol (DTT) and the samples heated at 100 °C for 1 min and cooled on ice. 4-vinylpyridine was added to a concentration of 50 mM, and the sample was left on a shaking platform for 30 min at room temperature to alkylate cysteine residues. The samples were subjected to electrophoresis on a BisTris 4-12% polyacrylamide gel, which was stained with colloidal blue and then autoradiographed. The phosphorylated moesin band was excised, cut into smaller pieces, washed sequentially for 15 min on a vibrating platform with 1 ml of the following: water, a 1:1 mixture of water and acetonitrile, 0.1 M ammonium bicarbonate, a 1:1 mixture of 0.2 M ammonium bicarbonate and acetonitrile and finally acetonitrile. The gel pieces were dried by speedi-vac and incubated in 0.1 ml of 50 mM ammonium bicarbonate, 0.1 % (w/v) n-octyl-glucoside containing 1 µg of mass spectroscopy grade trypsin (Promega). After 16 h, 0.1 ml of acetonitrile was added and the mixture incubated on a shaking platform for 10 min. The supernatant was removed and the gel pieces were further washed for 10 min in 0.3 ml of 50 mM ammonium bicarbonate, and 0.1% v/v trifluoroacetic acid. The combined supernatants, containing >90% of the 32P-radioactivity, were chromatographed on a Vydac 218TP5215 C18 column (Separations Group, Hesperia, CA) equilibrated in 0.1% v/v trifluoroacetic acid in water. The column was developed with a linear acetonitrile gradient (diagonal line) at a flow rate of 0.2 ml/min and fractions of 0.1 ml were collected. Phosphopeptides were further purified by Immobilised Metal-chelate Affinity Chromatography (IMAC) on Phospho-Select resin (Sigma). Phosphopeptide sequence analysis. Isolated phosphopeptides were analysed on an Applied Biosystems 4700 Proteomics Analyser (MALDI-TOF-TOF) using 5 µg/ml alpha cyannocinnamic acid as the matrix. Spectra were acquired in both reflectron and linear modes and the sequence of phosphopeptides were confirmed by performing MALDI-MS/MS on selected masses. The characteristic loss of phosphoric acid (M-98 Da) from the parent phosphopeptide as well as the neutral loss of dehydroalanine (M-69 kDa) for phosphoserine or dehydroaminobutyric acid (-83) for phosphothreonine was used to assign the position of the phosphorylation site(s). The site of phosphorylation of all the 32P-labelled peptides was determined by solid-phase Edman degradation on an Applied Biosystems 494C sequenator of the peptide coupled to Sequelon-AA membrane (Milligen) as described previously [16].

Assay of LRRK2 using moesin or MBP as substrates. Assays were set up in a total volume of 25 µl of Buffer A containing 0.5-0.7 µg of either wild type or mutant forms of LRRK2, 1 µM moesin (full length or indicated mutants, that had been left on ice or incubated at 65 °C for 15 min prior to assay) or 1 µM myelin basic protein, 10 mM MgCl2 and 0.1 mM [γ32P]-ATP (300 cpm/pmol). After incubation for 30 min at 30 °C, the reactions were stopped by the addition of LDS-Sample Buffer. The incorporation of phosphate into moesin or MBP substrates as well as LRRK2 autophosphorylation was determined after electrophoresis of samples on a 4-12 %-polyacrylamide gels and autoradiography of the dried Coomassie Blue-stained gels. The phosphorylated substrates were also excised from the gel and 32P-incorporation quantified by Cherenkov counting.

Assay of LRRK2 using LRRKtide as substrate. Assays were set up in a total volume of 50 µl of Buffer A containing 0.5-0.7 µg of either wild type or mutant forms LRRK2, 10 mM MgCl₂ and 0.1 mM [γ³²P]-ATP (300 cpm/pmol) in the presence of 300 µM or the indicated concentration of LRRKtide (RLGRDKYKTLRQIRQ) peptide substrate. After incubation for 30 min at 30 °C, reactions were terminated by applying 40 µl of the reaction mixture onto P81 phosphocellulose paper and phosphorylation of LRRKtide was quantified following washing the P81 phosphocellulose in 50 mM phosphoric acid and Cherenkov counting. One Unit (U) of LRRK2 activity was defined as the amount of enzyme that catalysed the incorporation of 1 nmol of ³²P into LRRKtide. Kₘ and Vₘₐₓ parameters were determined by performing the assay described above using varying concentration of LRRKtide. The Kₘ and Vₘₐₓ parameters were calculated using the Graph-Pad prism programme. Immunoblotting. Samples were heated at 70° C for 5 min in Sample Buffer, subjected to polyacrylamide gel electrophoresis and transferred to a nitrocellulose membrane. Membranes were blocked for 30 min in 50 mM Tris/HCl pH 7.5, 0.15 M NaCl, 0.2% (v/v) Tween (TBST Buffer) containing 10% (w/v) skimmed milk. The membranes were probed with 1 µg/ml of anti-GST antibody for 16 h at 4°C in TBST Buffer containing 5% (w/v) skimmed milk. Detection was performed using horseradish peroxidase conjugated secondary antibodies and the enhanced chemiluminescence reagent.

### Example 2: Assay formats suitable for compound screening

Protein kinase screening assay formats known in the art may be used, adapted in view of the identification of substrate polypeptides of the invention as substrates of LRRK2 polypeptides.

For example, the techniques used in Example 1 or in PCT/GB2008/001211 may be used in screening compounds. Assays similar to those described WO 03/087400 may be used. Screening assays which are capable of high throughput operation may be used. For example, assays using a substrate polypeptide of the invention, for example using an antibody binding to the phosphorylated form of the peptide but not the unphosphorylated for (or vice versa) may be suitable.

Cell based assays may be used, for example when assessing the effect of compounds on cell volume responses.

Protein-protein binding assays may be used, for example using surface plasmon resonance-based techniques or chip-based binding assays, as well known to those skilled in the art.

SPA-based (Scintillation Proximity Assay; Amersham International) assays may be used as well known to those skilled in the art For example, beads comprising scintillant and a substrate polypeptide of the invention may be prepared. The beads may be mixed with a sample comprising ³²P- or ³³P-γ-labelled ATP, a LRRK2 polypeptide and with the test compound. Conveniently this is done in a 96-swell format. The plate is then counted using a suitable scintillation counter, using known parameters for ³²P or ³³P SPA assays. Only ³²P or ³³P that is in proximity to the scintillant, i.e. only that bound to the substrate that is bound to the beads, is detected. Variants of such an assay, for example in which the substrate polypeptide is immobilised on the scintillant beads via binding to an antibody or antibody fragment, may also be used.

A non-radioactive assay, suitable for screening of small drug-like compound libraries, in an ELISA format can be used.

Anti-phospho-peptide antibodies can be raised in sheep, for example for use in western blotting. They are evaluated for use in the ELISA format, Immobilization of the substrate polypeptide to a microtitre plate, eg by absorption or capture via GST-tag, is not expected to affect the ability of the LRRK2 polypeptide to phosphorylate it.

The assay can be performed in maxisorp (Nunc) 384-clear plates. The substrate polypeptide, 30ng/well, is coated overnight at 4°C in Tris buffered saline (TBS) pH 7.4. Excess binding sites are blocked with 5% BSA in TBS containing 0.2% Tween (TBST) for 1 hour at room temperature and then washed three times with TBST. 15µl LRRK2 (1-1000ng) in reaction buffer (50 mM Tris pH 7.5, 0.01% BSA, 0.1 mM EGTA, 1mM DTT) is added to the well and 2µl of compound dissolved in 11% DMSO was added and incubated for 30 minutes. The reaction is initiated by the addition of 5 µL ATP (1-1000µM)/10 mM MgCl₂ and incubated at room temperature for 25 minutes. The reaction is stopped by addition of 20 µL 0.5 M EDTA. The plates were washed three times with TBST before the addition of 22 µL anti-phospho-polypeptide antibody (diluted 1:3700 fold in TBST containing 20µg/ml blocking peptide). After 1 hour the plates are washed three times with TBST and then 22 µL of anti-sheep-peroxidase conjugate (1:5000 dilution in 1 % BSA/TBST) is added to each well and incubated a further 1 hour. A final four washes of TBST are performed before addition of 22 µl peroxidase substrate 3,3',5,5'tetramethylbenzidine TMB in 50 mM acetic acid, 50mM sodium Acetate, 0.0009% H₂O₂. Colour is developed for 15 minutes and stopped by addition of 5 µL 1M HCl. Plates are read on an absorbance reader at 450 nm.

Alternative commercially available peroxidase substrates could be used, which would allow different colour detection. For example orthophenylenediamine (OPD) which is read at 492 nm or Diammonium2,2'-azino-bis(3-ethyl-benzothiazoline-6-sulfonate) which is read at 405 nm. Alternative detection technologies can also be applied using fluorescent substrates such as 10-acetyl-3.7,dihydroxyphenoxazine or luminal based sustrates for luminescence.

The assay is considered to be tolerant to a wide range of ATP concentrations (1-1000 µM) and 1% DMSO (compound storage solvent). Compound interference by autofluorescence, quenching or absorbance is considered to be minimised as it is heterogeneous involving several wash steps.

### Example 3: Generation of antibodies to LRRK2

Antibodies to LRRK2 were generated in sheep using the immunogen sequences shown in Table 2. Only the antibodies raised to LRRK2 (100-500) (immunogen shown in Figure 11) were considered to be useful in immunoprecipitating LRRK2 that retained protein kinase activity and was therefore useful in a protein kinase assay. The antibody S224C raised to LRRK2 (2078-2099) immunoprecipitated LRRK2 but was inhibitory to the kinase activity. Antibody LRRK2 (1245-1259) is considered to be useful in immunoblotting but is not considered to be useful for immunoprecipitation.

**Table 2.**

| Anti-LRRK2 antibodies | | |
|---|---|---|
| **Sheep No.** | **Antibody Name** | **Immunogen Sequence** |
| S869B | LRRK2 (1 - 190) | GST-LRRK2 [DU 6688] |
| S137C | LRRK2 (1326 -2527) | GST-LRRK2 [DU 10525] |
| S348C | LRRK2 (100-500) | LRRK2 (100 - 500) GST cleaved [DU 13636] |
| S407C | LRRK2 (100-500) | LRRK2 (100 - 500) GST cleaved [DU 13636] |
| S750B | LRRK2 (16 - 35) | LKKLlVRLNNVQEGKQIETL [residues 16 - 35 of human] |
| S224C | LRRK2 (2078 - 2099) | KFPNEFDELEIQGKLPDPVKEY [residues 2078 - 2099 of human] |
| S374C | LRRK2 (2498 - 2514) | CINLPHEVQNLEKHIEVR [residues 2498 - 2514 of human + N-terminal systeine for coupling]10 |
| S616B | LRRK2 (2508 - 2527) | EKHIEVRKELAEKMRRTSVE [residues 2508 - 2527 of human] |
| S357C | LRRK2 phospho Ser 910 | VKKKSNS*ISVGEFY [residues 904 - 917 of human] |
| S044C | LRRK2 phospho Thr 1503 | CLAKLRKT*IINESLN [residues 1497 - 1510 of human + N-terminal cysteine for coupling] |
| S146C | LRRK2 phospho Thr 1503 | CLAKLRKT*IINESLN [residues 1497 - 1510 of human + N-terminal cysteine for coupling] |
| | | |
| S994B | LRRK2 (1245 - 1259) | CRVEKLHLSHNKLKEI [residues 1245 - 1259 of mouse + N-terminal cysteine for coupling] |
| S994B | LRRK2 (2078 - 2096) | CFPNEFDELAIQGKLPDPV [residues 2078 - 2096 of mouse + N-terminal cysteine for coupling] |
| S184C | LRRK2 (2078 - 2096) | CFPNEFDELAIQGKLPDPV [residues 2078 - 2096 of mouse + N-terminal cysteine for coupling] |
| S225C | LRRK2 (2078 - 2099) | RFPNEFDELAIQGKLPDPVKEY [residues 2078 - 2099 of mouse] |

### References.

1 Paisan-Ruiz, C., Jain, S., Evans, E. W., Gilks, W. P., Simon, J., van der Brug, M., Lopez de Munain, A., Aparicio, S., Gil, A. M., Khan, N., Johnson, J., Martinez, J. R., Nicholl, D., Carrera, I. M., Pena, A. S., de Silva, R., Lees, A., Marti-Masso, J. F., Perez-Tur, J., Wood, N. W. and Singleton, A. B. (2004) Cloning of the gene containing mutations that cause PARK8-linked Parkinson's disease. Neuron 44, 595-600
2 Zimprich, A., Biskup, S., Leitner, P., Lichtner, P., Farrer, M., Lincoln, S., Kachergus, J., Hulihan, M., Uitti, R. J., Calne, D. B., Stoessl, A. J., Pfeiffer, R. F., Patenge, N., Carbajal, I. C., Vieregge, P., Asmus, F., Muller-Myhsok, B., Dickson, D. W., Meitinger, T., Strom, T. M., Wszolek, Z. K and Gasser, T. (2004) Mutations in LRRK2 cause autosomal-dominant parkinsonism with pleomorphic pathology. Neuron 44, 601-607
3 Mata, I. F., Wedemeyer, W. J., Farrer, M. J., Taylor, J. P. and Gallo, K. A. (2006) LRRK2 in Parkinson's disease: protein domains and functional insights. Trends Neurosci 29, 286-293
4 Taylor, J. P., Mata, I. F. and Farrer, M. J. (2006) LRRK2: a common pathway for parkinsonism, pathogenesis and prevention? Trends Mol Med 12, 76-82
5 Farrer, M., Stone, J., Mata, I. F., Lincoln, S., Kachergus, J., Hulihan, M., Strain, K. J. and Maraganore, D. M. (2005) LRRK2 mutations in Parkinson disease. Neurology 65, 738-740
6 Zabetian, C. P., Samii, A., Mosley, A. D., Roberts, J. W., Leis, B. C., Yearout, D., Raskind, W. H. and Griffith, A. (2005) A clinic-based study of the LRRK2 gene in Parkinson disease yields new mutations. Neurology 65, 741-744
7 Marin, I. (2006) The Parkinson disease gene LRRK2: evolutionary and structural insights. Mol Biol Evol 23, 2423-2433
8 Goldberg, J. M., Bosgraaf, L., Van Haastert, P. J. and Smith, J. L. (2002) Identification of four candidate cGMP targets in Dictyostelium. Proc Natl Acad Sci U S A 99, 6749-6754
9 Bosgraaf, L., Russcher, H., Smith, J. L., Wessels, D., Soil, D. R. and Van Haastert, P. J. (2002) A novel cGMP signalling pathway mediating myosin phosphorylation and chemotaxis in Dictyostelium. Embo J 21, 4560-4570
10 Bosgraaf, L. and Van Haastert, P. J. (2003) Roc, a Ras/GTPase domain in complex proteins. Biochim Biophys Acta 1643, 5-10
11 Manning, G., Whyte, D. B., Martinez, R., Hunter, T. and Sudarsanam, S. (2002) The protein kinase complement of the human genome. Science 298, 1912-1934
12 West, A. B., Moore, D. J., Biskup, S., Bugayenko, A., Smith, W. W., Ross, C. A., Dawson, V. L and Dawson, T. M. (2005) Parkinson's disease-associated mutations in leucine-rich repeat kinase 2 augment kinase activity. Proc Natl Acad Sci U S A 102,16842-16847
13 Greggio, E., Jain, S., Kingsbury, A., Bandopadhyay, R., Lewis, P., Kaganovich, A., van der Brug, M. P., Beilina, A., Blackinton, J., Thomas, K. J., Ahmad, R., Miller, D. W., Kesavapany, S., Singleton, A., Lees, A., Harvey, R. J., Harvey, K. and Cookson, M. R. (2006) Kinase activity is required for the toxic effects of mutant LRRK2/dardarin. Neurobiol Dis 23, 329-341
14 Cohen, P. and Knebel, A. (2006) KESTREL: a powerful method for identifying the physiological substrates of protein kinases. Biochem J 393, 1-6
15 Durocher, Y., Perret, S. and Kamen, A. (2002) High-level and high-throughput recombinant protein production by transient transfection of suspension-growing human 293-EBNA1 cells. Nucleic Acids Res 30, E9
16 Campbell, D. G. and Morrice, N. A. (2002) Identification of Protein Phosphorylation Sites by a Combination of Mass Spectrometry and Solid Phase Edman Sequencing. J. Biomol. Techn. 13, 121-132
17 Troiani, S., Uggeri, M., Moll, J., Isacchi, A., Kalisz, H. M., Rusconi, L. and Valsasina, B. (2005) Searching for biomarkers of Aurora-A kinase activity: identification of in vitro substrates through a modified KESTREL approach. J Proteome Res 4, 1296-1303
18 Bretscher, A., Edwards, K. and Fehon, R. G. (2002) ERM proteins and merlin: integrators at the cell cortex. Nat Rev Mol Cell Biol 3, 586-599
19 Polesello, C. and Payre, F. (2004) Small is beautiful: what flies tell us about ERM protein function in development. Trends Cell Biol 14, 294-302
20 Gary, R. and Bretscher, A. (1995) Ezrin self-association involves binding of an N-terminal domain to a normally masked C-terminal domain that includes the F-actin binding site. Mol Biol Cell 6, 1061-1075
21 Pestonjamasp, K., Amieva, M. R., Strassel, C. P., Nauseef, W. M., Furthmayr, H. and Luna, E. J. (1995) Moesin, ezrin, and p205 are actin-binding proteins associated with neutrophil plasma membranes. Mol Biol Cell 6, 247-259
22 Turunen, O., Wahlstrom, T. and Vaheri, A. (1994) Ezrin has a COOH-terminal actin-binding site that is conserved in the ezrin protein family. J Cell Biol 126, 1445-1453
23 Pearson, M. A., Reczek, D., Bretscher, A. and Karplus, P. A. (2000) Structure of the ERM protein moesin reveals the FERM domain fold masked by an extended actin binding tail domain. Cell 101, 259-270
24 Huang, L., Wong, T. Y., Lin, R. C. and Furthmayr, H. (1999) Replacement of threonine 558, a critical site of phosphorylation of moesin in vivo, with aspartate activates F-actin binding of moesin. Regulation by conformational change. J Biol Chem 274, 12803-12810
25 Nakamura, F., Huang, L., Pestonjamasp, K., Luna, E. J. and Furthmayr, H. (1999) Regulation of F-actin binding to platelet moesin in vitro by both phosphorylation of threonine 558 and polyphosphatidylinositides. Mol Biol Cell 10, 2669-2685
26 Matsui, T., Maeda, M., Doi, Y., Yonemura, S., Amano, M., Kaibuchi, K., Tsukita, S. and Tsukita, S. (1998) Rho-kinase phosphorylates COOH-terminal threonines of ezrin/radixin/moesin (ERM) proteins and regulates their head-to-tail association. J Cell Biol 140, 647-657
27 Oshiro, N., Fukata, Y. and Kaibuchi, K. (1998) Phosphorylation of moesin by rho-associated kinase (Rho-kinase) plays a crucial role in the formation of microvilli-like structures. J Biol Chem 273, 34663-34666
28 Tran Quang, C., Gautreau, A., Arpin, M. and Treisman, R. (2000) Ezrin function is required for ROCK-mediated fibroblast transformation by the Net and Dbl oncogenes. EMBO J 19, 4565-4576
29 Paglini, G., Kunda, P., Quiroga, S., Kosik, K. and Caceres, A. (1998) Suppression of radixin and moesin alters growth cone morphology, motility, and process formation in primary cultured neurons. J Cell Biol 143, 443-455
30 MacLeod, D., Dowman, J., Hammond, R., Leete, T., Inoue, K. and Abeliovich, A. (2006) The familial Parkinsonism gene LRRK2 regulates neurite process morphology. Neuron 52, 587-593
31 McClatchey, A. I. and Giovannini, M. (2005) Membrane organization and tumorigenesis-the NF2 tumor suppressor, Merlin. Genes Dev 19, 2265-2277
32 Boudeau, J., Miranda-Saavedra, D., Barton, G. J. and Alessi, D. R. (2006) Emerging roles of pseudokinases. Trends Cell Biol 16, 443-452
33 West, A. B., Moore, D. J., Choi, C., Andrabi, S. A., Li, X., Dikeman, D., Biskup, S., Zhang, Z., Lim, K. L., Dawson, V. L. and Dawson, T. M. (2007) Parkinson's disease-associated mutations in LRRK2 link enhanced GTP-binding and kinase activities to neuronal toxicity. Hum Mol Genet 16, 223-232
34 Gloeckner, C. J., Kinkl, N., Schumacher, A., Braun, R. J., O'Neill, E., Meitinger, T., Kolch, W., Prokisch, H. and Ueffing, M. (2006) The Parkinson disease causing LRRK2 mutation I2020T is associated with increased kinase activity. Hum Mol Genet 15, 223-232
35 Biskup, S., Moore, D. J., Celsi, F., Higashi, S., West, A. B., Andrabi, S. A., Kurkinen, K., Yu, S. W., Savitt, J. M., Waldvogel, H. J., Faull, R. L., Emson, P. C., Torp, R., Ottersen, O. P., Dawson, T. M. and Dawson, V. L. (2006) Localization of LRRK2 to membranous and vesicular structures in mammalian brain. Ann Neurol 60, 557-569
36 Farrer, M. J., Stone, J. T., Lin, C. H., Dachsel, J. C., Hulihan, M. M., Haugarvoll, K., Ross, O. A. and Wu, R. M. (2007) Lrrk2 G2385R is an ancestral risk factor for Parkinson's disease in Asia. Parkinsonism Relat Disord

### Example 4: Substrate specificity and inhibitors of the Parkins's disease mutated protein kinase LRRK2

The Leucine Rich Repeat Protein Kinase-2 (LRRK2) is mutated in a significant number of Parkinson's disease patients, but little is known about its regulation and function. A common mutation changing Gly2019 to Ser enhances catalytic activity, suggesting small molecule inhibitors might have utility in treating Parkinson's Disease. We utilised various approaches to explore the substrate specificity requirements of LRRK2 and elaborated a peptide substrate termed Nictide, that had 20-fold lower Kₘ and nearly 2-fold higher Vₘₐₓ than the widely deployed LRRKtide substrate. We demonstrate that LRRK2 has marked preference for phosphorylating Thr over Ser. We also observed that several Rho kinase (ROCK) inhibitors such as Y-27632 and H-1152, suppressed LRRK2 with similar potency to which they inhibited ROCK2. We also identified a mutant LRRK2[A2016T] that was normally active, but resistant to H-1152, Y-27632 as well as sunitinib, a structurally unrelated multikinase inhibitor that in contrast to other compounds also suppresses LRRK2, but not ROCK We have also developed the first assay to measure the protein kinase activity of endogenous LRRK2. Finally, we describe a pharmacological approach to validate whether substrates are phosphorylated by LRRK2 and use this to provide evidence that LRRK2 may not be rate-limiting for the phosphorylation of the proposed substrate moesin. Our findings reported in this study will aid with the investigation of the LRRK2 kinase.

Autosomal dominant point mutations within the gene encoding for the Leucine Rich Repeat protein Kinase-2 (LRRK2) predispose humans to Parkinson's disease (PD) [1, 2]. Patients with LRRK2 mutations generally develop PD at the normal age of 60-70 years, with clinical appearance and symptoms indistinguishable from idiopathic PD [3]. Mutations in LRRK2 account for 4% of familial PD, and observed in 1 % of sporadic PD patients [3]. Little is known about how LRRK2 is regulated, what are its substrates and how mutations cause PD.

LRRK2 is a large multi-domain protein kinase of 2527 residues, consisting of leucine rich repeats (residues 1010-1287), GTPase domain (residues 1335-1504), COR domain (residues 1517-1843), serine/threonine protein kinase domain (residues 1875-2132) and a WD40 repeat (2231-2276) [4]. Over 40 mutations have thus far been reported which mainly comprise amino acid substitutions [5]. The most frequent mutation comprises an amino acid substitution of the highly conserved Gly2019 located within the subdomain VII-DFG motif of the kinase domain to a Ser residue [5]. Several studies have reported that this mutation enhances the protein kinase activity of LRRK2 two to three-fold, suggesting that LRRK2 inhibitors may have utility for the treatment of PD [6]. Other than nonspecific/multi-kinase protein kinase inhibitors staurosporine (IC50 2 nM), K252 (IC50 4 nM), Su-11248/sunitinib (IC50 15 nM) [7, 8] no selective LRRK2 inhibitors have been reported thus far.

We previously undertook a KESTREL screen in rat brain extracts to identify proteins phosphorylated by the activated PD LRRK2[G2019S] mutant. This led to the observation that moesin, a member of the ERM proteins that anchors the actin-cytoskeleton to the plasma membrane is efficiently phosphorylated by LRRK2, at Thr558, a previously identified in vivo phosphorylation site that regulates the ability of moesin to bind actin [9]. LRRK2 also phosphorylated other ERM proteins, ezrin and radixin that are related to moesin, at the residue equivalent to Thr558, as well as a peptide encompassing Thr558 (LRRKtide) [9]. Previous work had suggested that the Rho-kinase (ROCK) could also phosphorylate ERM proteins at the residue equivalent to Thr558 of moesin both in vitro and when overexpressed in cells [10-12]. No evidence has been published to demonstrate that LRRK2 phosphorylates ERM proteins in cells.

To aid the functional characterisation of LRRK2, we have analysed the substrate specificities of LRRK2 and elaborated the peptide substrate Nictide that has a 20-fold lower Kₘ and nearly 2-fold higher Vₘₐₓ than the widely deployed LRRKtide substrate [9]. We also observed that some previously reported ROCK inhibitors also inhibited LRRK2 with similar potency as they inhibited ROCK Moreover, we demonstrate that sunitinib can be deployed as a control compound that inhibits LRRK2 but not ROCK. We generated an inhibitor-resistant mutant of LRRK2 that is normally active, but 20-fold less sensitive to inhibition by the LRRK2 inhibitors. We also develop the first robust assay that allows the protein kinase activity of endogenous LRRK2 to be quantified and present a pharmacological strategy that can be deployed to validate LRRK2 substrates. The findings presented in this study will help with dissecting the regulation and function of LRRK2.

### Materials and Methods

**Reagents and General methods. Tissue-culture reagents were from Life Technologies.** Glutathione Sepharose 4B was from Amersham Biosciences and [γ-³²P]-ATP and [γ-³³P]-ATP was from Perkin Elmer. P81 phosphocellulose paper was from Whatman. LRRKtide and its derivatives were synthesized by Pepceuticals. The Flp-in T-REx system was from Invitrogen and stable cell lines were generated as per manufacturer instructions by selection with hygromycin. Restriction-enzyme digests, DNA ligations and other recombinant DNA procedures were performed using standard protocols. All mutagenesis was carried out using the Quick-Change site-directed-mutagenesis kit (Stratagene). DNA constructs used for transfection were purified from Escherichia coli DH5□using Qiagen or Invitrogen plasmid Maxi kits according to the manufacturer's protocol. All DNA constructs were verified by DNA sequencing, which was performed by The Sequencing Service, School of Life Sciences, University of Dundee, Scotland, U.K, using DYEnamic ET terminator chemistry (Amersham Biosciences) on Applied Biosystems automated DNA sequencers.

**Buffers.** Lysis Buffer contained 50 mM Tris/HCl, pH 7.5, 1 mM EGTA, 1 mM EDTA, 1% (w/v) 1 mM sodium orthovanadate, 10 mM sodium β-glycerophosphate, 50 mM NaF, 5 mM sodium pyrophosphate, 0.27 M sucrose, 1 mM Benzamidine and 2 mM phenylmethanesulphonylfluoride (PMSF) and was supplemented with either Triton X-100 or 0.5% (v/v) NP-40 with 150 mM as indicated. Buffer A contained 50 mM Tris/HCl, pH 7.5, 50 mM NaCl, 0.1 mM EGTA and 0.1% (v/v) 2-mercaptoethanol, and 0.27 M sucrose. Sodium dodecyl sulfate (SDS) lysis buffer employed to lyse cells in Figure 6 was lysis buffer supplemented with 1% SDS and 0.1% 2-mercaptoethanol and pH adjusted 6.8.

**Cell culture, treatments and cell lysis.** HEK-293 cells were cultured in Dulbecco's Modified Eagle's medium supplemented with 10% FBS, 2mM glutamine and 1xantimycotic/antibiotic solution. T-REx cell lines were cultured in DMEM supplemented with 10% FBS and 2mM glutamine, 1X antimycotic/antibiotic, and 15µlg/ml Blastocidin and 100ug/ml hygromycin. Cell transfections were performed by the polyethylenimine method [14]. Cultures were induced to express the indicated protein by inclusion of 1µg/ml doxycycline in the culture medium for the indicated times. Where inhibitors are utilized, they were dissolved in DMSO and used at the indicated concentrations with an equivalent volume of DMSO used as a control. The final concentration of DMSO in the culture medium was never more than 0.1% (v/v). Inhibitors were added to the culture medium 60 min prior to lysis. Cells were lysed with 1.0 ml of lysis buffer per 15 cm dish supplemented with the indicated detergent and clarified by centrifugation at 16,000 x g at 4 °C for 10 minutes. After induction and inhibitor treatment, T-REx-GFP Rho expressing cells were lysed at room temperature with SDS lysis buffer after washing with PBS. SDS lysates were boiled and sonicated to reduce viscosity. All lysate supernatants were snap frozen in liquid nitrogen and stored at -80 °C until use. Protein concentrations were determined using the Bradford method with BSA as the standard.

**Purification of recombinant proteins.** Recombinant glutathione-s transferase (GST)-LRRK2 1326-2527 and variants thereof were prepared as described in [9], except that lysis buffer contained 0.5% (v/v) NP-40 and 150 mM NaCl. The following recombinant proteins were generated in the Division of Signal Transduction Thearapy at the University of Dundee: rat ROCK2 (amino acids 2-543), chicken maltose binding protein-MYPT (amino acids 714-1004), GST-ezrin (amino acids 1-586), GST-moesin (amino acids 1-577) and GST-LRRK2 (amino acids 100-500). Peptide substrates were displayed as GST fusions in the pGEX-6P vector. To induce the expression of GST-LRRKtide and GST-Nictide, Eschericia coli BL21 transformants were grown to an OD600 of 0.5 at 37 °C and induced at 16 °C by the addition of isopropyl β-D-1-thiogalactopyranoside to a final concentration of 1mM. Cells were lysed by sonication in lysis buffer with 1%(v/v) Triton X-100. The soluble fraction was retrieved by centrifugation at 15000 x g for 20 minutes. Recombinant protein was purified by glutathione sepharose chromatography and proteins were eluted in buffer A with 20 mM glutathione, 1 mM benzamidine and 2 mM PMSF.

**Antibodies.** A glutathione-s transferase (GST) fusion protein of amino acids was expressed in bacteria and purified by glutathione sepharose chromatography. Following cleavage of the GST tag, LRRK2 [100-500] was used as an immunogen to raise a polyclonal antibody (S348C). Antibodies were affinity purified from antisera using the LRRK2 [100-500] protein immunogen. Antibody (S374C) against LRRK2 was raised against a peptide immunogen similar to what was described previously [15], encompassing amino acids 2498-2514 (CINLPHEVQNLEKHIER with NH2 cysteine for coupling to keyhole limpet hemocyanin[KLH]). Antibodies were affinity purified against the peptide. Anti-moesin (S135C) and anti-ezrin antibodies (S245C) were generated by injection of purified full-length protein into sheep, followed by affinity purification of the antibody against the antigen. Pan phospho-ERM antibody (S296C) was generated by injection of the KLH conjugated phosphopeptide CDKYKTpLRQI into sheep and was affinity purified by positive and negative selection against the phospho and de-phospho peptides respectively. Sheep polyclonal antibody (S662B) was raised against MBP-MYPT chicken amino acids (714-1004). Rabbit polyclonal antibody against MYPT phosphothreonine 850 was from Upstate (#36-003). Anti GFP antibody (S268B) was raised against recombinant GFP protein and affinity purified against the antigen. Antibody (S221B) against ERK1/2 was raised against GST-ERK1 protein. Anti-FLAG M2 antibody and affinity matrix were from Sigma (A2220).

**Specificity kinase panel.** All assays were performed at the The National Centre for Protein Kinase Profiling (http://www.kinase-screen.mrc.ac.uk/) as previously described [16]. Briefly, all assays were carried out robotically at room temperature (approximately 21 °C) and were linear with respect to time and enzyme concentration under the conditions used. Assays were performed for 30 min using Multidrop Micro reagent dispensers (Thermo Electron Corporation, Waltham, MA, U.S.A.) in a 96-well format. The abbreviations for each kinase are defined in legend to Table 1. The concentration of magnesium acetate in the assays was 10 mM and [γ-³³P]ATP (-800 cpm/pmol) was used at 5 µM for CK2α, DYRK3, EF2K,ERK1, ERK8, GSK3ß, HIPK2, HER4, IGF1R, IRR, MARK3, MKK1, p38γ MAPK, p386 MAPK, PAK4, PIM2, Akt1, PLK1, PKCζ and PRK2; 20 µM for Aurora B, CaMKKβ, CDK2/cyclin A, CHK1, CHK2, CK1δ, CSK, EPH-B3, FGF-R1, GCK, IRAK4, IR, JNK1α1, JNK2α2, LKB1, MAPKAP-K2, MLK1, MLK3, MSK1, MST2, MST4, p38ß MAPK, NUAK, PKA, PAK5, PAK6, PDK1, PIM1, PIM3, PKCα, ROCKII, PRAK, S6K1, SGK1, SYK, TTK, VEGFR and YES1; or 50 µM for AMPK, BRSK2, BTK, CaMK1, DYRK1a, DYRK2, EPH-A2, ERK2, IKKβ, IKKε, LCK, MELK, MINK1, MNK1, MNK2, NEK2A, NEK6, p38α, PhKγ1, Akt2, PKD1, RSK1, RSK2, SRPK1 Src, and TBK, in order to be at or below the Kₘ for ATP for each enzyme.

**Peptide Kinase Assays.** Peptide Kinase Assays were set up in a total volume of 40 µl with recombinant kinase or kinase displayed as an immune-complex coupled to protein G sepharose in 50mM Tris pH 7.5, 0.1 mM EGTA, 10 mM MgCl2 and 0.1 mM [γ-³²P]ATP (∼500-1000 cpm/pmol) in the presence of the indicated concentration of peptide substrate. In reactions where kinase inhibitors were assayed, inhibitors were dissolved in dimethyl sulfoxide and were at 0.1% of the reaction volume. After incubation for 15 min at 30°C, reactions were terminated by applying 35 µl of the reaction mixture on to P81 phosphocellulose paper and immersion in 50 mM phosphoric acid. After extensive washing, reaction products were quantitated by Cerenkov counting. A unit (U) of LRRK2 activity was defined as the amount of enzyme that catalysed the incorporation of 1 nmol of ³²P into LRRKtide. *K*ₘ and *V*ₘₐₓ parameters were determined by performing the assay described above using various concentrations of LRRKtide or Nictide. The Kₘ and Vₘₐₓ parameters were calculated using the Graph-Pad Prism program. *K*m and *V*max values are rounded in Figure 1C to reflect the need to estimate values due to the nature of the peptide assay, wherein high concentrations of peptide became inhibitory to the kinase. IC50 values were calculated using non-linear regression analysis using Graph-Pad Prism.

**Protein substrate kinase assays.** Assays were set up in a total volume of 25 µl with recombinant kinase or kinase displayed as an immunecomplex coupled to protein G sepharose in 50mM Tris pH 7.5, 0.1 mM EGTA, 10 mM MgCl2 and 0.1 mM [γ-³²P] ATP (∼500cpm/pmol), with substrate at 2 µM. After incubation for 15 min at 30°C, the reactions were stopped by the addition of Laemelli sample buffer. Reaction products were resolved by electrophoresis on sodium dodecyl sulfate polyacrylamide gels. The incorporation of phosphate into protein substrates was determined by autoradiography and/or immunoblotting with phosphospecific antibodies.

**Immunological procedures.** Cell lysates (10-30µg) were resolved by electrophoresis on SDS polyacrylamide gels or Novex 4-12% gradient gels, and electroblotted to nitrocellulose membranes. Membranes were blocked with 5% (w/v) in Tris/HCl, pH 7.5, 0.15 M NaCl and 0.1% (v/v) Tween (TBST Buffer). For phospho-MYPT antibody, primary antibody was used at a concentration of 1 µg/ml, diluted in 5% BSA in TBST. Phospho-ERM antibody was used at 1 µg/ml in the presence of 10 µg/ml LRRKtide, diluted in 5% (w/v) skimmed milk in TBST. All other antibodies were used at 1 µg/ml in 5% (w/v) milk in TBST. Detection of immune-complexes was performed using horseradish-peroxidase-conjugated secondary antibodies (Pierce) and an enhanced-chemiluminescence reagent. For immunoprecipitations, antibody was non-covalently coupled to protein G-Sepharose at a ratio of 1 µg antibody/µl of beads, or anti-FLAG M2-agarose was utilized. The indicated amount of cell lysate was incubated with 5 µl bed volume of coupled antibody for 1 hour. Immune complexes were washed twice with lysis buffer supplemented with 0.5 M NaCl and twice with Buffer A. Precipitates were either used as a source of kinase or immediately analyzed by immunoblot.

**Isolation and mass fingerprint identification of endogenous LRRK2.** 20 µl of the above described antibody conjugate, either IgG or LRRK2, was incubated with 60 mg of Triton X-100 soluble Swiss 3T3 lysate that had been pre-cleared by incubation with protein GSepharose, for 2.5 hours at 4°C. Beads were washed four times with lysis buffer supplemented with 0.15 M NaCl and twice with buffer A. Precipitates were reduced with 10 mM dithiothreitol and then alkylated with 50 mM iodoacetamide for 30 min at room temperature. Samples were resolved on 4-12% Novex gels and stained with colloidal blue. Colloidal blue stained bands at the approximate size of LRRK2 were excised and mass fingerprinting of in-gel digested tryptic peptides. The LRRK2 band and the corresponding region of the IgG control immunoprecipitation was excised, cut into smaller pieces, washed sequentially for 15 min on a vibrating platform with 0.5 ml of the following: a 1:1 (v/v) mixture of water and acetonitrile, 0.1 M ammonium bicarbonate, a 1:1 (v/v) mixture of 0.1 M ammonium bicarbonate and acetonitrile, and finally acetonitrile. The gel pieces were dried in a speed-vac and then rehydrated in 25 mM triethylammonium bicarbonate containing 0.5 µglml of MS-grade trypsin (Promega). After 16 h, an equal volume of acetonitrile was added and the mixture incubated on a shaking platform for 10 min. The supernatant was dried and the remaining peptides in the gel pieces were further extracted with 0.1 ml of 50% acetonitrile/2.5% formic acid. Samples were analyzed on an LTQ Orbitrap XL mass spectrometer (Thermo). Masses were searched with the mascot server (matrixscience.com) using the International Protein Index mouse database. **Computer analysis.** Autoradiography films and immunoblot film were scanned on an epson4990 scanner and images were managed with Adobe Photoshop. Protein sequence alignments were performed with MUltiple Sequence Comparison by Log-Expectation (MUSCLE) EBI (http://www.ebi.ac.uk/Tools/muscle/index.html) and managed with JalView (hftp://www.jalview.org/). Enzyme kinetic analysis was performed with GraphPad Prism. Figures were generated in Adobe Illustrator.

### Results

**Comparison of the substrate specificities of LRRK2 and ROCK.** We first compared the rates at which recombinant ROCK2 and LRRK2 phosphorylated ezrin and MYPT (a well characterised ROCK substrate [17]). Under conditions in which equimolar MYPT and ezrin were present, ROCK2 phosphorylated MYPT but barely ezrin (Fig 12A). In contrast, LRRK2 phosphorylated ezrin, but did not phosphorylate MYPT (Fig 12A). Comparison of residues surrounding Thr850 (major ROCK phosphorylation site on MYPT [18]) and Thr567 (LRRK2 phosphorylation site on ezrin [9]), revealed that overall these peptides were dissimilar with only the positions -3(RMYPT/Kezrin) possessing homology (Fig 12A, lower panel). The human sequence surrounding the LRRK2 phosphorylation site of ezrin is identical in moesin and radixin, and also strikingly conserved in *Drosophila* and *C.elegans* ERM homologues (Fig 12B, lower panel). To investigate the substrate specificity determinants of LRRK2, we verified how mutation of different residues affected the kinetics of LRRK2 phosphorylation of the LRRKtide peptide that encompasses the Thr567 ERM Phosphorylation motif (Fig 12C). The wild type LRRKtide peptide was phosphorylated by LRRK2 with a Kₘ of 200 µM and Vₘₐₓ of 14 U/mg. The following mutations to Ala suppressed phosphorylation by increasing the Kₘ value: -5Arg (2.5 fold), -2Tyr (2.4-fold), +2Arg (4.5-fold) and +5Arg (4-fold) residues. Mutation of the -2Tyr to Glu increased Kₘ 4.4- fold, whilst its mutation to Arg slightly decreased Kₘ suggesting that an aromatic residue at this position is not essential (Fig 12C). Mutation of the +2 and + 5 residues of the peptide to Pro or Glu increased Kₘ similarly to the Ala mutation. Only mutation of the -4 (Asp) to Ala moderately enhanced peptide phosphorylation by decreasing Kₘ 2.3-fold (Fig 12C). Several mutations also markedly decreased Vₘₐₓ values which included: -2 Tyr (2 to 10-fold), + 1Leu (2-fold), +2 Arg (4 to 28-fold) and +5 Arg (mutation to Glu 4-fold). We also investigated how mutations in LRRKtide affected phosphorylation by ROCK2. In contrast to LRRK2, we observed that several mutations substantially improved peptide phosphorylation by decreasing the Kₘ value. The most dramatic change involved mutation of the -2Tyr to an Arg residue that is found in most ROCK substrates. This decreased the Kₘ value over 60-fold and increased the Vₘₐₓ over 5-fold. Mutation of the +1Leu residue to Ala abolished phosphorylation by ROCK2, but had no effect on LRRK2 phosphorylation (Fig 12C).

**Elaboration of an optimal LRRK2 peptide substrate.** To further investigate and improve the optimal phosphorylation motif for LRRK2, we utilized a positional scanning peptide library approach [19, 20]. This assay utilises 198 biotinylated peptide libraries. Each library contains a 1:1 mixture of serine and threonine at the central position and one additional position fixed to one of the 20 amino acids, phosphothreonine, or phosphotyrosine. Phosphothreonine and phosphotyrosine were included to allow identification of kinases that possess a requirement for priming phosphorylation events. All other positions contain an equimolar degenerate mixture of natural amino acids (except serine, threonine, and cysteine). Recombinant LRRK2[G2019S] or kinase inactive LRRK2[D2017A] was used to phosphorylate all 198 peptide libraries simultaneously in solution using µ-³²P-ATP, and biotinylated peptides were captured on a streptavidin-coated membrane. The relative preference for each amino acid at each position was determined by quantifying ³²Pradioactivity incorporation following phosphoimaging (Fig 3A). The quantitative results of the LRRK2[G2019S] screen were also input as a matrix into enoLOGOS programme [21] and the relative preferences for each amino acid is displayed in Figure 13B. We found that LRRK2 exhibited preferred sequence specificity at multiple positions relative to the phosphorylation site, with strong preferences for -5(Trp, Arg), -2(Phe, Tyr, His and Thr), -1(Tyr, Arg, Trp), +2 (Arg and Thr) and +3 (Arg) positions. This is consistent with the kinetic studies shown in Figure 12, demonstrating that mutation of these residues increased Kₘ and in some cases also decreased Vₘₐₓ values. An Asp or Glu residue at any position with the peptide reduced LRRK2 phosphorylation (Fig 13A). For experiments undertaken with kinase-inactive LRRK2[D2017A], vastly lower overall levels of phosphorylation were observed, but nevertheless some contaminant -kinase activity with preference for Arg residues at the -3 and -2 positions was still found. Similar results were also reported in previous studies employing recombinant kinase-inactive GST-IκB Knase-β derived from 293 cells [22]. This trace level of protein kinase activity probably results from protein kinases that contaminate the GSTpurified kinase from 293 cell extracts.

### Elaboration of Nictide LRRK2 substrate.

The data from the positional scanning peptide library indicated that the optimal LRRK2 phosphorylation motif between -5 and + 4 positions is WWRFYTLRRA. In order to generate an improved substrate for LRRK2, we substituted this motif into the moesin sequence, from which the LRRK2tide peptide was derived. As sequences as distant as the + 5 residues affected kinetics of LRRKtide phosphorylation. (Fig 12C) and the LRRKtide peptide terminated at the + 6 position, we decided to incorporate the WWRFYTLRRA motif into a longer variant of the LRRKtide peptide encompassing a further 6 residues of moesin. The resulting sequence RLG**WWRFYTLRRAR**QGNTKQR was termed Nictide (reflecting the names of the 2 first authors of this study). We first compared the phosphorylation by LRRK2[G2019S] of GST fused to the original LRRKtide sequence, the longer version of LRRKtide, the entire C-terminus of moesin (residues 500-577) as well as Nictide. This revealed that GST-Nictide was phosphorylated to a significantly greater extent by LRRK2 than the other GST-fusion proteins (Fig 13C). Mutation of the Thr residue predicted to comprise the LRRK2 phosphorylation site, virtually abolished phosphorylation of the GSTfusion proteins. Our results also demonstrate that the expanded LRRKtide sequence was more efficiently phosphorylated by LRRK2 than the original shorter variant (Fig 13C).

We next generated the synthetic Nictide peptide and found that it was phosphorylated by LRRK2[G2019S] with a Kₘ of 10 µM (20-fold lower than LRRKtide) and Vₘₐₓ of 26 U/mg (1.7-fold higher than LRRKtide). Nictide was phosphorylated by wild type LRRK2 with a similar Kₘ, but ∼2-fold lower Vₘₐₓ. consistent with previous work showing that the Gly2019Ser mutation stimulates LRRK2 activity [6]. We next studied the effects of mutating individual residues of Nictide on phosphorylation by LRRK2[G2019S] as well as wild type LRRK2 (Fig 14). The mutations affected wild type LRRK2 and LRRK2[G2019S] similarly. Most mutations moderately affected Kₘ values with the largest effect being the - 5Trp to Ala mutation increasing the Kₘ value 3-fold. Other mutations decreased the Vₘₐₓ value of phosphorylation 2 to 5-fold (-4Trp to Ala and +2 Arg to Ala) (Fig 14). Several mutations (-5Trp to Ala, -4Trp to Ala and -1 Tyr to Ala) increased Kₘ values 2 to 3-fold, but enhanced Vₘₐₓ of LRRK2 phosphorylation ∼2-fold. We also combined the -5, -4 and -1 mutations that enhanced Vₘₐₓ and found that although high Vₘₐₓ values were maintained, the Kₘ values were substantially increased 5 to 20-fold compared to Nictide. Interestingly, mutation of the Thr residue phosphorylated by LRRK2 to a Ser almost abolished phosphorylation of the peptide by LRRK2 (Fig 14). Surprisingly, mutation of the +1 Leu to a Pro residue, which would inhibit phosphorylation of most substrates by non-CMGC proline directed kinases, only decreased Vₘₐₓ of LRRK2 phosphorylation under 2-fold without affecting Kₘ. This suggests that LRRK2 despite not belonging to the CMGC kinase family does have the potential to phosphorylate Ser/Thr residues followed by a Pro residue. The positional scanning peptide library doata also indicated that there could be a preference of ra Thr residue at the -2 and + 2 positions (Fig 13A). As similar preferences for Thr at -2 and +2 positions has also been observed in other kinase scanning peptide library screens (Miller et al (2008) Linear motif atlas for phosphorylation-dependent signaling. Sci Signal 1, ra2), we decided to introduce THr at either -2 or +2 positions and found that this moderately increased the Kₘ and reduced the Vₘₐₓ value (Fig 14). Introduction of Thr residues at both the -2 and +2 positions increasd Kₘ value of over 10-fold suggesting that Thr at these positions are not well tolerated.

### Identification of selective small molecule tool inhibitors of LRRK2

In the course of comparing the substrate specificity of LRRK2[G2019S] and ROCK2, we observed that several commonly deployed ROCK inhibitors (Y-27632 [23], hydroxyfasudil [24], H-1152[25]) also inhibited LRRK2[G2019S] (Fig 15A & B). Y-27632 inhibited LRRK2[G2019S] (IC50 1 µM) with similar potency to ROCK2 (IC50 0.7 µM). LRRK2[G2019S] was inhibited by hydroxyfasudil (IC50 6.8 µM) and H-1152 (IC50 0.15 µM) ∼3-fold more weakly than ROCK (Fig 15B). H-1152 and hydroxyfasudil belong to a well-studied series of isoquinolinesulfonamides [26] whilst Y-27632 is structurally unrelated (Fig 15A). Other isoquinolinesulfonamides reported to inhibit ROCK namely H89 [27] and fasudil [28] only inhibited LRRK2[G2019S] weakly (Fig 15B).. We confirmed that structurally unrelated sunitinib, inhibited LRRK2 with similar potency (IC50 ∼ 19 nM) to previous reports [7, 8], but in contrast to Y-27632 and H-1152, sunitinib only weakly inhibited ROCK (IC50 ∼ 3700 nM). Comparing the potency of H-1152, Y-27632 and sunitinib for LRRK2[G2019S] and wild type LRRK2, we observed that wild type LRRK2 was moderately less sensitive to these drugs than the activated mutant. The IC50 of inhibition of wild type LRRK2 was increased 2-fold for H-1152 and Y-27632 and 4-fold for sunitinib (Fig 15B).

### LRRK2 tool compound selectivity profiles

To compare the relative selectivity profiles of Y-27632, H-1152 and fasudil (HA-1077) with sunitinib, we profiled these inhibitors against a panel of 85 protein kinases at ATP concentrations, which approximate the Kₘ constant for ATP (Table 1). This revealed that Y- 27632 is selective, and at 10 µM in addition to inhibiting ROCK2 only suppressed activity of PRK2 and MNK1 over 5-fold. H-1152 (1 µM) in addition to ROCK, inhibited Aurora B and BRSK2 over 5-fold. Fasudil, hydroxyfasudil and sunitinib were less selective. Fasudil (10 µM) inhibited RSK1, S6K1, PRK2, MSK1, MNK1, MELK, MSK1, MELK over 5-fold. Hydroxyfasudil (10 µM) inhibited RSK1, S6K1, PRK2, MSK1. Sunitinib (1 µM) significantly inhibited 12 kinases respectively out of the 85 kinases profiled (Table 1).

### Development of inhibitor resistant mutants.

Previous work has shown that the Ala215 residue on ROCK plays an important role in controlling the specificity of interaction with H-1152 by forming two van der Waals interactions with H-1152 [31]. In the case of LRRK2, the residue equivalent to Ala215 is Ala2016 (Fig 16A). This residue in both LRRK2 and ROCK1 lies just prior to the subdomain VII-DFG motif. PKA is inhibited ∼50-fold more weakly by H-1152, and has Thr183 in the equivalent position. Mutation of Thr183 to Ala in PKA did not affect basal activity, but enhanced its inhibition by H-1152 four-fold [32]. We therefore mutated Ala2016 in LRRK2 to a Thr residue and found that this did not inhibit LRRK2 [G2019S] basal activity, but increased the IC50 of inhibition by H-1152 ∼20-fold (Fig 16B). We also observed that the LRRK2 [A2016T] mutant was 13 and ∼12-fold more resistant to inhibition by Y-27632 and sunitinib, respectively (Fig 16B). In the LRRK2[A2016T] mutant the T2016 side chain would clash with these atoms of H-1152, probably forcing it to bind in a rotated and less favorable orientation in the ATP-site. This is the likely explanation of its reduced activity against the mutant compared to the wild-type LRRK2. We also mutated Ala2016 to other residues but found that these mutations markedly inhibited intrinsic LRRK2 activity (data not shown).

**Evaluation of ROCK and LRRK2 inhibitors in cells.** We next investigated the effect that sunitinib, H-1152 and Y-27632 had on phosphorylation of MYPT and ERM proteins in HEK 293 cells. We observed significant basal phosphorylation of MYPT at Thr850 and ERM proteins at site(s) equivalent to Thr567 on ezrin (Fig 17A). In order to activate ROCK we induced stable expression of a constitutively activated G14V-Rho mutant which increased phosphorylation of both MYPT and ezrin ∼ 3-fold (Fig 17A). Treatment of cells with 10 µM sunitinib did not ablate phosphorylation of MYPT at Thr850 to a similar extent to H-1152 and Y-27632, consistent with ROCK mediating this phosphorylation. However, in the same extracts neither sunitinib, H-1152 nor Y-27632 inhibited ERM protein phosphorylation either in the presence or absence of G14V-Rho (Fig 17A).

**Immunoprecipitation and assay of endogenous LRRK2**. In order to measure protein kinase activity of endogenous LRRK2 (which to our knowledge has previously not been achieved), we generated numerous LRRK2 antibodies and evaluated their ability to immunoprecipitate and immunoblot recombinant full length Flag-LRRK2. This revealed that the antibody raised against a fragment of LRRK2 encompassing amino acids 100-500 immunoprecipitated and immunoblotted Flag-LRRK2 with similar efficiency as anti-Flag antibody (Fig 18A). Moreover, overexpressed FLAG-LRRK2 was immunoprecipitated with the LRRK2 [100-500] antibody and possessed similar activity to enzyme immunoprecipitated with Flag-antibody, indicating that the LRRK2 [100-500] antibody is not inhibiting LRRK2 protein kinase activity (Fig 18A). We next attempted to immunoprecipitate endogenous LRRK2 from extracts derived from a panel of cell lines using the LRRK2 [100-500] antibody. This revealed that Swiss3T3 fibroblasts and RAW macrophages express detectable levels of endogenous LRRK2 protein (Fig 18B). The LRRK2 [100-500] immunoprecipitates derived from Swiss-3T3 cells were subjected to electrophoresis on a polyacrylamide gel. Staining with Colloidal blue revealed a protein migrating with a molecular weight of ∼280 kDa which was confirmed to comprise LRRK2 by mass spectroscopy analysis (Fig 18C). We next subjected LRRK2 and control immunoprecipitates derived from Swiss 3T3 and Raw cells to protein kinase assays employing Nictide as a substrate. This revealed significant protein activity with LRRK2 immunoprecipitates, but not with the control immunoprecipitate. Moreover, the protein kinase activity detected in the LRRK2 immunoprecipitate was suppressed by H-1152 and sunitinib.

### Discussion

Our data reveal that LRRK2 tolerates a wider range of amino acids in its substrates compared to some other protein kinases that have strong requirements for specific amino acids within the substrates that they phosphorylate (Fig 12 and 13). Significant substrate specificity preferences are -5(Trp, Arg), -2(Phe, Tyr, His), -1(Tyr, Arg, Trp), +2 (Arg) and +3 (Arg) positions. Importantly, or data suggest that LRRK2 has a strong preference for phosphorylating Thr, as mutation of the phosphorylated Thr residue in Nictide to Ser virtually abolished phosphorylation of the peptide by LRRK2 (Fig 14). Positional scanning peptide library analysis also suggested that LRRK2 poorly tolerated acidic Glu or Asp residues at all positions surrounding the phosphorylation site (Fig 13A). LRRKtide only possesses a single acidic residue (-4Asp), and the only mutation we tested that improved LRRK2 phosphorylation, was mutation of this residue to Ala (Fig 12C). It is possible that this knowledge of substrate specificity of LRRK2 may aid in the identification of LRRK2 substrates and/or potential phosphorylation sites within identified substrates. This analysis has also enabled us to generate the Nictide peptide, a much improved substrate compared to LRRKtide peptide that is widely deployed to assay LRRK2. A key advantage of Nictide is that it can be used at much lower concentrations in kinases assays. We have been able to assay the activity of endogenous LRRK2 using Nictide, with virtually no background activity observed in the control immunoprecipitate (Fig 18E). To our knowledge this is the first time that activity of endogenous LRRK2 has been assessed. When trying to assay activity of endogenous LRRK2 employing LRRKtide at concentrations of 300µM (Kₘ value), we observed significant background activity in the pre-immune immunoprecipitation (ND data not shown). Assessment of activity of endogenous LRRK2 is important, as it paves the way to study LRRK2 activity in cells/tissues derived from PD patients. It will enable evaluation of whether LRRK2 protein kinase activity is controlled by extracellular agonists and may also help in the screening for inhibitors for LRRK2. We also observed that fusing Nictide to GST, yielded a highly expressed protein in *E.coli* (4 mg/litre) which was efficiently phosphorylated by LRRK2 in vitro at a greater initial rate than GSTezrin [505-586] or GST-LRRKtide (Fig 13C). GST-Nictide would serve as a good positive control when evaluating efficiency of phosphorylation of LRRK2 substrates that are identified in future studies.

Our analysis reveals that the substrate specificity of LRRK2 is quite distinct from ROCK2. LRRK2 does not phosphorylate MYPT and ROCK2 poorly phosphorylates ezrin. Moreover, mutations in LRRKtide affected phosphorylation by LRRK2 and ROCK2 in different ways. For example, mutation of the +1 position of the LRRKtide peptide from a Leu to Ala abolished ROCK phosphorylation, without affecting LRRK2 phosphorylation. Many LRRKtide mutations enhanced phosphorylation by ROCK but inhibited phosphorylation by LRRK2. Consistent with ROCK2 phosphorylating ezrin poorly in vitro, we also found that in vivo various ROCK inhibitors failed to inhibit ERM phosphorylation under conditions which they suppressed MYPT phosphorylation. This is consistent with other studies where the Y-27632 ROCK inhibitor was found not to suppress ERM phosphorylation [33, 34]. Taken together this data casts doubt on earlier suggestions that ERM proteins are physiologically phosphorylated by ROCK isoforms. The finding that the H-1152, Y-27632 and sunitinib failed to suppress ERM phosphorylation indicates that either LRRK2 does not phosphorylate ERM in 293 cells or that LRRK2 is not the sole kinase that phosphorylates ERM proteins. As we were unable to detect significant levels of endogenous LRRK2 in 293 cells (Fig 18), we overexpressed LRRK2 and LRRK2 [G2019S] in 293 cells, but this also failed to induce phosphorylation of ERM proteins (JN, data not shown). Taken together, this suggests that although ERM proteins are efficiently phosphorylated by LRRK2 in vitro, there is no strong evidence that ERM proteins comprise physiological substrates for LRRK2. Recent studies in *Drosophila* [35, 36] and primary mouse lymphocytes [37] have suggested that the SILK/LOK STE20 protein kinase might be a key player in controlling ERM phosphorylation. Consistent with this, ERM phosphorylation is reduced but not abolished in lymphocytes derived from SLK/LOK knockout mice [37]. It was shown in 1994 that SILK has an optimal motif of R-R/K-F-G-SIT-L-R-R-F/I [38], which fits pretty well for the ERM site D-K-Y-K-T-L-R-Q-I and is also remarkably similar to the optimal substrate specificity of LRRK2, W-R-F-Y-T-L-R-R-A. It would also be interesting to test whether residual ERM phosphorylation observed in the SLK/LOK knockout cells was further reduced by treatment with sunitinib and Y-27632 LRRK2 inhibitors. Another recent study has indicated that another STE20 family kinase termed Mst4 kinase can phosphorylate ezrin in polarised epithelial cells in a pathway controlled by the LKB1 tumour suppressor [39]. MST4 was present in our kinase profiling panel and was not inhibited by H-1152, Y-27632 or sunitinib (Table 1).

The finding that widely utilised ROCK inhibitors Y-27632 (used in >1400 papers) as well as H-1152 and hydroxyfasudil inhibit recombinant as well as endogenous LRRK2 with similar potency to that which they target ROCK2 was unexpected, as LRRK2 and ROCK2 are not closely related kinases. LRRK lies within the tyrosine-like kinases of the human kinome whilst ROCK2 belongs to the distinct AGC branch [40]. It is therefore possible that some of the physiological effects observed with these ROCK inhibitors could result from inhibition of LRRK2 rather than ROCK isoforms. The finding that the LRRK2 [G2019S] mutant was 2 to 4-fold more sensitive H-1152, Y-27632 and sunitinib than the wild type LRRK2, also indicates that it may be possible to develop compounds that have greater potency towards the Parkinson's disease mutant. It has also been reported that the LRRK2[G2019S] and LRRK2[I202T] mutants that possess elevated activity were also moderately more sensitive to a panel of non-selective kinase inhibitors [41]. If compounds that specifically inhibited Parkinson's disease mutant forms of LRRK2 could be elaborated, they might have lower side effects and not suppress the normal functions of wild type LRRK2. In drug discovery screens being undertaken to identify LRRK2 inhibitors, it could be beneficial to screen compounds against both mutant and wild type forms of LRRK2. Molecular modelling of the kinase domain of LRRK2 and comparing it with the structures of other kinases revealed a model of how LRRK2 might interact with H-1152. Several residues in the active site of ROCK that are key for binding to H-1152 are also conserved in LRRK2. These include Ala2016, the equivalent of Ala215 in ROCK2 that plays an important role in mediating binding to the inhibitor [31]. Mutation of Ala2016 in LRRK2 to a Thr residue, equivalent to Thr182 in PKA that is weakly inhibited by H-1152, did not affect the basal LRRK2 activity, but markedly suppressed inhibition of LRRK2 by H-1152 and other ROCK kinase inhibitors. The inhibitor resistant LRRK2 [A2016T] mutant might aid in exploring the physiological roles of LRRK2. The wild type and the LRRK2 [A2016T] mutant could be overexpressed in cells and phosphorylation of any target should be less sensitive to inhibition by H-1152, Y-27632 or sunitinib in the cells overexpressing the drug resistant mutant.

Our findings also provide a pharmacological strategy in which phosphorylation of identified LRRK2 substrates could be validated. We suggest that phosphorylation of a LRRK2 substrate should be suppressed by Y-27632 and H-1152 (dual ROCK and LRRK2 inhibitors) as well as sunitinib (inhibits LRRK2 but not ROCK). In contrast, ROCK mediated processes should be sensitive to Y-27632 and H-1152, but should not be inhibited by sunitinib. Consistent with this, sunitinib does not inhibit the phosphorylation of MYPT at Thr850 under conditions where this phosphorylation is inhibited by Y-27632 and H-1152 (Fig 17).

In conclusion, we have undertaken some basic analysis of the LRRK2 substrate specificity and developed improved assays to isolate and assess its activity. This will aid in assessing how LRRK2 is regulated and might also facilitate identification of LRRK2 inhibitors that might have potential for treatment of Parkinson's disease. We have also developed a strategy making use of Y-276332 or H-1152 and sunitinib to explore the roles of the LRRK2 kinase.

### References for Example 4

1 Zimprich, A., Biskup, S., Leitner, P., Lichtner, P., Farrer, M., Lincoln, S., Kachergus, J., Hulihan, M., Uitti, R. J., Calne, D. B., Stoessl, A. J., Pfeiffer, R. F., Patenge, N., Carbajal, I. C., Vieregge, P., Asmus, F., Muller-Myhsok, B., Dickson, D. W., Meitinger, T., Strom, T. M., Wszolek, Z. K. and Gasser, T. (2004) Mutations in LRRK2 cause autosomal-dominant parkinsonism with pleomorphic pathology. Neuron 44, 601-607
2 Paisan-Ruiz, C., Jain, S., Evans, E. W., Gilks, W. P., Simon, J., van der Brug, M., Lopez de Munain, A., Aparicio, S., Gil, A. M., Khan, N., Johnson, J., Martinez, J. R., Nicholl, D., Carrera, I. M., Pena, A. S., de Silva, R., Lees, A., Marti-Masso, J. F., Perez-Tur, J., Wood, N. W. and Singleton, A. B. (2004) Cloning of the gene containing mutations that cause PARKB-linked Parkinson's disease. Neuron 44, 595-600
3 Healy, D. G., Falchi, M., O'Sullivan, S. S., Bonifati, V., Durr, A., Bressman, S., Brice, A., Aasly, J., Zabetian, C. P., Gotdwurm, S., Ferreira, J. J., Tolosa, E., Kay, D. M., Klein, C., Williams, D. R., Marras, C., Lang, A. E., Wszolek, Z. K., Berciano, J., Schapira, A. H., Lynch, T., Bhatia, K. P., Gasser, T., Lees, A. J. and Wood, N. W. (2008) Phenotype, genotype, and worldwide genetic penetrance of LRRK2-associated Parkinson's disease: a case-control study. Lancet Neurol 7, 583-590
4 Mata, I. F., Wedemeyer, W. J., Farrer, M. J., Taylor, J. P. and Gallo, K. A. (2006) LRRK2 in Parkinson's disease: protein domains and functional insights. Trends Neurosci 29, 286-293
5 Biskup, S. and West, A. B. (2008) Zeroing in on LRRK2-linked pathogenic mechanisms in Parkinson's disease. Biochim Biophys Acta
6 Greggio, E. and Cookson, M. R. (2009) Leucine Rich Repeat Kinase 2 mutations and Parkinson's disease: Three Questions. ASN Neuro In Press
7 Covy, J. P. and Giasson, B. I. (2009) Identification of compounds that inhibit the kinase activity of leucine-rich repeat kinase 2. Biochem Biophys Res Commun 378, 473-477
8 Anand, V. S., Reichling, L. J., Lipinski, K., Stochaj, W., Duan, W., Kelleher, K., Pungaliya, P., Brown, E. L., Reinhart, P. H., Somberg, R., Hirst, W. D., Riddle, S. M. and Steven, P. B. (2009) Investigation of leucine-rich repeat kinase 2: enzymological properties and novel assays. Febs J 276, 466-478
9 Jaleel, M., Nichols, R. J., Deak, M., Campbell, D. G., Gillardon, F., Knebel, A. and Alessi, D. R. (2007) LRRK2 phosphorylates moesin at threonine-558: characterization of how Parkinson's disease mutants affect kinase activity. Biochem J 405, 307-317
10 Matsui, T., Maeda, M., Doi, Y., Yonemura, S., Amano, M., Kaibuchi, K., Tsukita, S. and Tsukita, S. (1998) Rho-kinase phosphorylates COOH-terminal threonines of ezrin/radixin/moesin (ERM) proteins and regulates their head-to-tail association. J Cell Biol 140, 647-657
11 Oshiro, N., Fukata, Y. and Kaibuchi, K. (1998) Phosphorylation of moesin by rhoassociated kinase (Rho-kinase) plays a crucial role in the formation of microvilli-like structures. J Biol Chem 273, 34663-34666
12 Tran Quang, C., Gautreau, A., Arpin, M. and Treisman, R. (2000) Ezrin function is required for ROCK-mediated fibroblast transformation by the Net and Dbl oncogenes. Embo J 19, 4565-4576
14 Reed, S. E., Staley, E. M., Mayginnes, J. P., Pintel, D. J. and Tullis, G. E. (2006) Transfection of mammalian cells using linear polyethylenimine is a simple and effective means of producing recombinant adeno-associated virus vectors. J Virol Methods 138, 85-98
15 Biskup, S., Moore, D. J., Rea, A., Lorenz-Deperieux, B., Coombes, C. E., Dawson, V. L., Dawson, T. M. and West, A. B. (2007) Dynamic and redundant regulation of LRRK2 and LRRK1 expression. BMC Neurosci 8, 102
16 Bain, J., Plater, L., Elliott, M., Shpiro, N., Hastie, C. J., McLauchlan, H., Klevernic, I., Arthur, J. S.. Alessi, D. R. and Cohen, P. (2007) The selectivity of protein kinase inhibitors: a further update. Biochem J 408, 297-315
17 Sward, K., Dreja, K., Susnjar, M., Hellstrand, P., Hartshorne, D. J. and Walsh, M. P. (2000) Inhibition of Rho-associated kinase blocks agonist-induced Ca2+ sensitization of myosin phosphorylation and force in guinea-pig ileum. J Physiol 522 Pt 1, 33-49
18 Feng, J., Ito, M., Ichikawa, K., Isaka, N., Nishikawa, M., Hartshorne, D. J. and Nakano, T. (1999) Inhibitory phosphorylation site for Rho-associated kinase on smooth muscle myosin phosphatase. J Biol Chem 274, 37385-37390
19 Hutti, J. E., Jarrell, E. T., Chang, J. D., Abbott, D. W., Storz, P., Toker, A., Cantley, L. C. and Turk, B. E. (2004) A rapid method for determining protein kinase phosphorylation specificity. Nat Methods 1, 27-29
20 Turk, B. E., Hutti, J. E. and Cantley, L. C. (2006) Determining protein kinase substrate specificity by parallel solution-phase assay of large numbers of peptide substrates. Nat Protoc 1, 375-379
21 Workman, C. T., Yin, Y., Corcoran, D. L., Ideker, T., Stormo, G. D. and Benos, P. V. (2005) enoLOGOS: a versatile web tool for energy normalized sequence logos. Nucleic Acids Res 33, W389-392
22 Hutti, J. E., Turk, B. E., Asara, J. M., Ma, A., Cantley, L. C. and Abbott, D. W. (2007) IkappaB kinase beta phosphorylates the K63 deubiquitinase A20 to cause feedback inhibition of the NF-kappaB pathway. Mol Cell Biol 27, 7451-7461
23 Ishizaki, T., Uehata, M., Tamechika, I., Keel, J., Nonomura, K., Maekawa, M. and Narumiya, S. (2000) Pharmacological properties of Y-27632, a specific inhibitor of rho-associated kinases. Mol Pharmacol 57, 976-983
24 Shimokawa, H., Seto, M., Katsumata, N., Amano, M., Kozai, T., Yamawaki, T., Kuwata, K., Kandabashi, T., Egashira, K., Ikegaki, I., Asano, T., Kaibuchi, K. and Takeshita, A. (1999) Rho-kinase-mediated pathway induces enhanced myosin light chain phosphorylations in a swine model of coronary artery spasm. Cardiovasc Res 43, 1029-1039
25 Sasaki, Y., Suzuki, M. and Hidaka, H. (2002) The novel and specific Rho-kinase inhibitor (S)-(+)-2-methyl-1-[(4-methyl-5-isoquinoline)sulfonyl]-homopiperazine as a probing molecule for Rho-kinase-involved pathway. Pharmacol Ther 93, 225-232
26 Tamura, M., Nakao, H., Yoshizaki, H., Shiratsuchi, M., Shigyo, H., Yamada, H., Ozawa, T., Totsuka, J. and Hidaka, H. (2005) Development of specific Rho-kinase inhibitors and their clinical application. Biochim Biophys Acta 1754, 245-252
27 Chijiwa, T., Mishima, A., Hagiwara, M., Sano, M., Hayashi, K., Inoue, T., Naito, K., Toshioka, T. and Hidaka, H. (1990) Inhibition of forskolin-induced neurite outgrowth and protein phosphorylation by a newly synthesized selective inhibitor of cyclic AMP-dependent protein kinase, N-[2-(p-bromocinnamylamino)ethyl]-5-isoquinolinesulfonamide (H-89), of PC12D pheochromocytoma cells. J Biol Chem 265, 5267-5272
28 Shibuya, M., Suzuki, Y., Sugita, K., Saito, L, Sasaki, T., Takakura, K., Nagata, I., Kikuchi, H., Takemae, T., Hidaka, H. and et al. (1992) Effect of AT877 on cerebral vasospasm after aneurysmal subarachnoid hemorrhage. Results of a prospective placebo-controlled double-blind trial. J Neurosurg 76, 571-577
29 Doe, C., Bentley, R., Behm, D. J., Lafferty, R., Stavenger, R., Jung, D., Bamford, M., Panchal, T., Grygielko, E., Wright, L. L., Smith, G. K., Chen, 2., Webb, C., Khandekar, S., Yi, T., Kirkpatrick, R., Dul, E., Jolivette, L., Marino, J. P., Jr., Willette, R., Lee, D. and Hu, E. (2007) Novel Rho kinase inhibitors with anti-inflammatory and vasodilatory activities. J Pharmacol Exp Ther 320, 89-98
30 Yamaguchi, H., Kasa, M., Amano, M., Kaibuchi, K. and Hakoshima, T. (2006) Molecular mechanism for the regulation of rho-kinase by dimerization and its inhibition by fasudil. Structure 14, 589-600 16
31 Jacobs, M., Hayakawa, K., Swenson, L., Bellon, S., Fleming, M., Taslimi, P. and Doran, J. (2006) The structure of dimeric ROCK I reveals the mechanism for ligand selectivity. J Biol Chem 281, 260-268
32 Bonn, S., Herrero, S., Breitenlechner, C. B., Erlbruch, A., Lehmann, W., Engh, R. A., Gassel, M. and Bossemeyer, D. (2006) Structural analysis of protein kinase A mutants with Rho-kinase inhibitor specificity. J Biol Chem 281, 24818-24830
33 Matsui, T., Yonemura, S., Tsukita, S. and Tsukita, S. (1999) Activation of ERM proteins in vivo by Rho involves phosphatidyl-inositol 4-phosphate 5-kinase and not ROCK kinases. Curr Biol 9, 1259-1262
34 Lee, J. H., Katakai, T., Hara, T., Gonda, H., Sugai, M. and Shimizu, A. (2004) Roles of p-ERM and Rho-ROCK signaling in lymphocyte polarity and uropod formation. J Cell Biol 167, 327-337
35 Carreno, S., Kouranti, I., Glusman, E. S., Fuller, M. T., Echard, A. and Payre, F. (2008) Moesin and its activating kinase Slik are required for cortical stability and microtubule organization in mitotic cells. J Cell Biol 180, 739-746
36 Kunda, P., Pelling, A. E., Liu, T. and Baum, B. (2008) Moesin controls cortical rigidity, cell rounding, and spindle morphogenesis during mitosis. Curr Biol 18, 91-101
37 Belkina, N. V., Liu, Y., Hao, J. J., Karasuyama, H. and Shaw, S. (2009) LOK is a major ERM kinase in resting lymphocytes and regulates cytoskeletal rearrangement through ERM phosphorylation. Proc Natl Acad Sci U S A 106, 4707-4712
38 Songyang, Z., Blechner, S., Hoagland, N., Hoekstra, M. F., Piwnica-Worms, H. and Cantley, L. C. (1994) Use of an oriented peptide library to determine the optimal substrates of protein kinases. Curr Biol 4, 973-982
39 ten Klooster, J. P., Jansen, M., Yuan, J., Oorschot, V., Begthel, H., Di Giacomo, V., Colland, F., de Koning, J., Maurice, M. M., Hornbeck, P. and Clevers, H. (2009) Mst4 and Ezrin induce brush borders downstream of the Lkb1/Strad/Mo25 polarization complex. Dev Cell 16, 551-562
40 Manning, G., Whyte, D. B., Martinez, R., Hunter, T. and Sudarsanam, S. (2002) The protein kinase complement of the human genome. Science 298, 1912-1934
41 Reichling, L. J. and Riddle, S. M. (2009) Leucine-rich repeat kinase 2 mutants I2020T and G2019S exhibit altered kinase inhibitor sensitivity. Biochem Biophys Res Commun 384, 255-258

### Example 5: GST-Nictide

GST-Nictide and a plasmid useful in expressing GST-Nictide, as used in the preceding examples, are shown below.

| | |
|---|---|
| Plasmid Name | GEX6-LRKK2-TIDE (nic-tide) |
| Protein | GST-LRKK2-TIDE |
| Species | **Artificial** |
| Parental plasmid | **Gex6-P1** |
| Insert Source | |
| Restriction sites used | **Bamh1-Not1** |
| Protein sequence GST italic Precision site bold | MSPILGYWKIKGLVQPTRLLLEYLEEKYEEHLYERDEGDKWRNKKFELGLEFPNLPY YIDGDVKLTQSMAIIRYIADKHNMLGGCPKERAEISMLEGAVLDIRYGVSRIAYSKDFE TLKVDFLSKLPEMLKMFEDRLCHKTYLNGDHVTHPDFMLYDALDVVLYMDPMCLDA FPKLVCFKKRIEAIPQIDKYLKSSKYIAWPLQGWQATFGGGDHPPKSDLEVLFQGPL |
| Peptide underlined | GSRLGWWRFYTLRRARQGNTKQR |
| DNA sequence of GST-nictide | ATGTCCCCTATACTAGGTTATTGGAAAATTAAGGGCCTTGTGCAACCCACTCGAC TTCTTTTGGAATATCTTGAAGAAAAATATGAAGAGCATTTGTATGAGCGCGATGAA GGTGATAAATGGCGAAACAAAAAGTTTGAATTGGGTTTGGAGTTTCCCAATCTTC CTTATTATATTGATGGTGATGTTAAATTAACACAGTCTATGGCCATCATACGTTATA TAGCTGACAAGCACAACATGTTGGGTGGTTGTCCAAAAGAGCGTGCAGAGATTT CAATGCTTGAAGGAGCGGTTTTGGATATTAGATACGGTGTTTCGAGAATTGCATA TAGTAAAGACTTTGAAACTCTCAAAGTTGATTTTCTTAGCAAGCTACCTGAAATGC TGAAAATGTTCGAAGATCGTTTATGTCATAAAACATATTTAAATGGTGATCATGTA ACCCATCCTGACTTCATGTTGTATGACGCTCTTGATGTTGTTTTATACATGGACCC AATGTGCCTGGATGCGTTCCCAAAATTAGTTTGTTTTAAAAAACGTATTGAAGCTA TCCCACAAATTGATAAGTACTTGAAATCCAGCAAGTATATAGCATGGCCTTTGCA GGGCTGGCAAGCCACGTTTGGTGGTGGCGACCATCCTCCAAAATCGGATCTGGA AGTTCTGTTCCAGGGGCCCCTGGGATCCAGACTAGGTTGGTGGAGATTTTATAC ACTACGACGGGCCAGGCAGGGCAATACAAAGCAGAGATAGCGGCCGC |
| DNA sequence of the Gex6-GST-Nictide vector | ACGTTATCGACTGCACGGTGCACCAATGCTTCTGGCGTCAGGCAGCCATCGGAA GCTGTGGTATGGCTGTGCAGGTCGTAAATCACTGCATAATTCGTGTCGCTCAAG GCGCACTCCCGTTCTGGATAATGTTTTTTGCGCCGACATCATAACGGTTCTGGCA AATATTCTGAAATGAGCTGTTGACAATTAATCATCGGCTCGTATAATGTGTGGAAT TGTGAGCGGATAACAATTTCACACAGGAAACAGTATTCATGTCCCCTATACTAGG TTATTGGAAAATTAAGGGCCTTGTGCAACCCACTCGACTTCTTTTGGAATATCTTG AAGAAAAATATGAAGAGCATTTGTATGAGCGCGATGAAGGTGATAAATGGCGAAA CAAAAAGTTTGAATTGGGTTTGGAGTTTCCCAATCTTCCTTATTATATTGATGGTG ATGTTAAATTAACACAGTCTATGGCCATCATACGTTATATAGCTGACAAGCACAAC ATGTTGGGTGGTTGTCCAAAAGAGCGTGCAGAGATTTCAATGCTTGAAGGAGCG GTTTTGGATATTAGATACGGTGTTTCGAGAATTGCATATAGTAAAGACTTTGAAAC TCTCAAAGTTGATTTTCTTAGCAAGCTACCTGAAATGCTGAAAATGTTCGAAGATC GTTTATGTCATAAAACATATTTAAATGGTGATCATGTAACCCATCCTGACTTCATG TTGTATGACGCTCTTGATGTTGTTTTATACATGGACCCAATGTGCCTGGATGCGTT CCCAAAATTAGTTTGTTTTAAAAAACGTATTGAAGCTATCCCACAAATTGATAAGT ACTTGAAATCCAGCAAGTATATAGCATGGCCTTTGCAGGGCTGGCAAGCCACGTT TGGTGGTGGCGACCATCCTCCAAAATCGGATCTGGAAGTTCTGTTCCAGGGGCC CCTGGGATCCAGACTAGGTTGGTGGAGATTTTATACACTACGACGGCCAGGCA GGGCAATACAAAGCAGAGATAGCGGCCGCATCGTGACTGACTGACGATCTGCCT CGCGCGTTTCGGTGATGACGGTGAAAACCTCTGACACATGCAGCTCCCGGAGAC GGTCACAGCTTGTCTGTAAGCGGATGCCGGGAGCAGACAAGCCCGTCAGGGCG CGTCAGCGGGTGTTGGCGGGTGTCGGGGCGCAGCCATGACCCAGTCACGTAGC GATAGCGGAGTGTATAATTCTTGAAGACGAAAGGGCCTCGTGATACGCCTATTTT TATAGGTTAATGTCATGATAATAATGGTTTCTTAGACGTCAGGTGGCACTTTTCGG GGAAATGTGCGCGGAACCCCTATTTGTTTATTTTTCTAAATACATTCAAATATGTA TCCGCTCATGAGACAATAACCCTGATAAATGCTTCAATAATATTGAAAAAGGAAGA GTATGAGTATTCAACATTTCCGTGTCGCCCTTATTCCCTTTTTTGCGGCATTTTGC CTTCCTGTTTTTGCTCACCCAGAAACGCTGGTGAAAGTAAAAGATGCTGAAGATC AGTTGGGTGCACGAGTGGGTTACATCGAACTGGATCTCAACAGCGGTAAGATCC |
| | TTGAGAGTTTTCGCCCCGAAGAACGTTTTCCAATGATGAGCACTTTTAAAGTTCTG CTATGTGGCGCGGTATTATCCCGTGTTGACGCCGGGCAAGAGCAACTCGGTCGC CGCATACACTATTCTCAGAATGACTTGGTTGAGTACTCACCAGTCACAGAAAAGC ATCTTACGGATGGCATGACAGTAAGAGAATTATGCAGTGCTGCCATAACCATGAG TGATAACACTGCGGCCAACTTACTTCTGACAACGATCGGAGGACCGAAGGAGCT AACCGCTTTTTTGCACAACATGGGGGATCATGTAACTCGCCTTGATCGTTGGGAA CCGGAGCTGAATGAAGCCATACCAAACGACGAGCGTGACACCACGATGCCTGCA GCAATGGCAACAACGTTGCGCAAACTATTAACTGGCGAACTACTTACTCTAGCTT CCCGGCAACAATTAATAGACTGGATGGAGGCGGATAAAGTTGCAGGACCACTTC TGCGCTCGGCCCTTCCGGCTGGCTGGTTTATTGCTGATAAATCTGGAGCCGGTG AGCGTGGGTCTCGCGGTATCATTGCAGCACTGGGGCCAGATGGTAAGCCCTCC CGTATCGTAGTTATCTACACGACGGGGAGTCAGGCAACTATGGATGAACGAAATA GACAGATCGCTGAGATAGGTGCCTCACTGATTAAGCATTGGTAACTGTCAGACCA AGTTTACTCATATATACTTTAGATTGATTTAAAACTTCATTTTTAATTTAAAAGGATC TAGGTGAAGATCCTTTTTGATAATCTCATGACCAAAATCCCTTAACGTGAGTTTTC GTTCCACTGAGCGTCAGACCCCGTAGAAAAGATCAAAGGATCTTCTTGAGATCCT TTTTTTCTGCGCGTAATCTGCTGCTTGCAAACAAAAAAACCACCGCTACCAGCGG TGGTTTGTTTGCCGGATCAAGAGCTACCAACTCTTTTTCCGAAGGTAACTGGCTT CAGCAGAGCGCAGATACCAAATACTGTCCTTCTAGTGTAGCCGTAGTTAGGCCA CCACTTCAAGAACTCTGTAGCACCGCCTACATACCTCGCTCTGCTAATCCTGTTA CCAGTGGCTGCTGCCAGTGGCGATAAGTCGTGTCTTACCGGGTTGGACTCAAGA CGATAGTTACCGGATAAGGCGCAGCGGTCGGGCTGAACGGGGGGTTCGTGCAC ACAGCCCAGCTTGGAGCGAACGACCTACACCGAACTGAGATACCTACAGCGTGA GCTATGAGAAAGCGCCACGCTTCCCGAAGGGAGAAAGGCGGACAGGTATCCGG TAAGCGGCAGGGTCGGAACAGGAGAGCGCACGAGGGAGCTTCCAGGGGGAAA CGCCTGGTATCTTTATAGTCCTGTCGGGTTTCGCCACCTCTGACTTGAGCGTCGA TTTTTGTGATGCTCGTCAGGGGGGCGGAGCCTATGGAAAAACGCCAGCAACGCG GCCTTTTTACGGTTCCTGGCCTTTTGCTGGCCTTTTGCTCACATGTTCTTTCCTGC GTTATCCCCTGATTCTGTGGATAACCGTATTACCGCCTTTGAGTGAGCTGATACC GCTCGCCGCAGCCGAACGACCGAGCGCAGCGAGTCAGTGAGCGAGGAAGCGG AAGAGCGCCTGATGCGGTATTTTCTCCTTACGCATCTGTGCGGTATTTCACACCG CATAAATTCCGACACCATCGAATGGTGCAAAACCTTTCGCGGTATGGCATGATAG CGCCCGGAAGAGAGTCAATTCAGGGTGGTGAATGTGAAACCAGTAACGTTATAC GATGTCGCAGAGTATGCCGGTGTCTCTTATCAGACCGTTTCCCGCGTGGTGAAC CAGGCCAGCCACGTTTCTGCGAAAACGCGGGAAAAAGTGGAAGCGGCGATGGC GGAGCTGAATTACATTCCCAACCGCGTGGCACAACAACTGGCGGGCAAACAGTC GTTGCTGATTGGCGTTGCCACCTCCAGTCTGGCCCTGCACGCGCCGTCGCAAAT TGTCGCGGCGATTAAATCTCGCGCCGATCAACTGGGTGCCAGCGTGGTGGTGTC GATGGTAGAACGAAGCGGCGTCGAAGCCTGTAAAGCGGCGGTGCACAATCTTCT CGCGCAACGCGTCAGTGGGCTGATCATTAACTATCCGCTGGATGACCAGGATGC CATTGCTGTGGAAGCTGCCTGCACTAATGTTCCGGCGTTATTTCTTGATGTCTCT GACCAGACACCCATCAACAGTATTATTTTCTCCCATGAAGACGGTACGCGACTGG GCGTGGAGCATCTGGTCGCATTGGGTCACCAGCAAATCGCGCTGTTAGCGGGC CCATTAAGTTCTGTCTCGGCGCGTCTGCGTCTGGCTGGCTGGCATAAATATCTCA CTCGCAATCAAATTCAGCCGATAGCGGAACGGGAAGGCGACTGGAGTGCCATGT CCGGTTTTCAACAAACCATGCAAATGCTGAATGAGGGCATCGTTCCCACTGCGAT GCTGGTTGCCAACGATCAGATGGCGCTGGGCGCAATGCGCGCCATTACCGAGT |
| | CCGGGCTGCGCGTTGGTGCGGATATCTCGGTAGTGGGATACGACGATACCGAA GACAGCTCATGTTATATCCCGCCGTCAACCACCATCAAACAGGATTTTCGCCTGC TGGGGCAAACCAGCGTGGACCGCTTGCTGCAACTCTCTCAGGGCCAGGCGGTG AAGGGCAATCAGCTGTTGCCCGTCTCACTGGTGAAAAGAAAAACCACCCTGGCG CCCAATACGCAAACCGCCTCTCCCCGCGCGTTGGCCGATTCATTAATGCAGCTG GCACGACAGGTTTCCCGACTGGAAAGCGGGCAGTGAGCGCAACGCAATTAATGT GAGTTAGCTCACTCATTAGGCACCCCAGGCTTTACACTTTATGCTTCCGGCTCGT ATGTTGTGTGGAATTGTGAGCGGATAACAATTTCACACAGGAAACAGCTATGACC ATGATTACGGATTCACTGGCCGTCGTTTTACAACGTCGTGACTGGGAAAACCCTG GCGTTACCCAACTTAATCGCCTTGCAGCACATCCCCCTTTCGCCAGCTGGCGTAA TAGCGAAGAGGCCCGCACCGATCGCCCTTCCCAACAGTTGCGCAGCCTGAATG GCGAATGGCGCTTTGCCTGGTTTCCGGCACCAGAAGCGGTGCCGGAAAGCTGG CTGGAGTGCGATCTTCCTGAGGCCGATACTGTCGTCGTCCCCTCAAACTGGCAG ATGCACGGTTACGATGCGCCCATCTACACCAACGTAACCTATCCCATTACGGTCA ATCCGCCGTTTGTTCCCACGGAGAATCCGACGGGTTGTTACTCGCTCACATTTAA TGTTGATGAAAGCTGGCTACAGGAAGGCCAGACGCGAATTATTTTTGATGGCGTT GGAATT |

## Claims

1. A method for identifying a compound expected to be useful in modulating, for example inhibiting, LRRK2 protein kinase activity, the method comprising the steps of (1) determining whether a test compound modulates, for example inhibits, the protein kinase activity of a LRRK2 polypeptide on a substrate polypeptide and (2) selecting a compound which modulates, for example inhibits, the said LRRK2 polypeptide protein kinase activity, I wherein the substrate polypeptide comprises the sequence (W/F/R/K)(W/F/R/K)(R/K)(F/W/H/R)(Y/W/R)(S/T)(L/V/I)(R/K)(R/K)(A/Y) or (W/R)(X)(X)(F/Y/H/T)(Y/W/R)(T)(X)(R/T)(R)(X), where X represents any amino acid.

2. The method of Claim 1, wherein the method is for identifying a compound expected to be useful in treating or preventing Parkinson's Disease (PD) or Parkinsonism or other neurodegenerative condition.

3. The method of claim 1 wherein the LRRK2 polypeptide is wild type human LRRK2, a fragment thereof, or a fusion of either the wild type human LRRK2 or the fragment; wherein the fragment comprises at least residues 1326-2527 of wild type human LRRK2.

4. The method of claim 1 or 3 wherein the LRRK2 polypeptide is human LRRK2 having a naturally occurring mutation of wild type human LRRK2; a fragment thereof wherein the fragment corresponds to at least residues 1326-2527 of human LRRK2; or a fusion of either the human LRRK2 having a naturally occurring mutation of wild type human LRRK2 or the fragment; wherein optionally the naturally occurring mutation of human LRRK2 is a mutation associated with Parkinson's Disease (PD), wherein optionally the mutation, using the numbering of wild type human LRRK2, is G2019S or the mutation, using the numbering of wild type human LRRK2, is R1441C, R1441G, Y1699C, R1914H, I2012T, I2020T, T2356I, G2385R, K544E, P755L, R793M, Q930R, S973N, R1067Q, S1096C, I1122V, S1228T, I1371V, R1441H, A1442P, R1514Q, M1869T or G2019S.

5. The method of any one of claims 1, 3 to 4 wherein the LRRK2 polypeptide is a GST fusion polypeptide, for example GST-LRRK2[1326-2527, G2019S] and/or wherein the LRRK2 polypeptide is recombinant.

6. The method of any one of the preceding claims wherein the substrate polypeptide consists of or comprises the sequence WWKFYTLRRA. WWRFYTLRKA. RLGWWKFYTLRRARQGNTKQR, RLGWWRFYTLRKARQGNTKQR or RLGWWRFYTLRRARQGNTKQR and/or wherein the substrate polypeptide is a GST fusion polypeptide, for example GST- RLGWWRFYTLRRARQGNTKQR.

7. A purified preparation comprising i) a LRRK2 polypeptide or recombinant LRRK2 polynucleotide or antibody capable of binding to LRRK2 obtained or obtainable by a method comprising the step of raising the antibody to, or selecting the antibody on the basis of binding to, a polypeptide consisting of residues 100 to 498, or residues 100 to 500, of LRRK2, a fragment thereof, or a fusion of either the polypeptide or the fragment, other than with an LRRK2-derived sequence and ii) a substrate polypeptide as defined in any one of the preceding claims or a polynucleotide encoding a substrate polypeptide as defined in any one of the preceding claims.

8. A kit of parts comprising i) a LRRK2 polypeptide or recombinant LRRK2 polynucleotide or antibody capable of binding to LRRK2 obtained or obtainable by a method comprising the step of raising the antibody to, or selecting the antibody on the basis of binding to, a polypeptide consisting of residues 100 to 498, or residues 100 to 500, of LRRK2, a fragment thereof, or a fusion of either the polypeptide or the fragment, other than with an LRRK2-derived sequence and ii) a substrate polypeptide as defined in any one of the preceding claims or a polynucleotide encoding a substrate polypeptide as defined in any one of the preceding claims.

9. A recombinant cell capable of expressing a LRRK2 polypeptide and substrate polypeptide as defined in any one of claims 1 to 6 and comprising a recombinant LRRK2 polynucleotide and a recombinant polynucleotide encoding the substrate polypeptide.

10. A polypeptide comprising the sequence (W/F/R/K)(W/F/R/K)(R/K)(F/W/H/R)(Y/W/R)(T)(L/V/I)(R/K)(R/K)(A/Y) or (W/R)(X)(X)(F/Y/H/T)(Y/W/R)(T)(X)(R/T)(R)(X), where X represents any amino acid.

11. The polypeptide of claim 10 consisting of or comprising the sequence WWKFYTLRRA, WWRFYTLRKA, RLGWWKFYTLRRARQGNTKQR. RLGWWRFYTLRKARQGNTKQR or RLGWWRFYTLRRARQGNTKQ and/or wherein the polypeptide is a GST fusion polypeptide, for example GST-RLGWWRFYTLRRARQGNTKQR.

12. A polynucleotide encoding a polypeptide according to claim 10 or 11.

13. The method of any one of claims 1 to 6 comprising the step of assessing whether the compound modulates ERM family polypeptide phosphorylation in a whole cell, tissue or organism; or characteristics of Parkinsonism or Parkinson's Disease in an organism and a compound that modulates phosphorylation or disease characteristics is selected; optionally comprising the step of assessing whether the compound modulates the activity of an ERM family polypeptide in the whole cell, tissue or organism, and a compound that modulates the activity is selected.

## Patentansprüche

1. Verfahren zur Identifizierung einer Verbindung, von der erwartet wird, dass sie zur Modulierung, beispielsweise Hemmung, der LRRK2-Proteinkinaseaktivität verwendbar ist, wobei das Verfahren die Stufen (1) Bestimmen, ob eine Testverbindung die Proteinkinaseaktivität eines LRRK2-Polypeptids auf ein Substratpolypeptid moduliert, beispielsweise hemmt, und (2) Selektieren einer Verbindung, die die LLRK2-Polypeptid-Proteinkinaseaktivität moduliert, beispielsweise hemmt, wobei das Substratpolypeptid die Sequenz 
(W/F/R/K) (W/F/R/K) (R/K) (F/W/H/R) (Y/W/R) (S/T) (L/V/I) (R/K) (R/K)
(A/Y) oder (W/R) (X) (X) (F/Y/H/T) (Y/W/R) (T) (X) (R/T) (R) (X),
 wobei X für eine beliebige Aminosäure steht, umfasst.

2. Verfahren nach Anspruch 1, wobei das Verfahren zur Identifizierung einer Verbindung, von der erwartet wird, dass sie zur Behandlung oder Prävention von Parkinson-Krankheit (PD) oder Parkinsonismus oder einer anderen neurodegenerativen Erkrankung verwendbar ist, dient.

3. Verfahren nach Anspruch 1, wobei das LRRK2-Polypeptid der Wildtyp von humanem LRRK2, ein Fragment hiervon oder eine Fusion von entweder dem Wildtyp von humanem LRRK2 oder dem Fragment ist, wobei das Fragment mindestens die Reste 1326-2527 des Wildtyps von humanem LRRK2 umfasst.

4. Verfahren nach Anspruch 1 oder 3, wobei das LRRK2-Polypeptid ein humanes LRRK2, das eine natürlich vorkommende Mutation des Wildtyps von humanem LRRK2 aufweist; ein Fragment hiervon, wobei das Fragment mindestens den Resten 1326-2527 von humanem LRRK2 entspricht; oder eine Fusion von entweder dem humanen LRRK2, das eine natürlich vorkommende Mutation des Wildtyps von humanem LRRK2 aufweist, oder dem Fragment ist; wobei optional die natürlich vorkommende Mutation von humanem LRRK2 eine Mutation ist, die mit Parkinson-Krankheit (PD) in Verbindung steht, wobei optional die Mutation, unter Verwendung der Nummerierung des Wildtyps von humanem LRRK2, G2019S ist oder die Mutation, unter Verwendung der Nummerierung des Wildtyps von humanem LRRK2, R1441C, R1441G, Y1699C, R1914H, I2012T, I2020T, T2356I, G2358R, K544E, P755L, R793M, Q930R, S973N, R1067Q, S1096C, I1122V, S1228T, I1371V, R1441H, A1442P, R1514Q, M1869T oder G2019S ist.

5. Verfahren nach einem der Ansprüche 1, 3 bis 4, wobei das LRRK2-Polypeptid ein GST-Fusionspolypeptid, beispielsweise GST-LRRK2[1326-2527, G2019S], ist und/oder wobei das LRRK2-Polypetid rekombinant ist.

6. Verfahren nach einem der vorhergehenden Ansprüche, wobei das Substratpolypeptid aus der Sequenz WWKFYTLRRA, WWRFYTLRKA, RLGWWKFYTLRRARQGNTKQR, RLGWWRFYTLRKARQGNTKQR oder RLGWWRFYTLRRARQGNTKQR besteht oder diese umfasst und/oder wobei das Substratpolypeptid ein GST-Fusionspolypeptid, beispielsweise GST-RLGWWRFYTLRRARQGNTKQR, ist.

7. Gereinigte Zubereitung, umfassend i) ein LRRK2-Polypeptid oder rekombinantes LRRK2-Polynucleotid oder einen Antikörper, der zur Bindung an LRRK2 fähig ist, der durch ein Verfahren erhalten wird oder erhalten werden kann, das die Stufe der Bildung des Antikörpers gegen oder des Selektierens des Antikörpers auf der Basis der Bindung an ein Polypeptid, das aus den Resten 100 bis 498 oder den Resten 100 bis 500 von LRRK2 besteht, umfasst, ein Fragment hiervon oder ein Fusionsprodukt von entweder dem Polypeptid oder dem Fragment mit einer anderen als einer von LRRK2 abgeleiteten Sequenz und ii) ein Substratpolypeptid gemäß der Definition in einem der vorhergehenden Ansprüche oder ein Polynucleotid mit Codierung für ein Substratpolypeptid gemäß der Definition in einem der vorhergehenden Ansprüche.

8. Kit aus Teilen umfassend i) ein LRRK2-Polypeptid oder rekombinantes LRRK2-Polynucleotid oder einen Antikörper, der zur Bindung an LRRK2 fähig ist, der durch ein Verfahren erhalten wird oder erhalten werden kann, das die Stufe der Bildung des Antikörpers gegen oder des Selektierens des Antikörpers auf der Basis der Bindung an ein Polypeptid, das aus den Resten 100 bis 498 oder den Resten 100 bis 500 von LRRK2 besteht, umfasst, ein Fragment hiervon oder ein Fusionsprodukt von entweder dem Polypeptid oder dem Fragment mit einer anderen als einer von LRRK2 abgeleiteten Sequenz und ii) ein Substratpolypeptid gemäß der Definition in einem der vorhergehenden Ansprüche oder ein Polynucleotid mit Codierung für ein Substratpolypeptid gemäß der Definition in einem der vorhergehenden Ansprüche.

9. Rekombinante Zelle, die zur Expression eines LRRK2-Polypeptids und eines Substratpolypeptids gemäß der Definition in einem der Ansprüche 1 bis 6 fähig ist und ein rekombinantes LRRK2-Polynucleotid und ein rekombinantes Polynucleotid mit Codierung für das Substratpolypeptid umfasst.

10. Polypeptid, das die Sequenz
(W/F/R/K) (W/F/R/K) (R/K) (F/W/H/R) (Y/W/R) (T) (L/V/I) (R/K) (R/K) (A /Y) oder (W/R) (X) (X) (F/Y/H/T) (Y/W/R) (T) (X) (R/T) (R) (X), worin X für eine beliebige Aminosäure steht, umfasst.

11. Polypeptid nach Anspruch 10, das aus der Sequenz WWKFYTLRRA, WWRFYTLRKA, RLGWWKFYTLRRARQGNTKQR, RLGWWRFYTLRKARQGNTKQR oder RLGWWRFYTLRRARQGNTKQ besteht oder diese umfasst und/oder wobei das Polypeptid ein GST-Fusionspolypeptid, beispielsweise GST-RLGWWRFYTLRRARQGNTKQR, ist.

12. Polynucleotid mit Codierung für ein Polypeptid nach Anspruch 10 oder 11.

13. Verfahren nach einem der Ansprüche 1 bis 6, das die Stufe des Feststellens, ob die Verbindung die Phosphorylierung von Polypeptiden der ERM-Familie in einer ganzen Zelle, einem Gewebe oder einem Organismus oder Merkmale von Parkinsonismus oder Parkinson-Krankheit in einem Organismus moduliert, und das Selektieren einer Verbindung, die die Phosphorylierung oder Krankheitsmerkmale moduliert, umfasst; optional die Stufe des Feststellens, ob die Verbindung die Aktivität eines Polypeptids der ERM-Familie in der Vollzelle, dem Gewebe oder Organismus moduliert, und das Selektieren einer Verbindung, die die Aktivität moduliert, umfasst.

## Revendications

1. Procédé pour identifier un composé susceptible d'être utile dans la modulation, par exemple l'inhibition, d'une activité de protéine kinase LRRK2, le procédé comprenant les étapes consistant à (1) déterminer si un composé à tester module, par exemple inhibe, l'activité de protéine kinase d'un polypeptide LRRK2 sur un polypeptide substrat et (2) sélectionner un composé qui module, par exemple inhibe, ladite activité de protéine kinase du polypeptide LRRK2, dans lequel ledit polypeptide substrat comprend la séquence (W/F/R/K) (W/F/R/K) (R/K) (F/W/H/R) (Y/W/R) (S/T) (L/V/I) (R/K) (R/K) (A/Y) OU (W/R) (X) (X) (F/Y/H/T) (Y/W/R) (T) (X) (R/T) (R) (X), où X représente tout acide aminé.

2. Procédé selon la revendication 1, dans lequel le procédé est destiné à identifier un composé susceptible d'être utile dans le traitement ou la prévention de la maladie de Parkinson (MP) ou le parkinsonisme ou une autre affection neurodégénérative.

3. Procédé selon la revendication 1, dans lequel le polypeptide LRRK2 est la LRRK2 humaine de type sauvage, un fragment de celle-ci, ou une fusion soit de la LRRK2 humaine de type sauvage, soit du fragment ; dans lequel le fragment comprend au moins les résidus 1326 à 2527 de la LRRK2 humaine de type sauvage.

4. Procédé selon la revendication 1 ou 3, dans lequel le polypeptide LRRK2 est une LRRK2 humaine ayant une mutation naturelle de LRRK2 humaine de type sauvage ; un fragment de celle-ci dans lequel le fragment correspond au moins aux résidus 1326 à 2527 de la LRRK2 humaine ; ou une fusion soit de la LRRK2 humaine de type sauvage ayant une mutation naturelle de LRRK2 humaine de type sauvage, soit du fragment ; dans lequel éventuellement la mutation naturelle de la LRRK2 humaine est une mutation associée à la maladie de Parkinson (MP), dans lequel éventuellement la mutation, en utilisant la numérotation de la LRRK2 humaine de type sauvage, est G2019S ou la mutation, en utilisant la numérotation de la LRRK2 humaine de type sauvage, est R1441C, R1441G, Y1699C, R1914H, I2012T, I2020T, T2356I, G2385R, K544E, P755L, R793M, Q930R, S973N, R1067Q, S1096C, I1122V, S1228T, I1371V, R1441H, A1442P, R1514Q, M1869T ou G2019S.

5. Procédé selon l'une quelconque des revendications 1, 3 à 4, dans lequel le polypeptide LRRK2 est un polypeptide de fusion GST, par exemple GST-LRRK2[1326-2527, G2019S] et/ou dans lequel le polypeptide LRRK2 est recombinant.

6. Procédé selon l'une quelconque des revendications précédentes, dans lequel le polypeptide substrat consiste en par ou comprend la séquence WWKFYTLRRA, WWRFYTLRKA, RLGWWKFYTLRRARQGNTKQR, RLGWWRFYTLRKARQGNTKQR ou RLGWWRFYTLRRARQGNTKQR et/ou dans lequel le polypeptide substrat est un polypeptide de fusion GST, par exemple GST-RLGWWRFYTLRRARQGNTKQR.

7. Préparation purifiée comprenant i) un polypeptide LRRK2 ou un polynucléotide LRRK2 recombinant ou un anticorps capable de se lier à LRRK2 obtenu ou pouvant être obtenu par un procédé comprenant l'étape consistant à obtenir l'anticorps contre, ou sélectionner l'anticorps sur la base de la liaison à, un polypeptide consistant en les résidus 100 à 498, ou les résidus 100 à 500 de LRRK2, un fragment de celui-ci, ou une fusion soit du polypeptide soit du fragment, autre qu'avec une séquence dérivée de LRRK2 et ii) un polypeptide substrat tel que défini dans l'une quelconque des revendications précédentes ou un polynucléotide codant pour un polypeptide substrat tel que défini dans l'une quelconque des revendications précédentes.

8. Kit d'éléments comprenant i) un polypeptide LRRK2 ou un polynucléotide LRRK2 recombinant ou un anticorps capable de se lier à LRRK2 obtenu ou pouvant être obtenu par un procédé comprenant l'étape consistant à obtenir l'anticorps contre, ou sélectionner l'anticorps sur la base de la liaison à, un polypeptide consistant en les résidus 100 à 498, ou les résidus 100 à 500 de LRRK2, un fragment de celui-ci, ou une fusion soit du polypeptide soit du fragment, autre qu'avec une séquence dérivée de LRRK2 et ii) un polypeptide substrat tel que défini dans l'une quelconque des revendications précédentes ou un polynucléotide codant pour un polypeptide substrat tel que défini dans l'une quelconque des revendications précédentes.

9. Cellule recombinante capable d'exprimer un polypeptide LRRK2 et un polypeptide substrat tel que défini dans l'une quelconque des revendications 1 à 6, et comprenant un polynucléotide LRRK2 recombinant et un polynucléotide recombinant codant pour le polypeptide substrat.

10. Polypeptide comprenant la séquence (W/F/R/K) (W/F/R/K) (R/K) (F/W/H/R) (Y/W/R) (T) (L/V/I) (R/K) (R/K) (A/Y) ou (W/R) (X) (X) (F/Y/H/T) (Y/W/R) (T) (X) (R/T) (R) (X), où X représente tout acide aminé.

11. Polypeptide selon la revendication 10, consistant en ou comprenant la séquence WWKFYTLRRA, WWRFYTLRKA, RLGWWKFYTLRRARQGNTKQR, RLGWWRFYTLRKARQGNTKQR ou RLGWWRFYTLRRARQGNTKQR et/ou dans lequel le polypeptide est un polypeptide de fusion GST, par exemple GST-RLGWWRFYTLRRARQGNTKQR.

12. Polynucléotide codant un polypeptide selon la revendication 10 ou 11.

13. Procédé selon l'une quelconque des revendications 1 à 6, comprenant l'étape consistant à estimer si le composé module une phosphorylation de polypeptide de la famille ERM dans une cellule entière, un tissu ou un organisme ; ou des caractéristiques du parkinsonisme ou de la maladie de Parkinson dans un organisme et un composé qui module la phosphorylation ou des caractéristiques de la maladie est sélectionné ; comprenant éventuellement l'étape consistant à estimer si le composé module l'activité d'un polypeptide de la famille ERM dans la cellule entière, le tissu ou l'organisme, et un composé qui module l'activité est sélectionné.
